# EUROPEAN PATENT APPLICATION

(11) **EP 4 831 191 A1**
(43) Date of publication of application: **28.10.2026**
(21) Application number: 24907425.3
(22) Date of filing: 17.12.2024
(51) Int. Cl.: C07K 14/00, C07K 14/725, C07K 16/00, C07K 19/00, C12N 5/10, C12N 15/09, C12N 15/12, C12N 15/13, C12N 15/62, C12P 21/02, C12P 21/08

(54) **METHOD FOR PREPARING NOVEL POLYPEPTIDE BY ISOPEPTIDE BOND FORMATION**

(30) Priority: 18.12.2023 JP 2023213313; 29.11.2024 JP 2024209150
(71) Applicant: University Public Corporation Osaka, Osaka-shi, Osaka 5360025 (JP); National University Corporation Yamagata University, Yamagata-shi, Yamagata 990-8560 (JP); Cell Engineering Corporation, Osaka-shi, Osaka 532-0011 (JP)
(72) Inventor: NAKANISHI Takeshi, Sakai-shi, Osaka 599-8531 (JP); TACHIBANA Taro, Sakai-shi, Osaka 599-8531 (JP); KITAMURA Masaya, Sakai-shi, Osaka 599-8531 (JP); MAKABE Koki, Yonezawa-shi, Yamagata 992-8510 (JP); YOSHIMURA Saori, Osaka-shi, Osaka 532-0011 (JP)
(74) Representative: Klöckner, Christoph
(86) International application number: PCT/JP2024/044646
(87) International publication number: WO 2025/135040

(57) **Abstract**

To provide a novel polypeptide comprising an isopeptide bond.

In one aspect, a polypeptide comprising an isopeptide bond between a first and a second polypeptide chain is provided, the polypeptide comprising a) lysine, b) glutamine, glutamic acid, asparagine, or aspartic acid, and c) glutamic acid or aspartic acid, wherein the first and second polypeptide chains each comprise a) and b) in an interface region where the first and second polypeptide chains are in close proximity to each other in a three-dimensional structure of the polypeptide.

## Description

### Technical Field

The present disclosure provides polypeptides comprising an isopeptide bond and methods for producing the same.

### Background Art

Proteins have amide bonds in polypeptide chains as covalent bonds, and may also have disulfide bonds between cysteine side chains. Interchain interactions of polypeptide chains are important for proteins to form higher-order structures, and in particular, covalent interactions strongly link polypeptide chains to each other. Therefore, introduction of additional covalent bonds may be useful for manipulation of proteins.

A bond called an isopeptide bond, which is a type of covalent bond in proteins, is also known, but has been found only in limited proteins such as the Spy0128 protein of Streptococcus pyogenes (Non-Patent Literature 1).

### Prior Art Documents

### Non-Patent Literature

Non-Patent Literature 1: J Am Chem Soc. 2011 Jan 26;133(3):478-85.

### Summary of the Invention

The present inventors investigated polypeptide designs that form isopeptide bonds between polypeptide chains, and produced novel polypeptides comprising an isopeptide bond. Based on this, the present disclosure provides polypeptides comprising an isopeptide bond and methods for producing the same.

Therefore, the present invention provides the following.

### (Item 1)

A method for producing a modified polypeptide comprising an interpeptide linkage represented by the following formula: 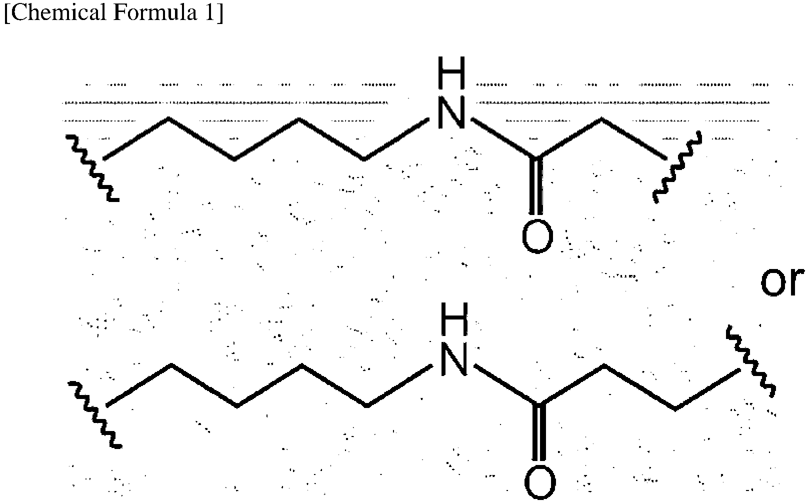 (wherein the dashed lines represent a first or second polypeptide chain, respectively) between a first and a second polypeptide chain, the method comprising:
a step of constructing a three-dimensional structure of an intermediate A comprising the first and second polypeptide chains;
a step of identifying an amino acid sequence present in an interface region where the first and second polypeptide chains are in close proximity to each other in the three-dimensional structure;
a step of mutating the intermediate A, wherein an intermediate B is prepared by introducing into the intermediate A at least one residue of
   a) lysine,
   b) glutamine, glutamic acid, asparagine, or aspartic acid, and
   c) glutamic acid or aspartic acid,
wherein the intermediate B comprises a), b), and c), and the first and second polypeptide chains in the intermediate B comprise a) and b) in the interface region, respectively; and
a step of obtaining the modified polypeptide by forming the interpeptide linkage in the intermediate B.

### (Item 2)

The method according to any of the preceding items, wherein a) and/or b) are introduced based on a distance between a first amino acid residue of the first polypeptide chain and a second amino acid residue of the second polypeptide chain present within the interface region, and types of the first and second amino acid residues.

### (Item 3)

The method according to any of the preceding items, wherein b) is asparagine or aspartic acid, and two amino acid positions at which a distance between α carbons is about 7 to 9 Å in the three-dimensional structure of the intermediate A are identified as amino acid positions for a) and b).

### (Item 4)

The method according to any of the preceding items, wherein b) is glutamine or glutamic acid, and two amino acid positions at which a distance between α carbons is about 8.5 to 10 Å in the three-dimensional structure of the intermediate A are identified as amino acid positions for a) and b).

### (Item 5)

The method according to any of the preceding items, wherein positions of two amino acids whose side chains are disposed in the interior of the three-dimensional structure in the three-dimensional structure of the intermediate A are identified as amino acid positions for a) and b).

### (Item 6)

The method according to any of the preceding items, further comprising a step of constructing a three-dimensional structure of the intermediate B.

### (Item 7)

The method according to any of the preceding items, wherein in the three-dimensional structure of the intermediate B, a distance between the ζ-position nitrogen atom of the residue of a) and an oxygen atom of a carboxyl group in the side chain of the residue of c) is about 2 to 5 Å.

### (Item 8)

The method according to any of the preceding items, wherein in the three-dimensional structure of the intermediate B, a distance between an oxygen atom of a carboxyl group or an amide group in the side chain of the residue of b) and an oxygen atom of a carboxyl group in the side chain of the residue of c) is about 2 to 5 Å.

### (Item 9)

The method according to any of the preceding items, wherein in the three-dimensional structure of the intermediate B, a distance between the ζ-position nitrogen atom of the residue of a) and an oxygen atom of a carboxyl group or an amide group in the side chain of the residue of b) is about 2 to 5 Å.

### (Item 10)

The method according to any of the preceding items, wherein the first polypeptide chain comprises c).

### (Item 11)

The method according to any of the preceding items, further comprising a step of determining hydrophobicity of the interface region.

### (Item 12)

The method according to any of the preceding items, further comprising a step of, in an amino acid sequence present in the interface region, a mutation that deletes a hydrophilic amino acid residue; a mutation that replaces an amino acid residue with a hydrophobic amino acid residue; or insertion of a hydrophobic amino acid residue.

### (Item 13)

The method according to any of the preceding items, wherein a hydrophobic amino acid having an α carbon at a location within 12 Å from the ζ-position nitrogen atom of the residue of a) in the three-dimensional structure of the intermediate B is introduced.

### (Item 14)

The method according to any of the preceding items, wherein hydrophobic amino acids having side chains of different volumes are introduced so as to fill space of the interface region.

### (Item 15)

The method according to any of the preceding items, wherein the first and second polypeptide chains are each contained in different molecules.

### (Item 16)

The method according to any of the preceding items, further comprising a step of introducing a mutation that deletes, or substitutes with another amino acid, at least one of two cysteine residues that form a disulfide bond between the first polypeptide chain and the second polypeptide chain in the intermediate A.

### (Item 17)

The method according to Item 15, further producing a secondary modified polypeptide comprising an interpeptide linkage represented by the following formula: 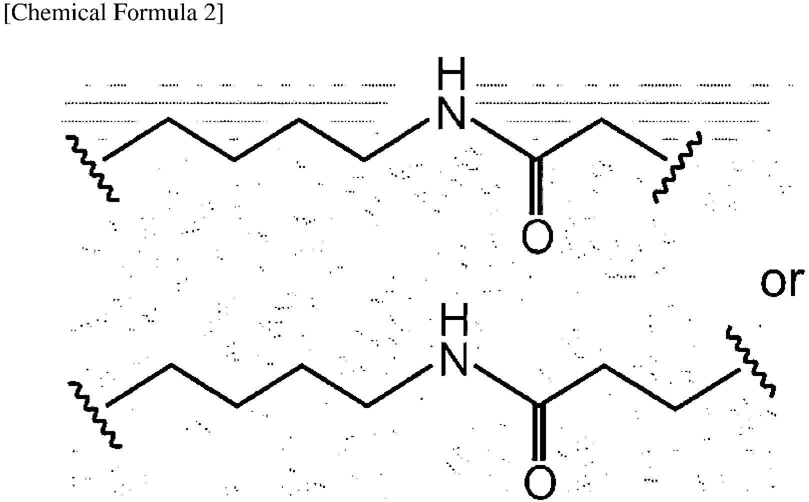 (wherein the dashed lines represent bonds to a polypeptide backbone)
between the modified polypeptide and a third polypeptide chain contained in a molecule different from the modified polypeptide, the method comprising:
a step of constructing a three-dimensional structure of a secondary intermediate A comprising the modified polypeptide and the third polypeptide chain;
a step of identifying an amino acid sequence present in an interface region where the modified polypeptide and the third polypeptide chain are in close proximity to each other in the three-dimensional structure;
a step of mutating the secondary intermediate A, wherein a secondary intermediate B is prepared by introducing into the secondary intermediate A at least one residue of
   a) lysine,
   b) glutamine, glutamic acid, asparagine, or aspartic acid, and
   c) glutamic acid or aspartic acid,
wherein the secondary intermediate B comprises a), b), and c), and the modified polypeptide and the third polypeptide chain in the secondary intermediate B comprise a) and b) in the interface region, respectively
   ; and
a step of obtaining the secondary modified polypeptide by forming the interpeptide linkage in the secondary intermediate B.

### (Item 18)

A polypeptide comprising an interpeptide linkage represented by the following formula: 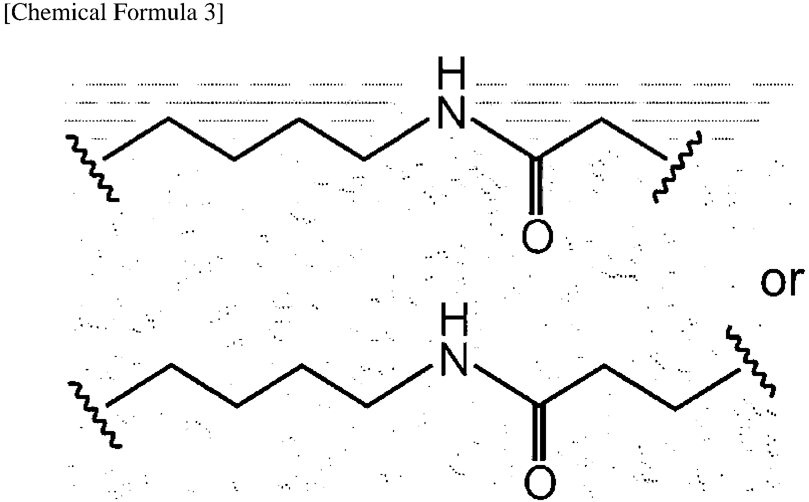 (wherein the dashed lines represent bonds to a polypeptide backbone)
between a first and a second polypeptide chain, the polypeptide comprising
   a) lysine,
   b) glutamine, glutamic acid, asparagine, or aspartic acid, and
   c) glutamic acid or aspartic acid,
wherein the first and second polypeptide chains each comprise a) and b) in an interface region where the first and second polypeptide chains are in close proximity to each other in a three-dimensional structure of the polypeptide.

### (Item 19)

The polypeptide according to any of the preceding items, which is an antibody or a T cell receptor (TCR).

### (Item 20)

The polypeptide according to any of the preceding items, wherein the interpeptide linkage is present in a constant region.

### (Item 21)

The polypeptide according to any of the preceding items, comprising an interpeptide linkage between a heavy chain constant region and a light chain constant region of the antibody.

### (Item 22)

The polypeptide according to any of the preceding items, wherein the first and second polypeptide chains are present on molecules different from each other.

### (Item 23)

A CH1 region in which the amino acid at position 183 of a heavy chain in EU numbering is substituted with asparagine, or a nucleic acid encoding the region;
a heavy chain constant region comprising the CH1 region, or a nucleic acid encoding the region; or
a heavy chain comprising the CH1 region, or a nucleic acid encoding the heavy chain.

### (Item 24)

A CL region in which the amino acid at position 133 of a light chain in EU numbering is substituted with glutamic acid and the amino acid at position 176 of the light chain is substituted with lysine, or a nucleic acid encoding the region;
a light chain constant region comprising the CL region, or a nucleic acid encoding the region; or
a light chain comprising the CL region, or a nucleic acid encoding the light chain.

### (Item 25)

An antibody comprising the heavy chain according to Item 23 and the light chain according to Item 24.

### (Item 26)

A Fab fragment, a Fab' fragment, or an F(ab')₂ fragment, comprising the CH1 region according to Item 23 and the CL region according to Item 24.

### (Item 27)

A chimeric antigen receptor having: a Fab fragment comprising the CH1 region according to Item 23 and the CL region according to Item 24; a hinge region; a transmembrane domain; and an intracellular domain.

### (Item 28)

A cell expressing the chimeric antigen receptor according to Item 27.

The present invention also provides a technology for forming an isopeptide bond between the CH1 region and the CL region of an antibody. Surprisingly, this isopeptide bond can be spontaneously formed in antibody-producing cells by amino acid substitution mutations of the CH1 region and the CL region. An antibody is not substantially impaired in its binding properties to an antigen even by the isopeptide bond, and can maintain its functionality. Therefore, all existing applications of antibodies can be replaced with antibodies having the isopeptide bond of the present invention. Since the isopeptide bond stabilizes Fab fragments and Fab' fragments, it will also be possible to replace various applications that were previously only achievable with scFv with Fab fragments and Fab' fragments stabilized by the isopeptide bond.

In the present invention, it is intended that the one or more features described above can be provided in further combinations in addition to the explicitly described combinations. Still further embodiments and advantages of the present invention will be recognized by a person skilled in the art upon reading and understanding the following detailed description as needed.

The present disclosure provides a novel polypeptide comprising an isopeptide bond. A polypeptide comprising an isopeptide bond of the present disclosure has one or more of utilities such as improved stability and production of a target substance with high purity. The present invention is applicable to the production of a wide range of polypeptides, including the production of recombinant antibodies having complex structures, such as antibodies, for example, multispecific antibodies (particularly bispecific or trispecific antibodies).

### Brief Description of the Drawings

[FIG. 1] FIG. 1 shows the structures of side chain portions of asparagine, lysine, and glutamic acid in the three-dimensional structure of a complex composed of isoCH1 and isoCL before isopeptide bond formation.
[FIG. 2] FIG. 2 shows the structure of the isopeptide bond portion in the three-dimensional structure of a complex composed of isoCH1 and isoCL (left) or a complex composed of isoCH1v2 and isoCLv2 (right) after isopeptide bond formation.
[FIG. 3] FIG. 3 shows the results of Western blotting indicating that the target complex was formed. The left panel shows the results of Coomassie blue staining, the center panel shows the results of staining with an Fc region-binding antibody, and the right panel shows the results of staining with a light chain kappa region-binding antibody. The left lane is a molecular weight marker, the center lane (1) is h4D5 IgG, and the right lane (2) is h4D5 iSoMAb.
[FIG. 4] FIG. 4 shows the results of flow cytometry indicating that h4D5 iSoMAb retains the binding ability of the original antibody. It is shown that fluorescently labeled h4D5 IgG and fluorescently labeled h4D5 iSoMAb have equivalent binding ability to HER2-expressing SKBR-3 cells.
[FIG. 5] FIG. 5 shows the results of SDS-polyacrylamide gel electrophoresis indicating that the target complex was formed. The left panel shows the results of a molecular weight marker, the center panel shows the results of h4D5 Fab, and the right panel shows the results of h4D5 iSoMAb Fab. Lane 1 is a culture supernatant sample, lane 2 is a flow-through sample, lane 3 is a wash sample, and lane 4 is an elution sample.
[FIG. 6] FIG. 6 shows the results of differential scanning fluorimetry of h4D5 iSoMAb Fab and h4D5 Fab under conditions with or without addition of a reducing agent (TCEP). Both conditions show destabilization at around 75 to 85 °C.
[FIG. 7] FIG. 7 shows the results of SDS-polyacrylamide gel electrophoresis indicating that the target iSoMAb based on an anti-CD63 antibody was formed. Lane M shows a molecular weight marker, lane 1 shows BSA (control), lanes 2 to 4 show the original antibody and iSoMAbs (two types) expressed in CHO cells, and lanes 5 to 6 show iSoMAbs (two types) expressed in Expi293F cells.
[FIG. 8] FIG. 8 shows the results of flow cytometry indicating that the iSoMAb based on an anti-CD63 antibody maintains binding to CD63-expressing cells. The upper row shows the results of the original antibody, the middle row shows the results of iSoMAb (v1), and the lower row shows the results of iSoMAb (v2).
[FIG. 9] FIG. 9 shows an overview of the structure of the bispecific antibody HER2/HER3 iSoMAb constructed in Example 4. This is a bispecific antibody comprising the variable regions of h4D5 (anti-HER2) and U1-59 (anti-HER3), and h4D5 comprises an isopeptide bond.
[FIG. 10] FIG. 10 shows the results of a binding confirmation test of h4D5xU1-59 iSoMAb bispecific IgG by sandwich ELISA. The left shows an overview of this test. The right shows the results and shows the results of monospecific h4D5 IgG and U1-59 IgG, and h4D5xU1-59 iSoMAb bispecific IgG. The vertical axis shows color development intensity, and the horizontal axis shows antibody concentration.
[FIG. 11-1] FIG. 11-1 shows alignment results of various antibody heavy chain sequences (positions 118 to 167). Residue numbers follow EU numbering.
[FIG. 11-2] FIG. 11-2 shows alignment results of various antibody heavy chain sequences (positions 168 to 217). Residue numbers follow EU numbering.
[FIG. 11-3] FIG. 11-3 shows alignment results of various antibody heavy chain sequences (positions 218 to 220). Residue numbers follow EU numbering.
[FIG. 11-4] FIG. 11-4 shows alignment results of various antibody heavy chain sequences (positions 221 to 270). Residue numbers follow EU numbering.
[FIG. 11-5] FIG. 11-5 shows alignment results of various antibody heavy chain sequences (positions 271 to 320). Residue numbers follow EU numbering.
[FIG. 11-6] FIG. 11-6 shows alignment results of various antibody heavy chain sequences (positions 321 to 369). Residue numbers follow EU numbering.
[FIG. 11-7] FIG. 11-7 shows alignment results of various antibody heavy chain sequences (positions 370 to 417). Residue numbers follow EU numbering.
[FIG. 11-8] FIG. 11-8 shows alignment results of various antibody heavy chain sequences (positions 418 to 447). Residue numbers follow EU numbering.
[FIG. 12-1] FIG. 12-1 shows alignment results of various antibody light chain kappa chain sequences (positions 108 to 156). Residue numbers of the kappa chain follow EU numbering, and residue numbers of the lambda chain follow Kabat numbering.
[FIG. 12-2] FIG. 12-2 shows alignment results of various antibody light chain lambda chain sequences (positions 107A to 156). Residue numbers of the kappa chain follow EU numbering, and residue numbers of the lambda chain follow Kabat numbering.
[FIG. 13] FIG. 13 shows the cytotoxic activity against cancer cells of the antibody-drug conjugates of WT MAb and iSoMAb v1, respectively, based on an anti-CD63 antibody. The vertical axis shows cytotoxic activity, and the horizontal axis shows the concentration of the antibody-drug conjugate.
[FIG. 14] FIG. 14 shows the cytotoxic activity against cancer cells of the antibody-drug conjugates of WT Fab and iSoMAb Fab, respectively, based on an anti-CD63 antibody Fab format. The vertical axis shows cytotoxic activity, and the horizontal axis shows the concentration of the antibody-drug conjugate.
[FIG. 15] FIG. 15 shows changes in blood concentration after intraperitoneal administration of h4D5 WT MAb and h4D5 iSoMAb to mice. The vertical axis shows blood concentration, and the horizontal axis shows time after intraperitoneal administration.
[FIG. 16] FIG. 16 shows binding to antigen-positive cells of WT Fab and iSoMAb Fab, respectively, based on an anti-CD63 antibody Fab format. The vertical axis shows cell count, and the horizontal axis shows fluorescence intensity.
[FIG. 17] FIG. 17 shows the results of analysis of purified h4D5 iSoMAb variants by SDS-PAGE.
[FIG. 18] FIG. 18 shows the results of analysis of purified h4D5 iSoMAb variants by SDS-PAGE.
[FIG. 19] FIG. 19 shows the results of analysis of h4D5 iSoMAb variants by SDS-PAGE and Western blotting.
[FIG. 20] FIG. 20 shows the binding behavior of h4D5 WT Fab and h4D5 iSoMAb Fab, respectively, to the HER2 extracellular domain by surface plasmon resonance. The vertical axis shows the binding amount, and the horizontal axis shows the elapsed time from the addition of Fab.
[FIG. 21] The left panel shows the electron density of the isopeptide bond introduction site in hCD63 iSoMAb Fab. The right panel shows the superimposition of the iSoMAb Fab crystal structure and the Fab crystal structure. The isopeptide bond site is also shown in a space-filling model.
[FIG. 22] FIG. 22 shows the results of analysis of Nivolumab iSoMAb by SDS-PAGE and Western blotting.
[FIG. 23] FIG. 23 shows the results of analysis of Panitumumab iSoMAb by SDS-PAGE and Western blotting.
[FIG. 24] FIG. 24 shows the results of analysis of purified 372 WT MAb and 372 iSoMAb by SDS-PAGE.
[FIG. 25] FIG. 25 shows the h4D5 iSoMAb Fab Brevibacillus expression plasmid that was constructed.
[FIG. 26] FIG. 26 shows the results of analysis of h4D5 iSoMAb Fab produced by Brevibacillus by SDS-PAGE and Western blotting.
[FIG. 27] FIG. 27 shows the results of analysis by SDS-PAGE of h4D5 iSoMAb Fab expressed using Brevibacillus and purified by a His-tag column.
[FIG. 28] FIG. 28 shows the CAR expression plasmids (i) and (ii) that were constructed.
[FIG. 29] FIG. 29 shows a blot with an anti-GFP antibody of lysates of HEK293 cells transfected with CAR expression vector (i) and/or CAR expression vector (ii). In cells transfected with CAR expression vector (i) and CAR expression vector (ii), a band containing isopeptide bond-formed trastuzumab Fab (indicated by an arrow) was detected on the high molecular weight side.
[FIG. 30] FIG. 30 shows fluorescence microscopy observation of HEK293 cells transfected with CAR expression vector (i) and CAR expression vector (ii).
[FIG. 31] FIG. 31 shows HEK293 cells 3 days after transfection with CAR expression vector (i) and/or CAR expression vector (ii), to which culture supernatant containing HER2-mouse Fc was added and stained with anti-mouse IgG-PE and Hoechst 33342. Ellipses indicate nuclei. Arrows on the cell membrane surface indicate the presence of HER2-mouse Fc.
[FIG. 32] FIG. 32 shows the results of analysis of the prepared iSoMAb by SDS-PAGE and Western blotting.
[FIG. 33] FIG. 33 shows the results of analysis of the prepared iSoMAb by SDS-PAGE and Western blotting.

### Detailed Description of the Invention

Hereinafter, the present disclosure is described while showing the best mode. Throughout the present specification, it should be understood that a singular expression also includes the concept of its plural form unless otherwise specified. Accordingly, it should be understood that singular articles (for example, "a", "an", "the" and the like in the case of English) also include the concept of their plural forms unless otherwise specified. It should also be understood that terms used in the present specification are used in the meanings commonly used in the art unless otherwise specified. Therefore, unless otherwise defined, all technical terms and scientific terms used in the present specification have the same meanings as commonly understood by a person skilled in the art to which the present disclosure belongs. In the case of conflict, the present specification (including definitions) shall prevail.

Definitions and/or basic technical contents of terms particularly used in the present specification are described below as appropriate.

In the present specification, the term "comprising" encompasses "consisting of." "Comprising" means that a third component not described may be included as long as the gist of the invention is not impaired, whereas "consisting of" means that a third component not described is substantially not included. "Substantially not included" is used in the meaning that a third component that is inevitably incorporated during the manufacturing process or a third component below the detection limit (for example, impurities and the like) may be included.

In the present specification, "amino acid" is used in its ordinary meaning in the art and refers to a compound having a carboxyl group and an amino group within a single molecule. Representatively, an amino acid is an α amino acid, but amino acids having a greater distance between the carboxyl group and the amino group, such as β amino acids and γ amino acids, may also be present. In the present specification, unless otherwise indicated, an amino acid refers to the L-form optical isomer, and in the case of the D-form, D (or d) is prefixed (for example, D-Ala (or d-Ala)), or it is represented by a lowercase letter of the alphabet (for example, a). Representative examples of amino acids are shown below, but other amino acids are also contemplated in the present specification.

**[Table 1]**

| Table 1 : Representative Amino Acids | | |
|---|---|---|
| Three-letter code | One-letter code | Amino acid name |
| Gly | G | Glycine |
| Ala | A | Alanine |
| Val | V | Valine |
| Leu | L | Leucine |
| Ile | I | Isoleucine |
| Ser | S | Serine |
| Thr | T | Threonine |
| Met | M | Methionine |
| Cys | C | Cysteine |
| Phe | F | Phenylalanine |
| Tyr | Y | Tyrosine |
| Trp | W | Tryptophan |
| His | H | Histidine |
| Lys | K | Lysine |
| Arg | R | Arginine |
| Glu | E | Glutamic acid |
| Asp | D | Aspartic acid |
| Gln | Q | Glutamine |
| Asn | N | Asparagine |
| Pro | P | Proline |

In the amino acid H₂N-C(R)-COOH, the carbon to which the amino group is bonded is identified as the α position, and positional notations for representative amino acids relating to isopeptide bond formation of the present disclosure are shown below.

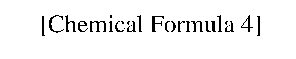

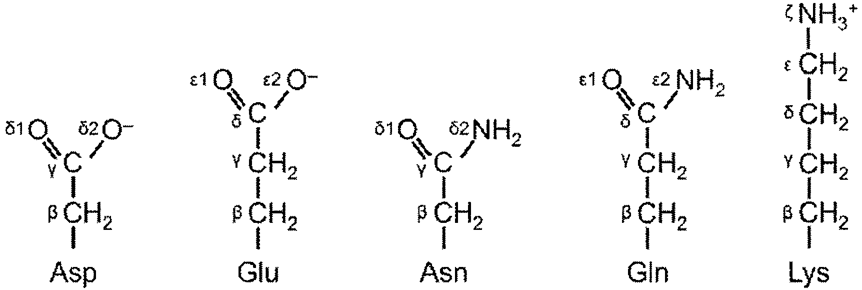

In the present specification, "polypeptide" refers to a molecule in which a plurality of amino acids are bonded. For modification of amino acids in the amino acid sequence of a polypeptide, known methods such as site-directed mutagenesis (Kunkel et al. (Proc. Natl. Acad. Sci. USA (1985) 82, 488-492)) and Overlap extension PCR may be appropriately employed. In addition, amino acid modification methods for substituting with amino acids other than natural amino acids may also be employed (Annu. Rev. Biophys. Biomol. Struct. (2006) 35, 225-249, Proc. Natl. Acad. Sci. U.S.A. (2003) 100 (11), 6353-6357). For example, a cell-free translation system (Clover Direct (Protein Express)) or the like in which a tRNA in which a non-natural amino acid is linked to a complementary amber suppressor tRNA of the UAG codon (amber codon), which is one of the stop codons, is included is suitably used.

In the present specification, an amino acid or nucleotide at a "corresponding region" or "corresponding position" refers to an amino acid or nucleotide in a certain polypeptide molecule or polynucleotide molecule that has, or is predicted to have, a function similar to that of an amino acid or nucleotide at a predetermined position in a polypeptide or polynucleotide serving as a reference for comparison. For example, a person skilled in the art can perform alignment between the original molecule and a variant thereof and identify the corresponding position by setting an appropriate threshold. For example, one amino acid insertion can increase the corresponding position of each amino acid residue after the insertion site by one. For example, when, in a sequence combining two regions in which at least two consecutive amino acid residues match and a region between them as a result of alignment, 50% or more of the amino acid residues match, this combined portion of the sequence can be identified as a corresponding region. For example, when, as a result of alignment, the following Sequence 1 and Sequence 2 in two amino acid sequences: Sequence 1: XXXGGXX, Sequence 2: XXXA-XX
are identified as corresponding regions, the corresponding positions may be identified such that "A" of Sequence 2 is position 4 and "-" of Sequence 2 is position 5, within this corresponding region.

In the present specification, "mutation" or "modification" of an amino acid refers to insertion, substitution, or deletion of an amino acid. One mutation or modification of an amino acid refers to insertion of one amino acid, substitution of one amino acid, or deletion of one amino acid. Addition of an amino acid to the terminus of an amino acid sequence is also included in amino acid insertion.

In the present specification, as an expression representing amino acid insertion, an expression combining the position of the inserted amino acid in the sequence after insertion and its one-letter code may be used as appropriate. For example, a mutation from the amino acid sequence XXXXXXXX to XXXXXGGXXX may be expressed as insertion of 6G and 7G. In the present specification, as an expression representing amino acid substitution, an expression combining the one-letter codes of the amino acid before and after substitution before and after a number representing a specific position may be used as appropriate. For example, the substitution G6A used with reference to the amino acid sequence XXXXXGXXX represents substitution of glycine at position 6 with alanine. In the present specification, as an expression representing amino acid deletion, an expression indicating the position of the deleted amino acid in the sequence before deletion (with the one-letter code added as necessary) may be used as appropriate. For example, a mutation from the amino acid sequence XXXXXGXXX to XXXXXXXX may be expressed as deletion of position 6 or deletion of 6G. Therefore, mutation at position 6 of an amino acid includes insertion of 6G, substitution of G6A, and deletion of 6G in the above examples.

The position of a mutation is not necessarily expressed based on the sequence before mutation, and for example, a mutation from the amino acid sequence XXXXXXXX to XXXXXGXXX can be expressed as insertion of 6G into the amino acid sequence XXXXXXXX before mutation, or the amino acid sequence XXXXXGXXX after mutation can be expressed as a sequence comprising an insertion at position 6. The position of a mutation is not necessarily expressed with reference to the full-length polypeptide, and may be identified based on the "corresponding region" and "corresponding position" described above.

In the present specification, "conservative amino acid substitution" refers to replacing an amino acid in a peptide with another amino acid having similar properties, and the properties of the peptide can be predicted to be similar before and after conservative substitution. Specific examples of conservative amino acid substitutions are provided elsewhere in the present specification.

In the present specification, unless otherwise stated, a reference to a certain molecule (for example, a protein, a nucleic acid) is also understood to be a reference to a variant of that molecule (for example, a variant having modifications in the amino acid sequence) that exerts a biological function similar to (though not necessarily to the same degree as) the biological function of that molecule. For example, a heavy chain protein comprising an Fc region of an antibody may include not only a protein having a specific amino acid sequence, but also a conjugate of this protein with a label molecule (such as a fluorescent label), an isotope-substituted form, a variant capable of associating with a light chain of an antibody and/or capable of associating with another Fc region of an antibody, and the like. Such variants may include fragments of the original molecule, molecules that are at least 30%, 40%, 50%, 60%, 70%, 80%, 90%, 95%, 98%, or 99% identical when compared with the sequence of the original molecule over the amino acid sequence or nucleic acid sequence of the same size as the original molecule, or aligned by a computer homology program known in the art. Variants may include molecules having modified amino acids (for example, modifications by disulfide bond formation, glycosylation, lipidation, acetylation, or phosphorylation).

In the present specification, "antibody" is used in the broadest sense and encompasses various antibody structures, including monoclonal antibodies, polyclonal antibodies, single domain antibodies, and multispecific antibodies (for example, bispecific antibodies or trispecific antibodies).

In the present specification, "humanized antibody" refers to an antibody having a structure in which CDR regions of an antibody produced in a non-human biological species are incorporated into a human antibody. In addition, "chimeric antibody" refers to an antibody having a structure in which variable regions of an antibody produced in a non-human biological species are incorporated into a human antibody.

In the present specification, "antigen-binding fragment" refers to a partial structure of an antibody having the ability to bind to an antigen of interest, and typically comprises at least a heavy chain variable region and a light chain variable region of the antibody. Examples of antigen-binding fragments include Fv, Fab, Fab', Fab'-SH, F(ab')2, diabodies, and single-chain variable fragments (scFv). In a preferred embodiment, the antigen-binding fragment comprises regions corresponding to the VH region and the CH1 region of the antibody, and regions corresponding to the VL region and the CL region.

In the present specification, "variable region" or "variable domain" refers to a domain of a heavy chain or a light chain of an antibody. The variable domains of the heavy chain and light chain of an antibody (VH and VL, respectively) typically each comprise four conserved framework regions (FR) and three complementarity-determining regions (CDR) (see, for example, Kindt et al. Kuby Immunology, 6th ed., W.H. Freeman and Co., page 91 (2007)).

In the present specification, "complementarity-determining region" or "CDR" refers to a region in the variable domain of an antibody that particularly contributes to antigen binding. Typically, an antibody comprises six CDRs: three in VH (H1, H2, H3), and three in VL (L1, L2, L3). There are multiple types of methods for determining CDR sequences, including the Kabat numbering system, the IMGT numbering system, the Paratome numbering system, and the Chothia numbering system (see, for example, Kabat et al., 1991, Sequences of Proteins of Immunological Interest, 5th ed., Public Health Service, National Institutes of Health, Bethesda, Md. (Kabat numbering system), Kunik, Ashkenazi and Ofran, 2012, "Paratome: an online tool for systematic identification of antigen-binding regions in antibodies based on sequence or structure" Nucl. Acids Res., 40: W521-W524 (Paratome numbering system), Lefranc et al., 2003, Dev Comparat Immunol 27: 55-77 (IMGT numbering system), Al-Lazikani et al., 1997, J. Mol. Biol 273: 927-948 (Chothia numbering system)).

"Framework" or "FR" refers to a region in the variable domain other than the complementarity-determining regions (CDR). The FR of a variable domain typically consists of four FR domains: FR1, FR2, FR3, and FR4. The sequences of CDRs and FRs typically appear in the VH (or VL) in the following order: FR1-H1 (L1)-FR2-H2 (L2)-FR3-H3 (L3)-FR4.

In the present specification, "constant region" or "constant domain" refers to a portion of an antibody other than the variable region. For example, an IgG antibody is a heterotetrameric glycoprotein of about 150,000 daltons composed of two identical light chains and two identical heavy chains linked by disulfide bonds, and from N-terminus to C-terminus, each heavy chain has a variable region (VH) and a heavy chain constant region (CH) comprising a CH1 domain, a hinge region, a CH2 domain, and a CH3 domain. Similarly, from N-terminus to C-terminus, each light chain has a variable region (VL) and a constant light chain (CL) domain. Two types of constant domains are known for the light chain of a native antibody, called kappa (κ) and lambda (λ).

In the present specification, "Fc region" refers to a region comprising at least a portion of the CH3, CH2, and hinge region of the constant domain of an antibody. In some cases, the Fc region may include a CH4 domain present in some antibody classes. The Fc region may comprise the entire hinge region of the constant domain of an antibody. The Fc region is recognized by T cells and the like and is associated with effector functions and the like. In the case of human IgG1, the heavy chain Fc region extends from Cys226, or from Pro230, to the carboxyl terminus of the heavy chain. However, the C-terminal lysine (Lys447) or glycine-lysine (Gly446-Lys447) of the Fc region may or may not be present (the numbering of amino acid residues in the Fc region follows the EU numbering system (EU index) described in Kabat et al., Sequences of Proteins of Immunological Interest, 5th Ed. Public Health Service, National Institutes of Health, Bethesda, MD 1991).

The "class" of an antibody is classified by the type of constant domain contained in the heavy chain of the antibody. There are five major classes of antibodies: IgA, IgD, IgE, IgG, and IgM. Some of these can be further divided into subclasses (isotypes). For example, IgG1, IgG2, IgG3, IgG4, IgA1, and IgA2.

In the present specification, "antigen" refers to a target molecule or a portion thereof to which an antibody binds. An antigenic determinant present in an antigen is called an epitope, and an antibody binds to the epitope portion of an antigen. Epitopes include linear epitopes and conformational epitopes. A linear epitope of a protein antigen is an epitope in which the amino acid primary sequence is recognized by the antibody. A linear epitope of a protein antigen is typically composed of at least 3, at least 5, for example, 8 to 10, or 6 to 20 amino acids. A conformational epitope of a protein antigen is, in contrast to a linear epitope, an epitope that cannot be defined solely by the primary sequence of amino acids. With respect to the recognition of conformational epitopes, an antibody recognizes the three-dimensional structure of a protein. Methods for determining the conformation of an epitope include, but are not limited to, for example, X-ray crystallography, two-dimensional nuclear magnetic resonance spectroscopy, site-specific spin labeling, and electromagnetic paramagnetic resonance spectroscopy. See, for example, Epitope Mapping Protocols in Methods in Molecular Biology (1996), vol. 66, Morris (ed.).

In the present specification, a "chimeric antigen receptor" (CAR) is a chimeric molecule having an antigen-binding fragment of an antibody (in particular, scFv) and an activation domain of an immune cell. A CAR is generally a molecule in which an scFv, an extracellular hinge domain, a transmembrane domain (for example, CD8α or CD28), and an activation signaling domain (for example, CD3ζ) are linked. A CAR can be introduced into a cell and expressed on the cell surface. A cell expressing a CAR can be targeted to a specific antigen. A CAR is introduced into immune cells such as T cells or NK cells, for example, and targets the immune cells such as T cells or NK cells to cancer. In first-generation CARs, an scFv, an extracellular hinge domain, a transmembrane domain (for example, CD8α or CD28), and an activation signaling domain (for example, CD3ζ) were linked, but second-generation CARs further comprise a costimulatory molecule signaling domain for activation of the immune cells into which the CAR has been introduced. As the costimulatory molecule signaling domain, costimulatory factors such as CD28, 4-1BB, OX40, CD27, and ICOS have been used. In third-generation CARs, a plurality of costimulatory factors are incorporated. Fourth-generation CARs have cells modified to have a function of producing cytokines (for example, inflammatory cytokines such as IL12). By releasing cytokines, the major microenvironment can be improved to promote activation of the immune system. Fifth-generation CARs are characterized by having a structure that utilizes cytokine receptor-related signaling pathways (for example, the JAK-STAT pathway) in addition to the costimulatory molecule signaling domain. Such structures include, for example, the intracellular domain of the IL-2 receptor β chain, the intracellular domain of the IL-7 receptor, the intracellular domain of the IL-15 receptor, and the intracellular domain of the IL-21 receptor. Thus, in CARs, improvements have been made to allow sustained proliferation of immune cells into which CARs have been introduced in vivo. It is preferred that regions other than the CDR regions of the scFv are humanized, and it is preferred that any domain other than the scFv portion is derived from a human protein. There may also be cases in which the scFv itself is derived from a human antibody. In addition, various modified versions of CARs have been developed, and in the present disclosure, the scFv region of all these CARs can be replaced with an antigen-binding fragment of an antibody having a heavy chain VH-CH1 domain and a light chain VL-CL domain. For example, in the present invention, an antigen-binding fragment of an antibody such as a Fab fragment or a Fab' fragment can be used in place of the scFv, and a CAR having an antigen-binding fragment of an antibody such as a stabilized Fab fragment or Fab' fragment in which an isopeptide bond is formed between CH1 and CL in the CAR-producing cell can be expressed on the cell surface.

In the present specification, comparison of identity of amino acid sequences or nucleotide sequences is calculated using the sequence analysis tool BLAST with default parameters. The search for identity can be performed using, for example, NCBI BLAST 2.7.1 (released October 19, 2017). The value of "identity" in the present specification usually refers to the value when aligned using the above-described BLAST under default conditions. "Identity" is calculated according to known methods as described above as the proportion of homologous amino acids or nucleotides in amino acid or nucleotide sequences between two or more sequences. Specifically, before calculating the proportion, the amino acid or nucleotide sequences of the amino acid or nucleotide sequence group to be compared are aligned, and gaps are introduced into a portion of the amino acid or nucleotide sequences when necessary to maximize the proportion of identical amino acids or nucleotides. Methods for alignment, methods for calculating proportions, comparison methods, and computer programs related thereto are well known in the art (for example, the above-described BLAST and the like). As the algorithm for comparing amino acid or nucleotide sequences with BLAST, Blastp can be used with default settings.

In the present specification, "pharmaceutical component" means any component that can constitute a pharmaceutical, and examples include an active ingredient (one that itself exhibits a pharmaceutical effect), an additive component (one that itself is not expected to have a pharmaceutical effect, but is expected to play a certain role (for example, an excipient, a lubricant, a surfactant, and the like) when included as a pharmaceutical), and the like. A pharmaceutical component may be a single substance or a combination of a plurality of substances or agents. Any combination such as a combination of an active ingredient and an additive component, a combination of an adjuvant and an active ingredient, and the like may also be included.

In the present specification, "active ingredient" refers to a component that exerts an intended pharmaceutical effect, and a single or a plurality of components may correspond thereto.

In the present specification, "additive component" refers to any component that is not expected to have a pharmaceutical effect but plays a certain role when included as a pharmaceutical, and examples include a pharmaceutically acceptable carrier, a stabilizer, an (auxiliary) agent, a solubility enhancer, a solubilizer, a diluent, an excipient, a buffer, a binder, a diluent, a flavoring agent, and a lubricant.

In the present specification, "drug", "agent", or "factor" (all corresponding to "agent" in English) are used interchangeably in a broad sense, and may be any substance or other element (for example, energy such as light, radioactivity, heat, electricity, and the like) that can achieve an intended purpose. Such substances include, for example, antibodies, proteins, polypeptides, oligopeptides, peptides, polynucleotides, oligonucleotides, nucleotides, nucleic acids (including, for example, DNA such as cDNA and genomic DNA, and RNA such as mRNA), polysaccharides, oligosaccharides, lipids, small organic molecules (for example, hormones, ligands, signal transduction substances, small organic molecules, molecules synthesized by combinatorial chemistry, small molecules that can be used as pharmaceuticals (for example, small molecule ligands and the like), and the like), complex molecules thereof, and mixtures thereof, but are not limited thereto.

In the present specification, "label" refers to an entity (for example, a substance, energy, electromagnetic waves, and the like) for distinguishing a target molecule or substance from others. Examples of such labeling methods include the RI (radioisotope) method, the fluorescence method, the biotin method, the chemiluminescence method, and the like. When a plurality of target proteins or factors or means for capturing them are labeled by the fluorescence method, labeling is performed with fluorescent substances having mutually different fluorescence emission maximum wavelengths. It is preferred that the difference in fluorescence emission maximum wavelengths is 10 nm or more. Any label that does not affect function can be used, and examples of fluorescent substances include Alexa(TM) Fluor. Alexa(TM) Fluor is a water-soluble fluorescent dye obtained by modifying coumarin, rhodamine, fluorescein, cyanine, and the like, is a series covering a wide range of fluorescence wavelengths, and is very stable, bright, and has low pH sensitivity compared with other fluorescent dyes at corresponding wavelengths. Examples of combinations of fluorescent dyes having a fluorescence maximum wavelength difference of 10 nm or more include the combination of Alexa(TM) 555 and Alexa(TM) 633, the combination of Alexa(TM) 488 and Alexa(TM) 555, and the like. Other fluorescent labels include cyanine dyes (for example, Cy3, Cy5, and the like of the CyDye(TM) series), rhodamine 6G reagents, N-acetoxy-N2-acetylaminofluorene (AAF), AAIF (iodine derivative of AAF), and the like. In the present disclosure, such labels can be used to modify a target of interest so as to be detectable by the detection means used. Such modification is known in the art, and a person skilled in the art can appropriately carry out such methods depending on the label and the target of interest.

In the present specification, "kit" refers to a unit in which parts to be provided (for example, antibodies, instructions, and the like) are provided in two or more divided compartments. The kit form is preferred when the objective is to provide a composition that should not be provided as a mixture for reasons such as stability and that is preferably mixed and used immediately before use. Such a kit is advantageously provided with instructions or an explanatory document describing how to use the parts provided or how to process the reagents. When a kit is used as a reagent kit in the present specification, the kit usually includes instructions and the like describing how to use the antibody and the like.

In the present specification, "instructions" are those in which a description of methods of using the present disclosure is written for a physician or other user. These instructions have written therein wording instructing administration of a pharmaceutical and the like of the present disclosure. In addition, the instructions may have written therein wording instructing the dosage form. These instructions are prepared in accordance with the format prescribed by the regulatory authority of the country in which the present disclosure is practiced (for example, the Ministry of Health, Labour and Welfare in the case of Japan, the Food and Drug Administration (FDA) in the case of the United States, and the like), and it is clearly stated that approval has been obtained from the regulatory authority. The instructions are so-called package inserts and are usually provided in a paper medium, but are not limited thereto, and may also be provided in the form of, for example, an electronic medium (for example, a homepage provided on the Internet, an electronic mail).

The term "about" refers to a range of the indicated value plus or minus 10%. When "about" is used with respect to a temperature, it refers to a range of the indicated temperature plus or minus 5 °C, and when "about" is used with respect to a pH, it refers to a range of the indicated pH plus or minus 0.5.

In the present specification, "an interface region where the first and second polypeptide chains are in close proximity to each other" refers to a region between backbone chains of two polypeptides in the first polypeptide and the second polypeptide. In the present specification, "the first and second polypeptide chains are in close proximity to each other" means that the distance between α carbon atoms of a first amino acid in the first polypeptide chain and a second amino acid in the second polypeptide chain is 10 Å or less, and "an interface region where the first and second polypeptide chains are in close proximity to each other" refers to a region within 15 Å (a spherical region with a radius of 15 Å) from the midpoint of a line segment connecting the α carbon atom of such first amino acid and the α carbon atom of the second amino acid.

### (Preferred Embodiments)

Preferred embodiments of the present disclosure are described below. The embodiments provided below are provided for a better understanding of the present disclosure, and it is understood that the scope of the present disclosure should not be limited to the following description. Accordingly, it is apparent that a person skilled in the art can make appropriate modifications within the scope of the present disclosure in view of the description in the present specification. It is also understood that the following embodiments can be used alone or in combination thereof.

In one aspect, the present disclosure provides an isopeptide bond in a polypeptide. Any means for achieving this is contemplated to be within the scope of the present disclosure. For example, even without explicit description, description of a method for producing a polypeptide that forms an isopeptide bond simultaneously contemplates embodiments reflecting other means, such as the produced polypeptide, compositions comprising the same, and uses thereof.

### (Method for Producing a Polypeptide that Forms an Isopeptide Bond)

The present invention provides a method for producing a polypeptide that forms an isopeptide bond. In the present specification, an isopeptide bond refers to an interpeptide linkage having the following structure: 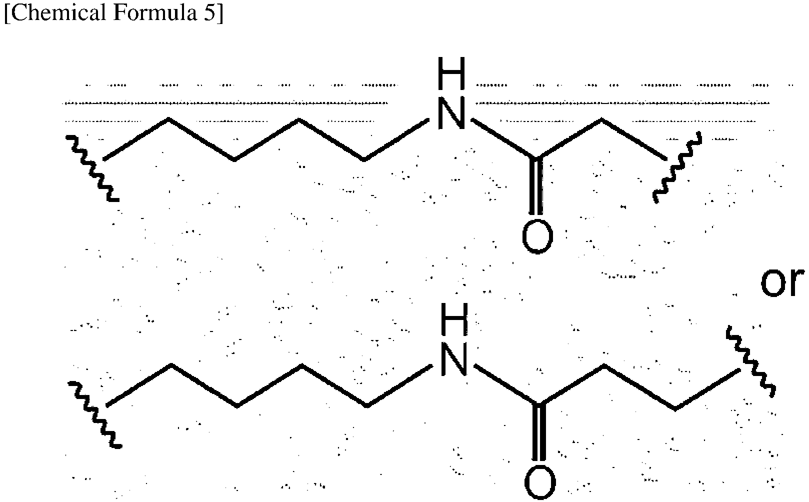 wherein the dashed lines represent the first or second polypeptide chain, respectively, indicating linkage to the α-carbon of each polypeptide. The interpeptide linkage is formed by the bonding of the side chain of lysine of the first or second polypeptide chain with the side chain of asparagine, aspartic acid, glutamine, or glutamic acid. In a representative embodiment, the present disclosure provides a method for producing a modified polypeptide comprising an interpeptide linkage of an isopeptide bond between a first and a second polypeptide chain, the method comprising:
a step of constructing a three-dimensional structure of an intermediate A comprising the first and second polypeptide chains;
a step of identifying amino acid sequences present at the interface region where the first and second polypeptide chains in the three-dimensional structure are in close proximity to each other;
a step of mutating the intermediate A to introduce into the intermediate A at least one residue of:
   a) lysine,
   b) glutamine, glutamic acid, asparagine, or aspartic acid, and
   c) glutamic acid or aspartic acid,
thereby preparing an intermediate B, wherein the intermediate B comprises a), b), and c), and the first and second polypeptide chains in the intermediate B respectively comprise a) and b) in the interface region; and
a step of obtaining the modified polypeptide by forming the interpeptide linkage in the intermediate B.
Herein, the intermediate A corresponds to the starting structure, the intermediate B corresponds to the structure after introduction of mutations but before isopeptide bond formation, and the modified polypeptide corresponds to the structure after introduction of mutations and after isopeptide bond formation.

The formation reaction of the isopeptide bond is considered to proceed in the absence of additional elements such as enzymes, provided that the polypeptide is appropriately designed. However, since only limited examples of proteins having an isopeptide bond are known, such as the Spy0128 protein of Streptococcus pyogenes (J Am Chem Soc. 2011 Jan 26;133(3):478-85.), the design of a polypeptide that promotes the formation reaction of the isopeptide bond is important. An isopeptide bond is formed between the lysine of a) and the glutamine, glutamic acid, asparagine, or aspartic acid of b), and the glutamic acid or aspartic acid of c) has a particularly important role in promoting this reaction. c) may be present in the same molecule as the first or second polypeptide chain, or may be present in a different polypeptide chain. In one embodiment, c) is present in the same molecule as the first polypeptide chain. In one embodiment, c) is present on the N-terminus side of the lysine residue of a) on the first polypeptide chain. In one embodiment, c) is present at a position separated by 5 or more residues from the lysine residue of a) on the first polypeptide chain.

A polypeptide chain comprising or into which a) is introduced is referred to herein as a first polypeptide chain, and a polypeptide chain comprising or into which b) is introduced is referred to herein as a second polypeptide chain. The first polypeptide chain and the second polypeptide chain may be present in the same molecule or may be present in different molecules. In one embodiment, the first polypeptide chain and the second polypeptide chain are present in different molecules. In particular, the fact that the present disclosure enables the formation of an isopeptide bond between different molecules provides advantages such as those in the formation of hetero-complexes as described in the present specification.

In the present specification, the "interface region where the first and second polypeptide chains are in close proximity to each other" refers to a region between the backbone chains of two polypeptides in the first polypeptide and the second polypeptide. In the present specification, the "first and second polypeptide chains are in close proximity to each other" means that there exists at least one combination (also referred to as a "proximal amino acid pair") of a first amino acid and a second amino acid in which the distance between the α carbon atoms of a first amino acid in the first polypeptide chain and a second amino acid in the second polypeptide chain is equal to or less than 10 Å, and the "interface region where the first and second polypeptide chains are in close proximity to each other" refers to a region within 15 Å from the midpoint of the line segment connecting the α carbon atom of such a first amino acid and the α carbon atom of such a second amino acid. When a plurality of proximal amino acid pairs are present between the first polypeptide chain and the second polypeptide chain, the "interface region where the first and second polypeptide chains are in close proximity to each other" refers to a region obtained by overlaying, for all proximal amino acid pairs, regions within 15 Å from the midpoint of the line segment connecting the α carbon atoms of the amino acids of each proximal amino acid pair. In the present disclosure, an isopeptide bond does not necessarily need to be formed between the amino acids of a proximal amino acid pair, but is formed between amino acids whose side chains are contained within the interface region. In a representative embodiment, a region within about 25 Å, within about 20 Å, within about 15 Å, within about 10 Å, within about 7 Å, or within about 5 Å from the midpoint of the line segment connecting the α carbon atom of the amino acid of a) and the α carbon atom of the amino acid of b) may be defined as the interface region. Since the orientation of amino acid side chains protruding from a polypeptide chain is typically not constant, it is particularly preferable to design the amino acid side chains of a) and b) that form the isopeptide bond so as to extend into the interface region toward the other polypeptide. In one embodiment, positions of two amino acids whose side chains are disposed in the interior of the three-dimensional structure are identified as amino acid positions for a) and b).

The construction of the three-dimensional structure may be based on a known three-dimensional structure. For example, a known three-dimensional structure can be obtained from Protein Database or the like. The three-dimensional structure of the starting polypeptide (an intermediate A) can be constructed, for example, by downloading coordinate data of the crystal structure of the starting polypeptide registered in Protein Data Bank (https://www.rcsb.org/) and using PyMOL (https://pymol.org/2/). Based on the constructed three-dimensional structure as needed, the three-dimensional structure of the polypeptide after modification can be constructed by constructing a homology model using SWISS-MODEL (https://swissmodel.expasy.org/) and using Coot (https://www2.mrc-lmb.cam.ac.uk/personal/pemsley/coot/). Default values are used for parameters if necessary. The three-dimensional structures described in the present specification may be those constructed in this manner. A three-dimensional structure of a polypeptide intermediate can be constructed after introducing any mutation. In one embodiment, a three-dimensional structure of the intermediate B is constructed.

In one embodiment, a) and/or b) are introduced based on a distance between a first amino acid residue (side chain) of the first polypeptide chain present within the interface region and a second amino acid residue (side chain) of the second polypeptide chain present within the interface region, and types of the first and second amino acid residues. In one embodiment, b) is asparagine or aspartic acid, and two amino acid positions at which a distance between α carbons is within about 6 to 10 Å, for example, about 6 Å, about 6.5 Å, about 7 Å, about 7.5 Å, about 8 Å, about 8.5 Å, about 9 Å, about 9.5 Å, about 10 Å, or within a range between any two of these values, in the three-dimensional structure of the intermediate A are identified as amino acid positions for a) and b). In one embodiment, b) is glutamine or glutamic acid, and two amino acid positions at which a distance between α carbons is within about 7.5 to 11 Å, for example, about 7.5 Å, about 8 Å, about 8.5 Å, about 9 Å, about 9.5 Å, about 10 Å, about 10.5 Å, about 11 Å
, or within a range between any two of these values, in the three-dimensional structure of the intermediate A are identified as amino acid positions for a) and b).

Atoms of amino acid residues forming the interaction interface between polypeptide chains are present at distances close to each other, and when atoms present in different polypeptide chains are at distances equal to or less than the following: C-C: 4.1 Å, C-N: 3.8 Å, C-O: 3.7 Å, O-O: 3.3 Å, O-N: 3.4 Å, N-N: 3.4 Å, those atoms are referred to herein as an "interacting atom pair" (see S. Sheriff, W.A. Hendrickson, and J.L. Smith, J. Mol. Biol. 197, 273-296 (1987).). On the other hand, if the pair of an atom present in a certain polypeptide chain and the atom present in a different polypeptide chain that is at the closest distance thereto is an interacting atom pair, the two polypeptide chains can interact. In one embodiment, in the three-dimensional structure of the intermediate B (after mutation introduction, before isopeptide bond formation), the side chains of amino acids surrounding a) and b) of the first and second polypeptide chains (for example, amino acids within 7, 6, 5, 4, 3, 2, or 1 amino acid(s) from a) or b), including a) and b)) have interacting atom pairs. In one embodiment, 1, 2, 3, 4, 5, or more amino acid pairs among the amino acid pairs of amino acids surrounding a) and b) have interacting atom pairs. In one embodiment, the side chains of amino acid pairs having interacting atom pairs among the amino acid pairs of amino acids surrounding a) and b) have 1, 2, 3, 4, 5, or more interacting atom pairs. Here, one element (amino acid or atom) can form a plurality of pairs with a plurality of different elements. In one embodiment, amino acid pairs between amino acids within 7, 6, 5, 4, 3, 2, or 1 amino acid(s) on the N-terminus side of a) on the first polypeptide chain and amino acids surrounding b) of the second polypeptide chain have interacting atom pairs.

In one embodiment, in the three-dimensional structure of the intermediate B (after mutation introduction, before isopeptide bond formation), the distance between the ζ-position nitrogen atom of the residue of a) and an oxygen atom of a carboxyl group in the side chain of the residue of c) is within about 1 to 6 Å, for example, about 1 Å, about 1.5 Å, about 2 Å, about 2.5 Å, about 3 Å, about 3.5 Å, about 5 Å, about 5.5 Å, about 6 Å, or within a range between any two of these values. In one embodiment, in the three-dimensional structure of the intermediate B, the distance between an oxygen atom of a carboxyl group or an amide group in the side chain of the residue of b) and an oxygen atom of a carboxyl group in the side chain of the residue of c) is within about 1 to 6 Å, for example, about 1 Å, about 1.5 Å, about 2 Å, about 2.5 Å, about 3 Å, about 3.5 Å, about 5 Å, about 5.5 Å, about 6 Å, or within a range between any two of these values. In one embodiment, in the three-dimensional structure of the intermediate B, the distance between the ζ-position nitrogen atom of the residue of a) and an oxygen atom of a carboxyl group or an amide group in the side chain of the residue of b) is within about 1 to 6 Å, for example, about 1 Å, about 1.5 Å, about 2 Å, about 2.5 Å, about 3 Å, about 3.5 Å, about 5 Å, about 5.5 Å, about 6 Å, or within a range between any two of these values.

In one embodiment, two amino acid positions at which a distance between the α carbons of a) and c) is within about 5 to 10 Å, for example, about 5 Å, about 5.5 Å, about 6 Å, about 6.5 Å, about 7 Å, about 7.5 Å, about 8 Å, about 8.5 Å, about 9 Å, about 9.5 Å, about 10 Å, or within a range between any two of these values, in the three-dimensional structure of the intermediate A are identified as amino acid positions for a) and c). In one embodiment, two amino acid positions at which a distance between the α carbons of b) and c) is within about 5 to 10 Å, for example, about 5 Å, about 5.5 Å, about 6 Å, about 6.5 Å, about 7 Å, about 7.5 Å, about 8 Å, about 8.5 Å, about 9 Å, about 9.5 Å, about 10 Å, or within a range between any two of these values, in the three-dimensional structure of the intermediate A are identified as amino acid positions for a) and c). In one embodiment, three amino acid positions satisfying the above distance between the α carbons of a) and c) and the above distance between the α carbons of b) and c) in the three-dimensional structure of the intermediate A are identified as amino acid positions for a), b), and c).

In one embodiment, in the three-dimensional structure of the intermediate B, the angle between a vector from the α carbon to the β carbon of the residue of a) and a vector from the α carbon to the β carbon of the residue of b) is, without being particularly limited, for example, about 180 °, about 170 ° about 160 °, about 150 °, about 140 °, about 130 °, about 120 °, about 110 °, about 100 °, about 90 °, about 80°, about 70 °, about 60 °, or within a range between any two of these values. In one embodiment, in the three-dimensional structure of the intermediate B, the angle between a vector from the α carbon of the residue of a) to the α carbon of the residue of b) and a vector from the α carbon to the β carbon of the residue of c) is, without being particularly limited, for example, about 130 °, about 120 °, about 110 °, about 100 °, about 90 °, about 80 °, about 70 °, about 60 °, about 50 °, about 40 °, about 30 °, about 20 °, or within a range between any two of these values.

In one embodiment, in the three-dimensional structure of the intermediate B (after mutation introduction, before isopeptide bond formation), one, two, or three of a), b), and c) are not present in a domain. In Spy protein, the reaction proceeds between residues present in a domain to form an isopeptide bond, but in the present disclosure, an isopeptide bond can be formed independently of domain formation. For example, whether a certain residue is present in a domain can be determined based on its solvent-accessible surface area. The solvent-accessible surface area of a residue can be calculated by the AREAIMOL program. AREAIMOL is described in J. Mol. Biol. 55, 379-400 (1971) and is available as a program included in the CCP4 protein structure analysis program package (https://www.ccp4.ac.uk/). It can be calculated by reading the coordinate data of each atom constituting the molecule and using default parameters. For example, the calculation result for the isopeptide bond-forming Asn in isoCH1 (SEQ ID NO: 1) of the examples of the present application was 0.24, the calculation result for the isopeptide bond-forming Lys in isoCL (SEQ ID NO: 2) was 0.24, and the calculation result for the catalytic Glu was 0.25. In one embodiment, a residue may be determined to be present in a domain when the solvent-accessible surface area of the residue is equal to or greater than 0.1, equal to or greater than 0.11, equal to or greater than 0.12, equal to or greater than 0.13, equal to or greater than 0.14, equal to or greater than 0.15, equal to or greater than 0.16, equal to or greater than 0.17, equal to or greater than 0.18, equal to or greater than 0.19, equal to or greater than 0.2, equal to or greater than 0.21, equal to or greater than 0.22, equal to or greater than 0.23, equal to or greater than 0.24, or equal to or greater than 0.25.

Since the isopeptide bond formation reaction between a) and b) catalyzed by c) can be promoted in a hydrophobic environment, it may be preferable that the interface region is a hydrophobic environment. In one embodiment, a step of determining hydrophobicity of the interface region is included. In one embodiment, a mutation that deletes a hydrophilic amino acid residue in an amino acid sequence present in the interface region, a mutation that replaces an amino acid residue with a hydrophobic amino acid residue, or a mutation that inserts a hydrophobic amino acid residue is introduced.

In one embodiment, the hydrophilic amino acids may include glutamic acid, aspartic acid, lysine, arginine, serine, and threonine. In one embodiment, the hydrophilic amino acids may include glutamic acid, aspartic acid, lysine, arginine, serine, threonine, tyrosine, cysteine, and histidine. In one embodiment, the hydrophobic amino acids may include phenylalanine, tryptophan, valine, leucine, and isoleucine. In one embodiment, the hydrophobic amino acids may include phenylalanine, tryptophan, valine, leucine, isoleucine, methionine, and proline.

In one embodiment, in the three-dimensional structure of the intermediate B, a hydrophobic amino acid having an α carbon at a location within about 20 Å, within about 18 Å, within about 16 Å, within about 14 Å, within about 12 Å, within about 10 Å, within about 8 Å, within about 6 Å, or within about 4 Å from the ζ-position nitrogen atom of the residue of a) is introduced. As needed, in the three-dimensional structure of the intermediate B, the α carbon of the introduced hydrophobic amino acid is separated by about 3 Å or more, about 3.5 Å or more, about 4 Å or more, about 4.5 Å or more, or about 5 Å or more from the ζ-position nitrogen atom of the residue of a).

In one embodiment, hydrophobic amino acids having side chains of different volumes are introduced (inserted or substituted) so as to fill space of the interface region. Mutations may be introduced such that the proportion of volume occupied by side chains in the interface region in the three-dimensional structure of the polypeptide after introduction (recalculated) exceeds the proportion of volume occupied by side chains in the interface region in the three-dimensional structure of the polypeptide before introduction. Even when an amino acid having a side chain with a large volume, such as tryptophan, is introduced, the proportion of volume occupied by side chains in the interface region does not necessarily increase, and the proportion may rather decrease due to an increase in the distance between polypeptide backbone chains. The volumes of amino acid side chains can be arranged in order from smallest as follows: glycine, proline, alanine, valine, leucine = isoleucine, methionine, phenylalanine, tryptophan. In one embodiment, in the three-dimensional structure of the intermediate B, at least 60%, at least 62%, at least 64%, at least 66%, at least 68%, at least 70%, at least 72%, at least 74%, at least 76%, at least 78%, at least 80%, at least 82%, or at least 84% of the volume of the interface region where the first and second polypeptide chains are in close proximity to each other is occupied by residues of the first and second polypeptide chains. Here, for the calculation of side chain volume, each atom is given the following atomic radii: hydrogen: 0.53 Å, carbon: 0.67 Å, nitrogen: 0.56 Å, oxygen: 0.48 Å, sulfur: 0.88 Å. When the spherical volumes of atoms overlap, the overlapping portion is subtracted. In one embodiment, in the three-dimensional structure of the intermediate B, at least 60%, at least 62%, at least 64%, at least 66%, at least 68%, at least 70%, at least 72%, at least 74%, at least 76%, at least 78%, at least 80%, at least 82%, or at least 84% of the volume of a sphere with a radius of 15 Å from the epsilon nitrogen atom of the lysine of a) is occupied by residues of the first and second polypeptide chains.

In the step of obtaining the modified polypeptide by forming the interpeptide linkage in the intermediate B (polypeptide after mutation introduction and before isopeptide bond formation) described in the present specification, additional elements such as enzymes may be unnecessary. This step can be performed both in vitro and in vivo (intracellularly). For example, the first and second polypeptide chains can be produced in different cells, and thereafter, the isopeptide bond can be formed in vitro from these polypeptide chains. In a cell that co-expresses the first and second polypeptide chains, the isopeptide bond can be formed in vivo. By manipulating the intracellular localization of the first and second polypeptide chains (for example, by fusing a localization signal molecule), the formation of the isopeptide bond can be suppressed or promoted.

Compared to the starting polypeptide (an intermediate A), the final polypeptide (modified polypeptide) may have an amino acid sequence that is at least about 70%, about 80%, about 90%, about 95%, about 96%, about 97%, about 98%, or about 99% identical when compared to the sequence of the original molecule aligned by performing alignment using a computer homology program known in the art. The final polypeptide (modified polypeptide) may contain any mutation, but in one embodiment, at least about 30%, about 40%, about 50%, about 60%, about 70%, about 80%, about 90%, or about 95% of the mutations are conservative amino acid substitutions. In one embodiment, a conservative amino acid substitution is replacement of an amino acid in the following groups of amino acids with another amino acid within the same group:
Group 1: glycine, alanine, valine, leucine, and isoleucine;
Group 2: serine and threonine;
Group 3: phenylalanine, tyrosine, and tryptophan;
Group 4: lysine, hydroxylysine, arginine, and histidine;
Group 5: cysteine, methionine, methionine sulfoxide, and homocysteine;
Group 6: proline and hydroxyproline;
Group 7: glutamic acid and aspartic acid;
Group 8: glutamine and asparagine;
Group 9: serine and cysteine.

### (Polypeptide Comprising an Isopeptide Bond)

In one aspect, the present disclosure provides a polypeptide comprising an interpeptide linkage represented by the following formula between first and second polypeptide chains: 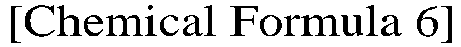 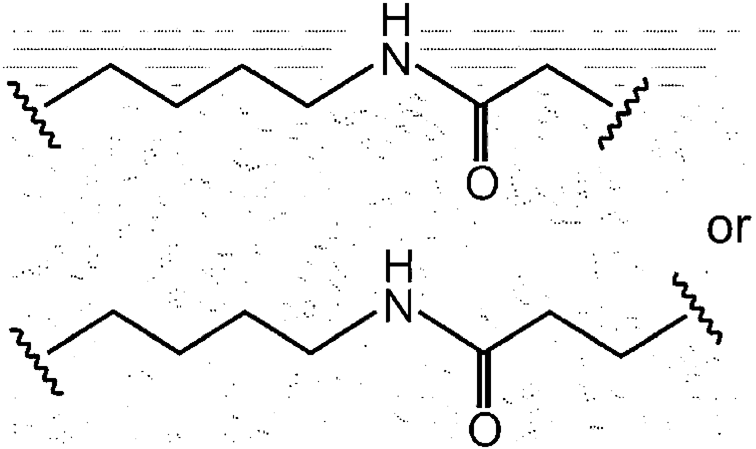 (wherein the dashed lines represent bonds to the polypeptide backbone), the polypeptide comprising:
a) lysine,
b) glutamine, glutamic acid, asparagine, or aspartic acid, and
c) glutamic acid or aspartic acid,
wherein, in the interface region where the first and second polypeptide chains are in close proximity to each other in the three-dimensional structure of the polypeptide, the first and second polypeptide chains respectively comprise a) and b).

The polypeptide comprising an isopeptide bond of the present disclosure may be one produced by the method for producing a polypeptide that forms an isopeptide bond of the present disclosure. In one embodiment, the polypeptide comprising an isopeptide bond of the present disclosure has a structural difference from a known polypeptide or a natural polypeptide in that a mutation in the method for producing a polypeptide that forms an isopeptide bond of the present disclosure has been introduced. In one embodiment, the polypeptide comprising an isopeptide bond of the present disclosure may have the arrangement of a), b), c), and/or hydrophobic residues described above. The arrangement of these residues is generally maintained before and after isopeptide bond formation. In one embodiment, the polypeptide comprising an isopeptide bond of the present disclosure may have an amino acid sequence that is at least about 70%, about 80%, about 90%, about 95%, about 96%, about 97%, about 98%, or about 99% identical when compared to the sequence of the original molecule aligned by performing alignment using a computer homology program known in the art, as compared to a known polypeptide or a natural polypeptide.

In one embodiment, the polypeptide comprising an isopeptide bond of the present disclosure is a polypeptide molecule in which different polypeptide molecules are linked by an isopeptide bond, and this can be determined by the fact that two polypeptide chains linked by the isopeptide bond are not linked by a peptide bond.

In one embodiment, the polypeptide comprising an isopeptide bond of the present disclosure is an antibody or a T cell receptor (TCR). In one embodiment, the isopeptide bond (interpeptide linkage) is present in a constant region. For example, if an isopeptide bond is introduced into a constant region of an antibody (such as a constant region involved in the binding of a heavy chain and a light chain), antibodies having various target specificities can be provided by combining with various variable regions while maintaining the isopeptide bond-forming ability. In one embodiment, the polypeptide comprising an isopeptide bond of the present disclosure is a therapeutic protein used in enzyme replacement therapy, a cytokine, or a chemokine. In one embodiment, examples of the polypeptide comprising an isopeptide bond of the present disclosure include, but are not limited to, Fc receptors such as FcRn, and cytokine receptors such as TNF-α receptor.

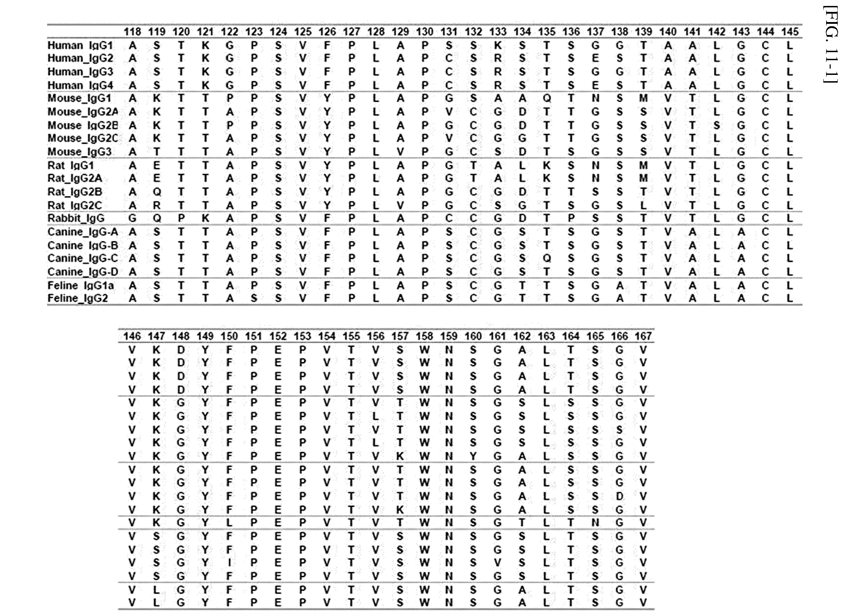

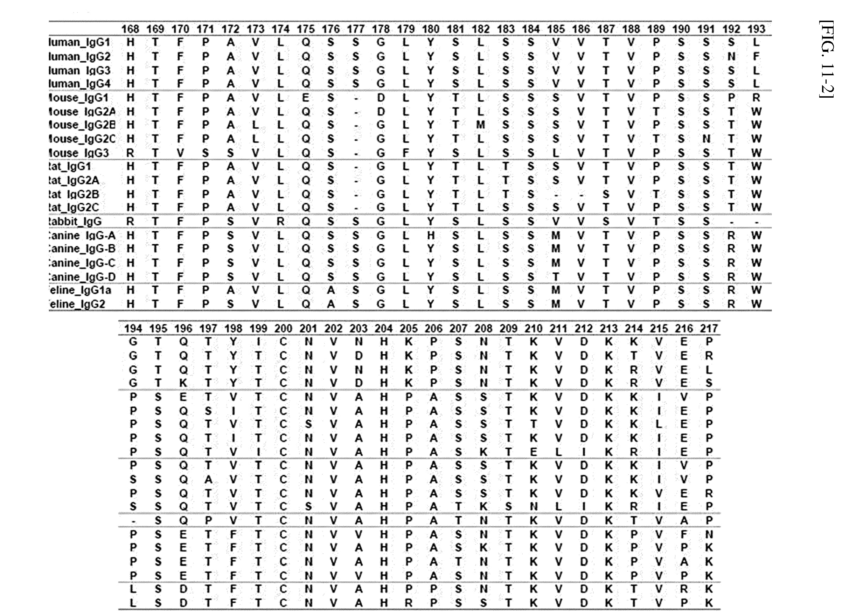

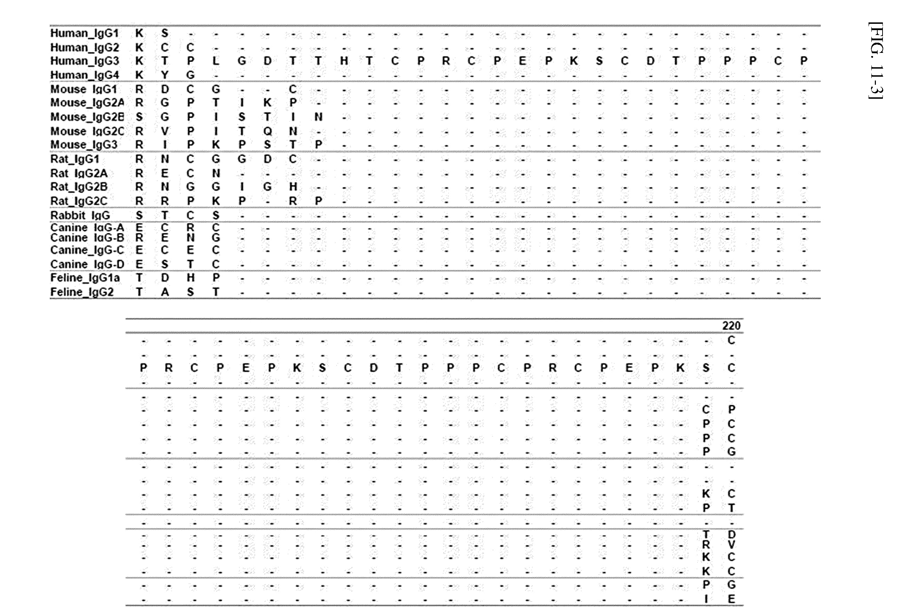

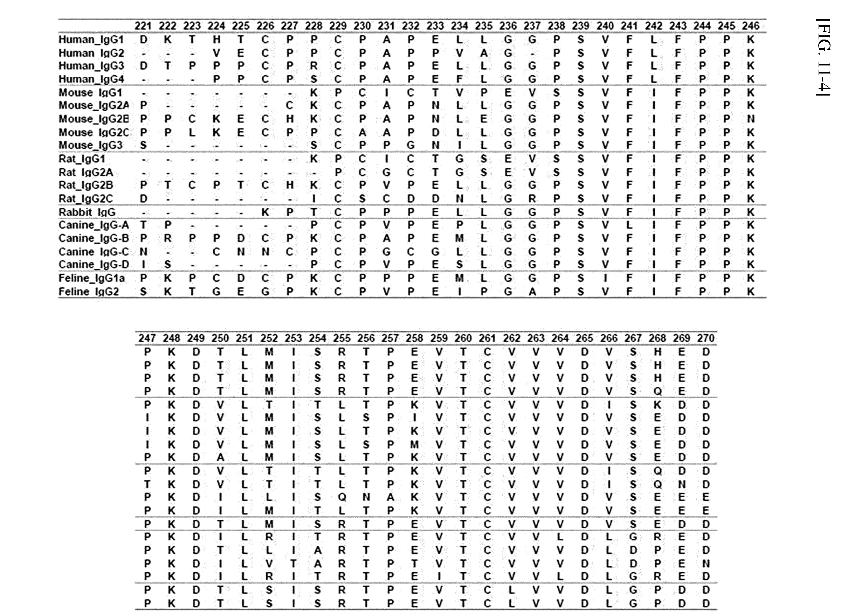

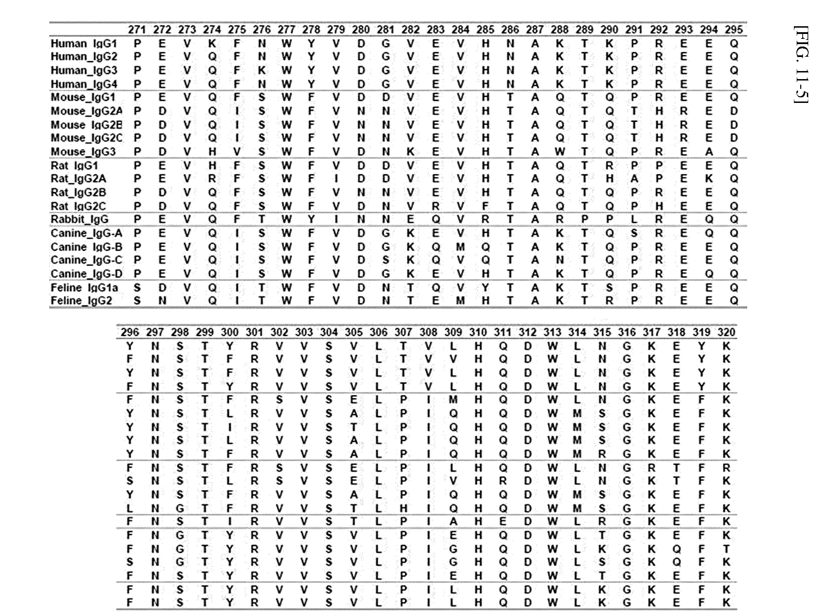

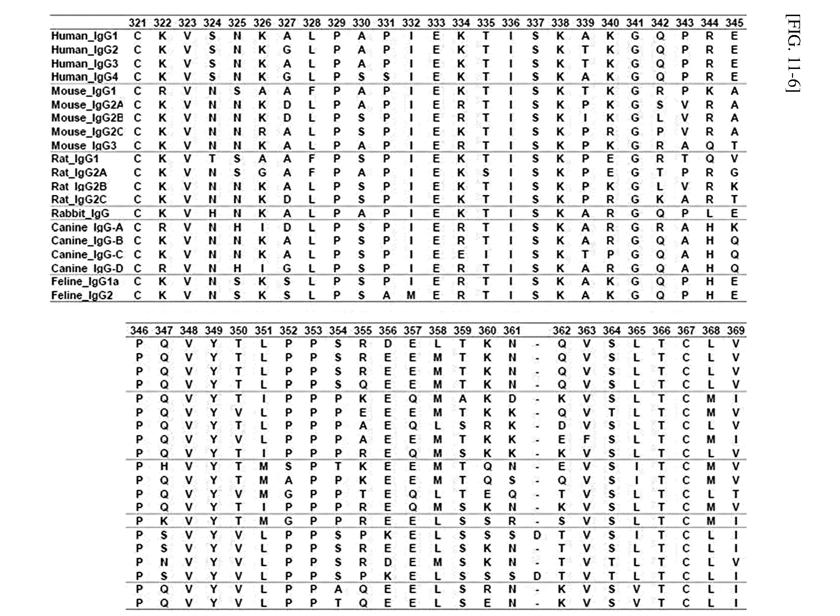

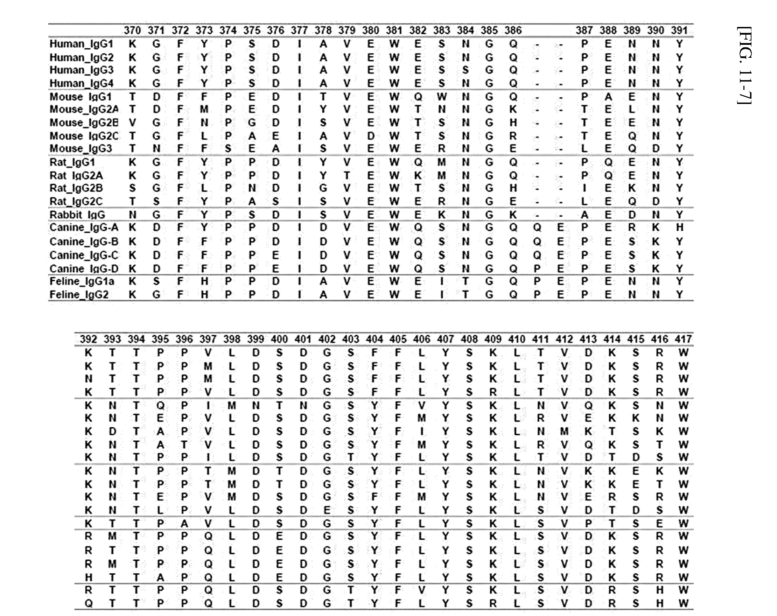

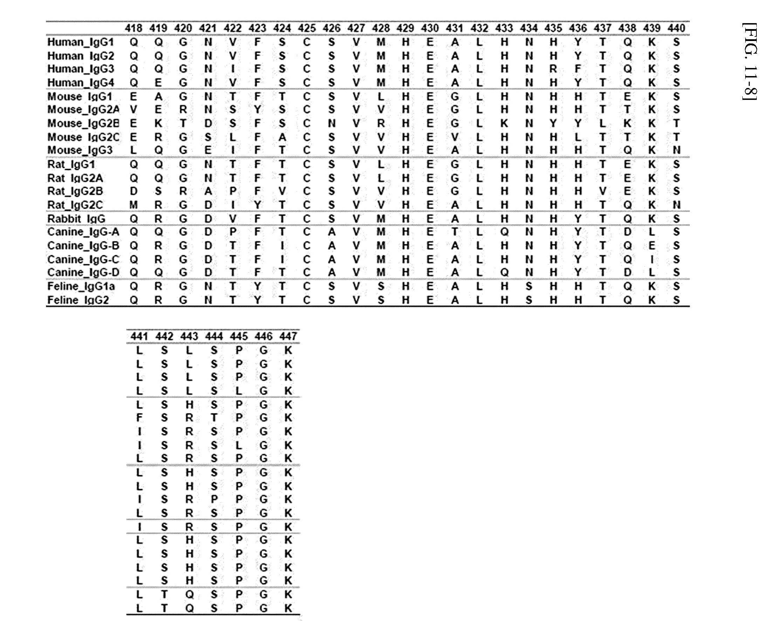

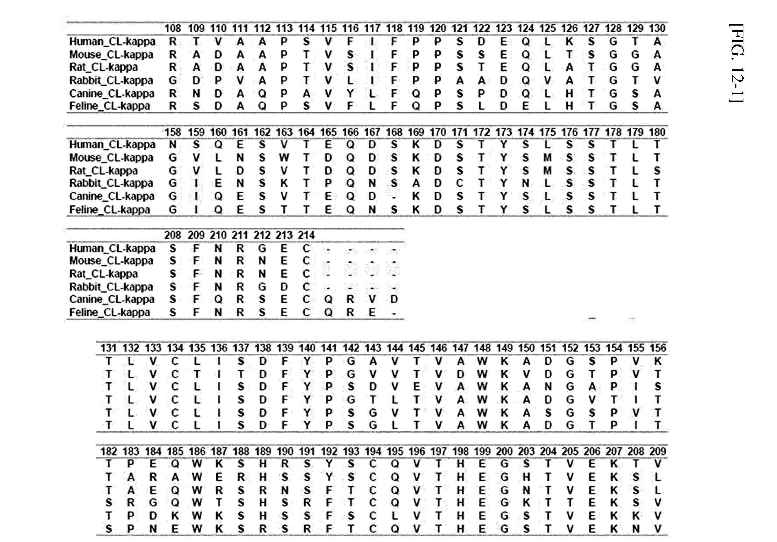

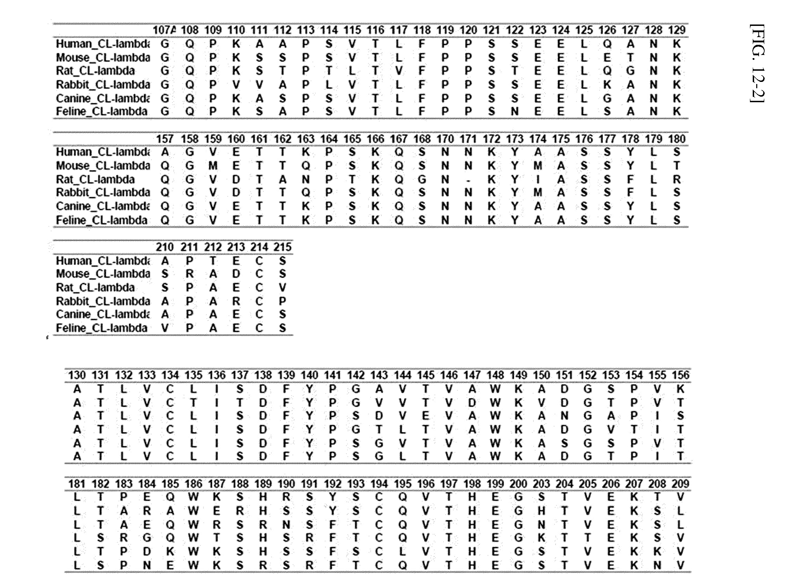

For further description of embodiments of antibodies comprising an isopeptide bond, alignment results of representative antibody sequences are shown in FIG. 11 to 12. Even in the case of antibodies derived from other biological species, sequence information can be obtained and the sites for introducing iSoMAb mutations can be similarly determined. These were created and numbered using CLUSTALW (J.D. Thompson, D.G. Higgins, and T.J. Gibson, Nucleic Acids Res. 22, 4673-4680 (1994)). Residue numbers of the heavy chain constant region and the kappa chain constant region follow EU numbering, and residue numbers of the lambda chain constant region follow Kabat numbering, and in the present specification, residues of antibodies may be referred to with the numbers shown in FIG. 11 to 12. Based on successful examples of antibodies comprising an isopeptide bond shown in the examples of the present specification, antibodies comprising modifications similar to those in the examples may also form an isopeptide bond. In one embodiment, the antibody comprising an isopeptide bond of the present disclosure comprises asparagine or aspartic acid at heavy chain position 183, glutamic acid at light chain position 133, and lysine at light chain position 176, and optionally comprises one or more of the following: isoleucine at heavy chain position 173, leucine at heavy chain position 181, valine at light chain position 131, methionine at light chain position 162, leucine at light chain position 178, and glutamine at light chain position 180. In one embodiment, the antibody comprising an isopeptide bond of the present disclosure comprises lysine at heavy chain position 183, glutamic acid at heavy chain position 170, and asparagine or aspartic acid at light chain position 176, and optionally comprises one or more of the following: isoleucine at heavy chain position 173, leucine at heavy chain position 181, methionine at light chain position 162, leucine at light chain position 178, and glutamine at light chain position 180. It can be understood that by introducing the above mutations into an antibody, an isopeptide bond is formed between the heavy chain and the light chain in antibodies of different subclasses derived from various animal species (see FIG. 11 and 12). In certain preferred embodiments, for example, the antibody may be human IgG1, human IgG2, or human IgG4. In certain preferred embodiments, the light chain may be a human κ chain or a human λ chain.

The present invention also provides an antibody comprising a CH1 region in which the amino acid at heavy chain position 183 is substituted with asparagine, and a CL region in which the amino acid at light chain position 133 is substituted with glutamic acid and the amino acid at light chain position 176 is substituted with lysine, wherein an isopeptide bond is formed between the asparagine and the glutamic acid. In this embodiment, the substitution with asparagine at heavy chain position 183 may be a substitution with glutamine, glutamic acid, or aspartic acid.

The present invention also provides a CH1 region in which the amino acid at heavy chain position 183 is substituted with asparagine, and a nucleic acid encoding the region. The present invention also provides a heavy chain constant region having a CH1 region in which the amino acid at heavy chain position 183 is substituted with asparagine, and a nucleic acid encoding the region. The present invention also provides a heavy chain having a CH1 region in which the amino acid at heavy chain position 183 is substituted with asparagine, and a nucleic acid encoding the heavy chain. In this embodiment, the substitution with asparagine at heavy chain position 183 may be a substitution with glutamine, glutamic acid, or aspartic acid.

The present invention also provides a CL region in which the amino acid at light chain position 133 is substituted with glutamic acid and the amino acid at light chain position 176 is substituted with lysine, and a nucleic acid encoding the region. The present invention also provides a light chain comprising a CL region in which the amino acid at light chain position 133 is substituted with glutamic acid and the amino acid at light chain position 176 is substituted with lysine, and a nucleic acid encoding the light chain.

The present invention also provides a combination of a CH1 region in which the amino acid at heavy chain position 183 is substituted with asparagine or a nucleic acid encoding the region, and a CL region in which the amino acid at light chain position 133 is substituted with glutamic acid and the amino acid at light chain position 176 is substituted with lysine or a nucleic acid encoding the region. The present invention also provides a combination of a heavy chain constant region in which the amino acid at heavy chain position 183 is substituted with asparagine or a nucleic acid encoding the region, and a CL region in which the amino acid at light chain position 133 is substituted with glutamic acid and the amino acid at light chain position 176 is substituted with lysine or a nucleic acid encoding the region. The present invention also provides a combination of a heavy chain comprising a CH1 region in which the amino acid at heavy chain position 183 is substituted with asparagine or a nucleic acid encoding the heavy chain, and a light chain comprising a CL region in which the amino acid at light chain position 133 is substituted with glutamic acid and the amino acid at light chain position 176 is substituted with lysine or a nucleic acid encoding the light chain. In this embodiment, the substitution with asparagine at heavy chain position 183 may be a substitution with glutamine, glutamic acid, or aspartic acid.

The CH1 region may further have a substitution of the amino acid at position 173 with a hydrophobic amino acid residue (for example, isoleucine). The CH1 region may further have a substitution of the amino acid at position 181 in EU numbering with a hydrophobic amino acid residue (for example, leucine). In certain preferred embodiments, the CH1 region may further have a substitution of the amino acid at position 173 with isoleucine and a substitution of the amino acid at position 181 with, for example, leucine.

The CL region may further have a substitution of the amino acid at position 131 with a hydrophobic amino acid residue (for example, valine). The CL region may further comprise a substitution of the amino acid at position 162 with methionine. The CL region may further have a substitution of the amino acid at position 178 with a hydrophobic amino acid residue (for example, leucine). The CL region may further have a substitution of the amino acid at position 180 in Kabat numbering with glutamine. In certain preferred embodiments, the CL region may have a substitution at position 131 with valine, a substitution of the amino acid at position 162 with methionine, a substitution of the amino acid at position 178 with leucine, and a substitution of the amino acid at position 180 with glutamine. In certain preferred embodiments, for example, the antibody may be human IgG1, human IgG2, or human IgG4. In certain preferred embodiments, the light chain may be a human κ chain or a human λ chain.

In certain embodiments, the antibody of the present disclosure having an isopeptide bond may have stability equal to or greater than, or exceeding, that of an antibody having a heavy chain having the amino acid sequence set forth in SEQ ID NO: 61 and a light chain having the amino acid sequence of SEQ ID NO: 77. In certain embodiments, antigen-binding fragments (for example, formats such as Fab, Fab', and F(ab')₂ fragments) of the antibody of the present disclosure having an isopeptide bond may have stability equal to or greater than, or exceeding, that of an antigen-binding fragment in the corresponding format of an antibody having a heavy chain having the amino acid sequence set forth in SEQ ID NO: 61 and a light chain having the amino acid sequence of SEQ ID NO: 77. In certain embodiments, the antibody of the present disclosure having an isopeptide bond and antigen-binding fragments thereof may have a CH1 region in which the amino acid at heavy chain position 183 is substituted with asparagine, and a CL region in which the amino acid at light chain position 133 is substituted with glutamic acid and the amino acid at light chain position 176 is substituted with lysine. In certain embodiments, the antibody of the present disclosure having an isopeptide bond and antigen-binding fragments thereof may have at least a CH1 region in which the amino acid at heavy chain position 183 is substituted with asparagine, and at least a CL region in which the amino acid at light chain position 133 is substituted with glutamic acid and the amino acid at light chain position 176 is substituted with lysine. In this embodiment, the substitution with asparagine at heavy chain position 183 may be a substitution with glutamine, glutamic acid, or aspartic acid.

In all of the above embodiments, the amino acid at heavy chain position 183 may be glutamine or glutamic acid, the amino acid at light chain position 133 may be asparagine, and the amino acid at light chain position 176 may be lysine. Also, in all of the above embodiments, the amino acid at heavy chain position 170 may be glutamine or glutamic acid, the amino acid at heavy chain position 183 may be lysine, and the amino acid at light chain position 176 may be asparagine. In this embodiment, the substitution with asparagine at light chain position 176 may be a substitution with glutamine, glutamic acid, or aspartic acid.

In certain embodiments, the antibody of the present disclosure having an isopeptide bond may have stability or yield equal to or greater than, or exceeding, that of an antibody having a heavy chain having the amino acid sequence set forth in SEQ ID NO: 92 and a light chain having the amino acid sequence of SEQ ID NO: 94. In certain embodiments, antigen-binding fragments (for example, formats such as Fab, Fab', and F(ab')₂ fragments) of the antibody of the present disclosure having an isopeptide bond may have stability or yield equal to or greater than, or exceeding, that of an antigen-binding fragment in the corresponding format of an antibody having a heavy chain having the amino acid sequence set forth in SEQ ID NO: 92 and a light chain having the amino acid sequence of SEQ ID NO: 94. In certain embodiments, the antibody of the present disclosure having an isopeptide bond and antigen-binding fragments thereof may have a CH1 region in which heavy chain position 170 is substituted with glutamic acid and heavy chain position 183 is substituted with lysine, and a CL region in which light chain position 176 is substituted with asparagine. In certain embodiments, the antibody of the present disclosure having an isopeptide bond and antigen-binding fragments thereof may have at least a CH1 region in which heavy chain position 170 is substituted with glutamic acid and heavy chain position 183 is substituted with lysine, and at least a CL region in which light chain position 176 is substituted with asparagine.

In certain embodiments, a heavy chain CH1 region, a heavy chain variable region, and a heavy chain having an amino acid sequence of a CH1 region (particularly a region corresponding to positions 170 to 183 in EU numbering) of a sequence selected from the group consisting of SEQ ID NOs: 45, 47, 49, 53, 55, 57, 59, 60, 61, 62, 81, 83, 85, 88, 90, 91, and 92, and an antibody comprising any of these, or a nucleic acid encoding any of these, are provided.

In certain embodiments, a CL region, a light chain variable region, and a light chain having an amino acid sequence of a CL region (particularly a region corresponding to positions 176 to 180 (particularly a region corresponding to position 176 of mut3), for example, a region corresponding to positions 162 to 180, for example, a region corresponding to positions 131 to 180) of a sequence selected from the group consisting of SEQ ID NOs: 46, 48, 50, 54, 56, 58, 63, 64, 65, 66, 67, 68, 69, 70, 71, 72, 73, 74, 75, 76, 77, 78, 82, 84, 86, 87, 90, 93, and 94, and an antibody comprising any of these, or a nucleic acid encoding any of these, are provided.

In certain embodiments, a combination of a heavy chain CH1 region, a heavy chain variable region, and a heavy chain having an amino acid sequence of a CH1 region (particularly a region corresponding to positions 170 to 183 in EU numbering) of a sequence selected from the group consisting of SEQ ID NOs: 45, 47, 49, 53, 55, 57, 59, 60, 61, 62, 81, 83, 85, 88, 90, 91, and 92, and an antibody comprising any of these, or a nucleic acid encoding any of these; and
a CL region, a light chain variable region, and a light chain having an amino acid sequence of a CL region (particularly a region corresponding to positions 176 to 180) of a sequence selected from the group consisting of SEQ ID NOs: 46, 48, 50, 54, 56, 58, 63, 64, 65, 66, 67, 68, 69, 70, 71, 72, 73, 74, 75, 76, 77, 78, 82, 84, 86, 87, 90, 93, and 94, and an antibody comprising any of these, or a nucleic acid encoding any of these, may be provided.

In certain embodiments, a heavy chain CH1 region having an amino acid sequence of a CH1 region (particularly a region corresponding to positions 170 to 183 in EU numbering) of a sequence selected from the above group may be linked to various VH regions of antibodies.

In certain embodiments, a CL region having an amino acid sequence of a CL region (particularly a region corresponding to positions 176 to 180) of a sequence selected from the above group may be linked to various VL regions.

In certain embodiments, a heavy chain CH1 region having an amino acid sequence of a CH1 region (particularly a region corresponding to positions 170 to 183 in EU numbering) of a sequence selected from the above group may be linked to a hinge and CH2-CH3. In certain embodiments, a CL region having an amino acid sequence of a CL region (particularly a region corresponding to positions 176 to 180) of a sequence selected from the above group, rather than a CH1 region, may be linked to a hinge and CH2-CH3.

In the present invention, the heavy chain of the antibody may be derived from any one selected from the group consisting of human IgG1, human IgG2, human IgG3, and human IgG4, or an allotype thereof. Human IgG1, human IgG2, human IgG3, and human IgG4 may have, for example, SEQ ID NOs: 11 to 14, respectively. In the present invention, the light chain of the antibody may be derived from any one selected from the group consisting of a human κ chain and a human λ chain, or an allotype thereof. The human κ chain and the human λ chain may have, for example, SEQ ID NOs: 31 and 37, respectively. Accordingly, the antibody or antigen-binding fragment of an antibody of the present invention may be derived from the above human-derived heavy chain and human-derived light chain.

The heavy chain and light chain constant regions may each further have additional useful mutations, such as mutations (for example, selected from the group consisting of insertion, addition, substitution, and deletion) including silent Fc mutations, CH/CL interface control, and deletion of position 446 and/or position 447 (EU numbering) for improving C-terminal heterogeneity. Mutations to the heavy chain constant region are acceptable as long as they do not significantly inhibit antibody function (particularly expected antibody function), but may be equal to or less than 15%, equal to or less than 10%, or equal to or less than 5% of the number of amino acids contained in the sequence, based on the amino acid sequence of the heavy chain constant region contained in any of SEQ ID NOs: 11 to 14. Mutations to the light chain constant region are acceptable as long as they do not significantly inhibit antibody function (particularly expected antibody function), but may be equal to or less than 15%, equal to or less than 10%, or equal to or less than 5% of the number of amino acids contained in the sequence, based on the amino acid sequence of the light chain constant region contained in any of SEQ ID NOs: 31 and 37.

According to the present invention, a cell having the above nucleic acid (particularly an antibody-producing cell, preferably a Chinese hamster ovary cell), and a cell having a combination of the above nucleic acids (particularly an antibody-producing cell, preferably a Chinese hamster ovary cell) may be provided. According to the present invention, an antigen-binding fragment of an antibody comprising the above CH1 region and CL region is provided. Examples of the antigen-binding fragment include a Fab fragment, a Fab' fragment, or an F(ab')₂ fragment, or fragments comprising these. The antibody or antigen-binding fragment thereof may be fused with other proteins or the like. The antibody or antigen-binding fragment thereof may be multispecific (for example, bispecific).

According to the present invention, a chimeric antigen receptor (CAR) comprising a Fab fragment comprising the above CH1 region and CL region is provided. A CAR usually has scFv as an antigen-binding domain, but in the present invention, a CAR may comprise a Fab fragment comprising the above CH1 region and CL region instead of scFv. In these fragments and CAR, it is preferable that an isopeptide bond is formed between the CH1 region and the CL region. The isopeptide bond is formed intracellularly during CAR production, and therefore, a stable CAR having a Fab fragment or a Fab' fragment can be expressed on the cell surface. In the present invention, examples of cells expressing the above CAR include immune cells, for example, NK cells, T cells, macrophages, neutrophils, and the like. By using a Fab fragment or a Fab' fragment, a CAR with strong binding affinity for an antigen can be obtained relatively easily.

According to the present invention, a conjugate (ADC) of an antibody comprising a heavy chain comprising the above CH1 region and a light chain comprising the CL region, or an antigen-binding fragment thereof (for example, a Fab fragment and a Fab' fragment) with a cytotoxic agent is provided. This ADC preferably forms an isopeptide bond between the heavy chain comprising the above CH1 region and the light chain comprising the CL region, whereby the bond between the heavy chain and the light chain is stable even if the intermolecular SS bond is used for linking with the drug. The ADC may have a drug-to-antibody ratio of 1 to 8, for example, 4 to 8, preferably 6 to 8, and more preferably 8. In a preferred embodiment, an ADC having an isopeptide bond may have higher stability and/or higher cytotoxic activity than an ADC without it.

According to the present invention, a conjugate (ADC) of an antigen-binding fragment (for example, a Fab fragment, a Fab' fragment, an F(ab')₂ fragment, and the like) of an antibody comprising a heavy chain comprising the above CH1 region and a light chain comprising the CL region with a cytotoxic agent is provided. The same applies to conjugates (ADCs) of antigen-binding fragments (for example, Fab fragments, Fab' fragments, F(ab')₂ fragments, and the like) with cytotoxic agents, and by covalently linking CH1 and CL via an isopeptide bond, the bond between the heavy chain and the light chain is stable even if the intermolecular SS bond is used for linking with the drug. Accordingly, the introduction of an isopeptide bond may be particularly beneficial in ADCs.

According to the present invention, a peptide (first peptide) comprising an amino acid sequence including the amino acid at position 181 of the CH1 region, preferably an amino acid sequence including the amino acid at position 173 and the amino acid at position 181, is provided. According to the present invention, a peptide (second peptide) comprising an amino acid sequence including the amino acid at position 133 and the amino acid at position 176 of the CL region is also provided. These peptides can spontaneously form an isopeptide bond between the peptides by allowing them to coexist under physiological conditions. Accordingly, a stable bond can be formed between the first peptide and the second peptide. By fusing the first peptide and the second peptide as tags to target proteins, respectively, a plurality of target proteins can be caused to interact or be linked by an isopeptide bond. The first peptide and the second peptide may each be 5 amino acids in length or more, 10 amino acids in length or more, 15 amino acids in length or more, 20 amino acids in length or more, 30 amino acids in length or more, 40 amino acids in length or more, 50 amino acids in length or more, 60 amino acids in length or more, 70 amino acids in length or more, 80 amino acids in length or more, 90 amino acids in length or more, or 100 amino acids in length or more, or the full length of a CH1 region or a CL1 region. According to the present invention, a peptide may further comprise a heavy chain variable region (VH) or a light chain variable region (VL). In a preferred embodiment, the peptide comprises VH, CH1, VL, and CL regions and has all or a part of binding to an antigen.

Various antibody modifications are known, and any antibody modification may be introduced into an antibody comprising an isopeptide bond. For example, following EU numbering of the heavy chain with reference to FIG. 11, mutations promoting Fc heterodimerization include the mutation combinations of first chain: 354C and 366W; second chain: 349C, 366S, 368A, and 407V, the mutation combinations of first chain: 356K, 357K, and 399K; second chain: 370E, 409D, and 439E, the mutation combinations of first chain: 364H and 405A; second chain: 349T and 394F, the mutation combinations of first chain: 405L; second chain: 409R, the mutation combinations of first chain: 350V, 351Y, 400E, 405A, and 407V; second chain: 350V, 366L, 390R, 392M, and 394W, and the mutation combinations of first chain: 351D and 368E; second chain: 351K and 366K. Mutations enhancing effector function include the mutation combinations of 298A, 333A, and 334A, the mutation combinations of 239D, 330L, and 332E, the mutation combinations of 236A, 239D, and 332E, the mutation combinations of 236A, 330L, and 332E, the mutation combinations of 236A, 239D, 330L, and 332E, the mutation combinations of 243L, 292P, 300L, 305I, and 396L, the mutation combinations of 235V, 243L, 292P, 300L, and 396L, and the mutation combinations of first chain: 234Y, 235Q, 236W, 239M, 268D, 270E, and 298A; second chain: 270E, 326D, 330M, and 334E. Mutations enhancing CDC activity include the mutation combinations of 326W and 333S, the mutation combinations of 345R, 430G, and 440Y, and the mutation combinations of 236A, 267E, 268F, 324T, and 332E. Mutations enhancing binding activity to Fc-γ-RIIB include the mutation combinations of 267E and 328F, and the mutation combinations of 233D, 237D, 238D, 268D, 271G, and 330R. Mutations suppressing effector function include the single mutation of 297A, the single mutation of 297Q, the single mutation of 297G, the single mutation of 265A, the mutation combinations of 234A and 235A (LALA mutation), the mutation combinations of 235A, 237A, and 318A, the mutation combinations of 234A and 235A, the mutation combinations of 228P and 235E, the mutation combinations of 236R and 328R, the mutation combinations of 298G and 299A, the mutation combinations of 234F, 235E, and 331S, the mutation combinations of 268Q, 309L, 330S, and 331S, the mutation combinations of 233P, 234V, 235A, 236 deletion, and 267K, the mutation combinations of 234A, 235A, and 329G, the mutation combinations of 234A, 237A, 238S, 268A, 309L, 330S, and 331S, and the mutation combinations of 234F, 235E, and 265A, as well as combinations of these single mutations and/or mutation combinations. Mutations extending blood half-life include, but are not limited to, the mutation combinations of 252Y, 254T, and 256E, the mutation combinations of 250Q and 428L, the mutation combinations of 433K and 434F, the mutation combinations of 307A, 380A, and 434A, the mutation combinations of 428L and 434S, the single mutation of 308P, the mutation combinations of 252Y, 308P, and 434Y, the mutation combinations of 285D, 307Q, and 378V, and the mutation combinations of 309D, 311H, and 434S.

### (Performance of Polypeptide Comprising an Isopeptide Bond and Further Development)

Since an isopeptide bond can be stable and strong compared to other forms of interchain bonds such as disulfide bonds, it can confer beneficial effects on a polypeptide such as stability at high temperature, pH, high concentration, and/or high osmolarity (for example, reduction of aggregate formation), and resistance to degradation by enzymes and the like (for example, increase in plasma half-life). The stability of a polypeptide can be advantageous not only in the use of the polypeptide but also in the production of the polypeptide (for example, more stringent conditions can be used for purification). The high-concentration stability of a polypeptide can reduce the burden on patients by enabling the administration of the same amount of a therapeutic polypeptide in a smaller volume.

Since an isopeptide bond is formed between at least the different amino acids of a) and b), a polypeptide having formed an isopeptide bond has asymmetric heterochains. Therefore, the formation of complexes other than the intended complex, which is often problematic when associating different molecules to form a heterodimer, can be reduced. In particular, since it may be necessary for paired polypeptide chains to have structures specific to each other in order to form an isopeptide bond, even when a plurality of types of polypeptide chains comprising a) or b) coexist, the intended complex can be efficiently produced. Utilizing such advantages, complexes composed of three or more polypeptide chains (such as trispecific antibodies) can also be efficiently produced. DuetMab technology, which introduces a new disulfide bond at a site different from the original CH1-CL interchain disulfide bond to suppress mismatch between heavy chain and light chain, is known, but it has been reported that byproducts arise due to disulfide bond formation with the newly introduced Cys residues (Journal of Pharmaceutical Sciences 110 (2021) 2904-2915). In addition, knob-into-hole is a technology for forming complexes of specific pairs, but complexes using isopeptide bonds of the present disclosure have stronger binding force between pairs and can form complexes with a more compact domain. For example, an isopeptide bond may be formed between the constant regions of two heavy chains of a bispecific antibody.

In one embodiment, the polypeptide comprising an isopeptide bond of the present disclosure is a polypeptide molecule comprising at least two (for example, 2, 3, 4, 5) isopeptide bonds. In one embodiment, the polypeptide comprising an isopeptide bond of the present disclosure is a polypeptide molecule in which at least three (for example, 3, 4, 5, 6) different polypeptide molecules are linked by isopeptide bonds. In one embodiment, the polypeptide comprising an isopeptide bond of the present disclosure is a polypeptide molecule in which different polypeptide molecules are linked by isopeptide bonds, and one of the different polypeptide molecules is bound to another different polypeptide molecule by at least two (for example, 2, 3, 4, 5) isopeptide bonds.

In one embodiment, a protein that is naturally expressed as a single molecule can be expressed as mutually different polypeptide molecules by removal of a linker region and the like, and the three-dimensional structure of the natural protein can be maintained by isopeptide bonds.

It was considered that disulfide bonds in known polypeptides have a function of stabilizing the structure of the polypeptide, and that the proximity of polypeptide chains at the disulfide bond formation site may be lost when the disulfide bond is removed; however, the inventors unexpectedly found that an isopeptide bond can be introduced to replace a disulfide bond. In one embodiment, in the polypeptide comprising an isopeptide bond of the present disclosure, an isopeptide bond is introduced to replace a disulfide bond. For example, two cysteine residues that form a disulfide bond are identified in the three-dimensional structure of a known polypeptide, and at least one of the two cysteine residues is deleted. Here, by removing the disulfide bond-forming cysteine residues, the formation of unintended complexes by disulfide bonds (which enable symmetric reactions) can be prevented. Since parts that form disulfide bonds in known polypeptides are considered to come into proximity before disulfide bond formation with or without proteins that assist in folding, such as chaperones, by introducing a) and/or b) near the disulfide bond formation site, the proximity of a) and b) can be promoted by a mechanism similar to that of the polypeptide before modification. In addition, by introducing an isopeptide bond as an alternative to a disulfide bond, the higher-order structure of the protein can be maintained and the function can be maintained. In one embodiment, a) and/or b) are introduced into an interface region between two domains that are in contact with each other through a disulfide bond. In one embodiment, a) and/or b) are introduced near the disulfide bond formation site (for example, at a residue present within 5 residues, within 4 residues, within 3 residues, within 2 residues, or within 1 residue from a cysteine involved in disulfide bond formation).

In one embodiment, the polypeptide comprising an isopeptide bond of the present disclosure is expressed intracellularly, and at least one of the different polypeptide molecules linked by the isopeptide bond is expressed from a transgene. For example, a CAR-T cell expressing a chimeric antigen receptor comprising a Fab-type antibody as the polypeptide comprising an isopeptide bond of the present disclosure is provided.

In one embodiment, the method for producing a polypeptide comprising an isopeptide bond of the present disclosure further produces a secondary modified polypeptide comprising an interpeptide linkage represented by the following formula between the modified polypeptide and a third polypeptide chain contained in a molecule different from the modified polypeptide: 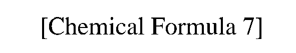 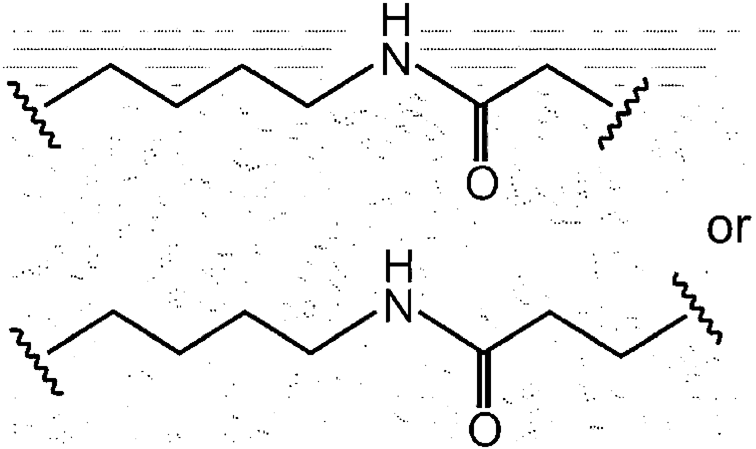 (wherein the dashed lines represent bonds to the polypeptide backbone), further comprising:
a step of constructing a three-dimensional structure of a secondary intermediate A comprising the modified polypeptide and the third polypeptide chain;
a step of identifying amino acid sequences present at the interface region where the modified polypeptide and the third polypeptide chain in the three-dimensional structure are in close proximity to each other;
a step of mutating the secondary intermediate A to introduce into Secondary intermediate A at least one residue of:
   a) lysine,
   b) glutamine, glutamic acid, asparagine, or aspartic acid, and
   c) glutamic acid or aspartic acid,
thereby preparing a secondary intermediate B, wherein the secondary intermediate B comprises a), b), and c), and the modified polypeptide and the third polypeptide chain in the secondary intermediate B respectively comprise a) and b) in the interface region; and
a step of obtaining the secondary modified polypeptide by forming the interpeptide linkage in the secondary intermediate B.
Similarly, by designing interpeptide linkages between a fourth polypeptide chain, a fifth polypeptide chain, and so on, polypeptides in which even more polypeptide chains are linked by isopeptide bonds can be produced.

A person skilled in the art can produce the polypeptide chains that form the polypeptide comprising the isopeptide bond described in the present specification using any system.

The produced polypeptide can be purified or isolated by any purification method. Examples include size fractionation, fractionation by salting out using ammonium sulfate or sodium sulfate, PEG fractionation, ethanol fractionation, and immunoglobulin purification methods such as DEAE ion exchange chromatography, gel filtration, and protein A/G affinity chromatography. In particular, when the monoclonal antibody is mouse IgG, the monoclonal antibody can be efficiently purified by affinity chromatography using a protein A-bound carrier or an anti-mouse immunoglobulin-bound carrier.

### (Uses/Usage)

The polypeptide comprising the isopeptide bond of the present disclosure can be used for known uses of polypeptides, and for example, when the polypeptide is an antibody, it can be used for detection of an antigen and/or treatment or prevention of a disease associated with the antigen. In one aspect, the present disclosure provides use of the polypeptide comprising the isopeptide bond of the present disclosure. In one aspect, the present disclosure provides a composition comprising the polypeptide comprising the isopeptide bond of the present disclosure. The composition can be used for known uses of polypeptides.

### (Dosage Form, etc.)

The composition described in the present specification can be provided in various forms. The form of the composition may be, for example, an injection, a capsule, a tablet, a granule, or the like. An aqueous solution for injection may be stored in, for example, a vial or a stainless steel container. The aqueous solution for injection may also be formulated with, for example, physiological saline, a sugar (for example, trehalose), NaCl, or NaOH, or the like.

In one embodiment, the composition of the present disclosure comprises a pharmaceutically acceptable carrier or excipient. Such carriers can also be sterile liquids, for example water and oils, including those of petroleum, animal, vegetable, or synthetic origin, including, but not limited to, peanut oil, soybean oil, mineral oil, sesame oil, and the like. When the pharmaceutical is administered orally, water is a preferred carrier. When the pharmaceutical composition is administered intravenously, physiological saline and aqueous dextrose are preferred carriers. Preferably, physiological saline solutions, and aqueous dextrose and glycerol solutions are used as liquid carriers for injectable solutions. Suitable excipients include light anhydrous silicic acid, microcrystalline cellulose, mannitol, starch, glucose, lactose, sucrose, gelatin, malt, rice, wheat flour, chalk, silica gel, sodium stearate, glycerol monostearate, talc, sodium chloride, nonfat dry milk, glycerol, propylene glycol, water, ethanol, carmellose calcium, carmellose sodium, hydroxypropyl cellulose, hydroxypropyl methylcellulose, polyvinyl acetal diethylaminoacetate, polyvinylpyrrolidone, gelatin, medium-chain fatty acid triglyceride, polyoxyethylene hydrogenated castor oil 60, white sugar, carboxymethyl cellulose, corn starch, inorganic salts, and the like. The composition can also contain small amounts of wetting agents or emulsifying agents, or pH buffering agents, if desired. These compositions can also take the form of solutions, suspensions, emulsions, tablets, pills, capsules, powders, sustained-release formulations, and the like. The composition can also be formulated as a suppository using traditional binders and carriers, such as triglycerides. Oral formulations can also include standard carriers such as pharmaceutical grade mannitol, lactose, starch, magnesium stearate, sodium saccharin, cellulose, magnesium carbonate, and the like. Examples of suitable carriers are described in E. W. Martin, Remington's Pharmaceutical Sciences (Mark Publishing Company, Easton, U.S.A). In addition to these, the composition may also comprise, for example, surfactants, excipients, colorants, flavoring agents, preservatives, stabilizers, buffering agents, suspending agents, isotonizing agents, binders, disintegrants, lubricants, fluidity enhancers, corrigents, and the like. In one embodiment, the pH of any liquid composition of the present disclosure can be in the range of about 3, about 3.5, about 4, about 4.5, about 5, about 5.5, about 6, about 6.5, about 7, about 7.5, about 8, about 8.5, about 9, about 9.5, about 10, about 10.5, about 11, or between any two of these values.

Any component of the composition of the present disclosure can be provided as a pharmaceutically acceptable salt, and can be, for example, salts formed with free carboxyl groups derived from hydrochloric acid, phosphoric acid, acetic acid, oxalic acid, tartaric acid, and the like, salts formed with free amine groups such as those derived from isopropylamine, triethylamine, 2-ethylaminoethanol, histidine, procaine, and the like, and salts formed with sodium, potassium, ammonium, calcium, and ferric hydroxide.

In a preferred embodiment, the composition can be formulated as a pharmaceutical composition adapted for administration to humans in accordance with known methods. Such compositions can be administered by injection. Typically, compositions for injection administration are solutions in sterile isotonic aqueous buffers. Where necessary, the composition can also include a solubilizing agent and a local anesthetic such as lidocaine to alleviate pain at the injection site. Generally, the ingredients can be supplied either separately, or mixed together in unit dosage form, and supplied, for example, as a lyophilized powder or water-free concentrate in a sealed container such as an ampoule or sachet indicating the amount of active ingredient. Where the composition is to be administered by infusion, it can also be dispensed with an infusion bottle containing sterile pharmaceutical grade water or physiological saline. Where the composition is to be administered by injection, an ampoule of sterile water for injection or physiological saline can be provided so that the ingredients can be mixed prior to administration.

When the composition of the present disclosure is applied to a subject, the subject is not particularly limited, and can be a mammal (for example, mouse, rat, hamster, rabbit, cat, dog, cow, sheep, pig, monkey, human, and the like), a bird, a reptile, an amphibian, an arthropod, a fish, and the like.

The amount of the composition and/or active ingredient of the present disclosure may vary depending on the nature of the disorder or condition to be treated or prevented, but can be determined by a person skilled in the art by standard clinical techniques based on the description of the present specification. In some cases, in vitro assays can also be used to assist in identifying optimal dosage ranges. The exact dose to be used in the formulation will also vary depending on the route of administration and the severity of the disease or disorder, and should be determined in accordance with the judgment of the attending physician and the circumstances of each patient. The dosage of the composition and/or active ingredient of the present disclosure is not particularly limited, but may be, for example, 0.00001, 0.0001, 0.001, 1, 5, 10, 15, 100, or 1000 mg/kg body weight per dose, and may be within the range of any two of those values. The dosing interval is not particularly limited, but may be, for example, one or two administrations per 1, 7, 14, 21, or 28 days, and may be one or two administrations per the range of any two of those values. The dosage, dosing interval, and administration method may be appropriately selected depending on the age and body weight of the patient, symptoms, target organ, and the like.

The route of administration of the composition described in the present specification may be, for example, intravenous, intradermal, subcutaneous, intramuscular, intraperitoneal, intranasal, epidural, or oral administration, or the like. In one embodiment, the composition of the present disclosure can be used together with various delivery systems. Such systems include, for example, encapsulation in liposomes, microparticles, and microcapsules; use of receptor-mediated endocytosis; construction of therapeutic nucleic acids as part of retroviral vectors or other vectors, and the like. The pharmaceutical can also be administered by any suitable route, for example by infusion, by bolus injection, by absorption through epithelial or mucocutaneous linings (such as oral, rectal, and intestinal mucosa, and the like), by using inhalers or nebulizers with an aerosolizing agent as needed, and can also be administered together with other agents. Administration can also be systemic or local.

The composition of the present disclosure can be provided as a kit. In one embodiment, the present disclosure provides a pharmaceutical pack or kit comprising one or more containers filled with one or more components that can be added to the composition of the present disclosure. In some cases, information indicating approval by a governmental agency for manufacturing, use, or sale for human administration can also be presented in association with such containers, in the form prescribed by a governmental agency that regulates the manufacturing, use, or sale of pharmaceuticals or biological products.

Procedures for formulating the composition of the present disclosure as a pharmaceutical or the like are known in the art, and are described in, for example, the Japanese Pharmacopoeia, the United States Pharmacopeia, pharmacopoeias of other countries, and the like. Therefore, a person skilled in the art can determine embodiments such as the amount to be used without undue experimentation, given the description of the present specification.

The antibody of the present disclosure has intrachain mutations for introducing the isopeptide bond (for example, amino acid substitution mutations as described above), but may further have various mutations other than said mutations, as long as the antibody function (for example, expected antibody function) is not significantly inhibited or lost. The mutations can be selected and introduced as appropriate depending on the purpose, but since mutations may generate the possibility of increasing the antigenicity of the antibody, it is desirable to minimize them. For example, mutations that can be further introduced into the antibody include, for example, mutations for reducing binding ability to Fcγ receptors (binding ability to any of Fcγ receptors FcγI, FcγIIA, FcγIIB, FcγIIIA, and FcγIIIB) (i.e., silent Fc mutations), mutations to amino acids at positions 297 and 299 for suppressing or eliminating glycosylation, mutations for improving C-terminal heterogeneity (for example, deletion of G446 and/or K447), and the like.

When the antibody is a bispecific antibody, without being particularly limited, for example, mutations for hetero Fc association (for example, knobs-into-holes mutations, and the like), mutations for CH/CL interface control, and mutations for reducing binding to protein A could also be introduced.

In a bispecific antibody having two Fab-type arms, an isopeptide bond can be formed in the CH1-CL interface of one of the Fab-type arms. This can promote complex formation between the correct heavy chain and light chain.

As used in the present specification, "or" is used when "at least one or more" of the items listed in the sentence can be adopted. The same applies to "alternatively." In the present specification, when "within the range" of "two values" is explicitly stated, that range also includes the two values themselves.

The references such as scientific literature, patents, and patent applications cited in the present specification are incorporated herein by reference in their entirety to the same extent as if each were specifically described.

The present disclosure has been described above by showing preferred embodiments for ease of understanding. The present disclosure is described below based on examples, but the above description and the following examples are provided only for the purpose of illustration and are not provided for the purpose of limiting the present disclosure. Therefore, the scope of the present disclosure is not limited to the embodiments or examples specifically described in the present specification, and is limited only by the claims.

### [Examples]

Reagents used were specifically the products described in the Examples, but equivalent products from other manufacturers (Sigma-Aldrich, Wako Pure Chemical, Nacalai, R&D Systems, USCN Life Science INC, etc.) can also be substituted.

### (Example 1: Design of Isopeptide Bond-Forming Trastuzumab IgG)

Formation of an isopeptide bond between the heavy chain and light chain of an antibody was attempted. Anti-HER2 antibody h4D5 (trastuzumab) was used as the starting protein.

Coordinate data of the trastuzumab Fab-HER2 complex crystal structure (PDB ID: 1N8Z) registered in the Protein Data Bank (https://www.rcsb.org/) were downloaded, and a three-dimensional structure was constructed using PyMOL (https://pymol.org/2/).

Based on the three-dimensional structure constructed above, a region where the constant region of the heavy chain and the constant region of the light chain are in close proximity to each other was identified, and asparagine and lysine were introduced into this region. Asparagine was introduced into isoCH1, and lysine was introduced into isoCL. Next, hydrophobic amino acids were introduced so that the surroundings of the side chains of the introduced asparagine and lysine would become a hydrophobic environment. In addition, the cysteines involved in the formation of the disulfide bond were replaced with serine. Here, a homology model was constructed as appropriate by SWISS-MODEL (https://swissmodel.expasy.org/) based on sequence data, and a structural model into which the isopeptide bond was introduced was produced using Coot (https://www2.mrc-lmb.cam.ac.uk/personal/pemsley/coot/).

As a result, the following sequences were designed. In the present Example, the polypeptide modified for the formation of an isopeptide bond may be referred to as "iso-" or "iSo-" (isoCH1, iSoMAb, etc.). MAb means monoclonal antibody.

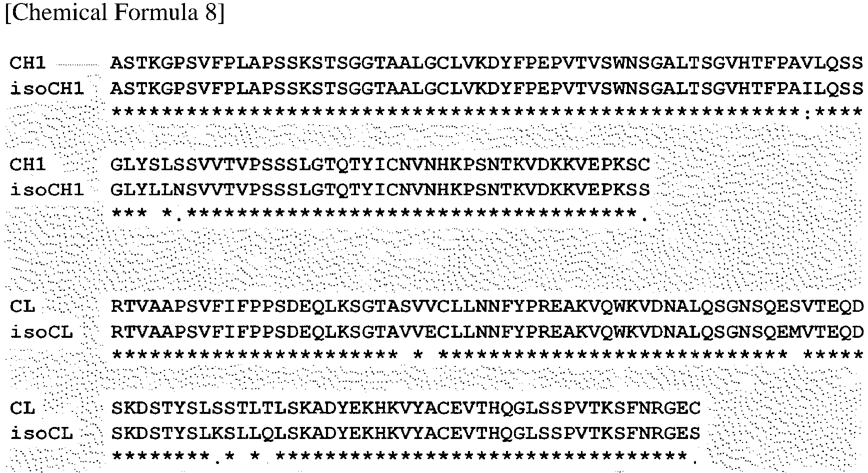

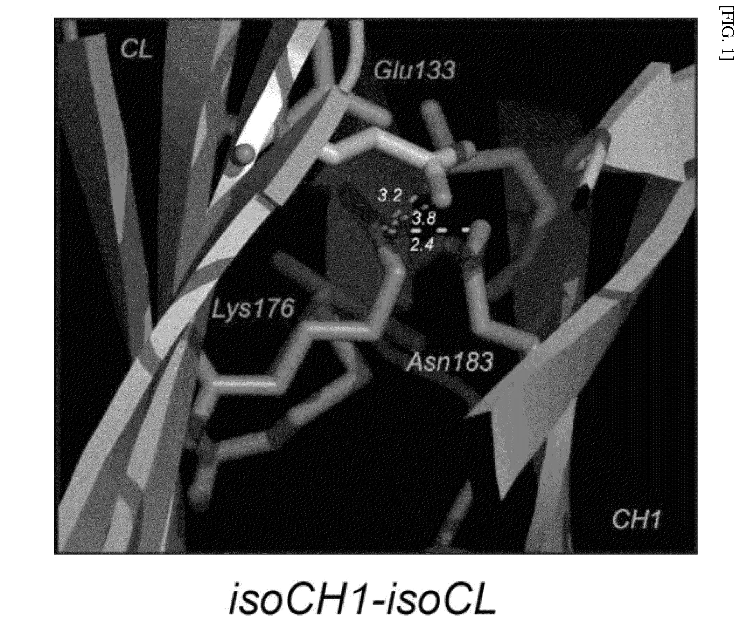

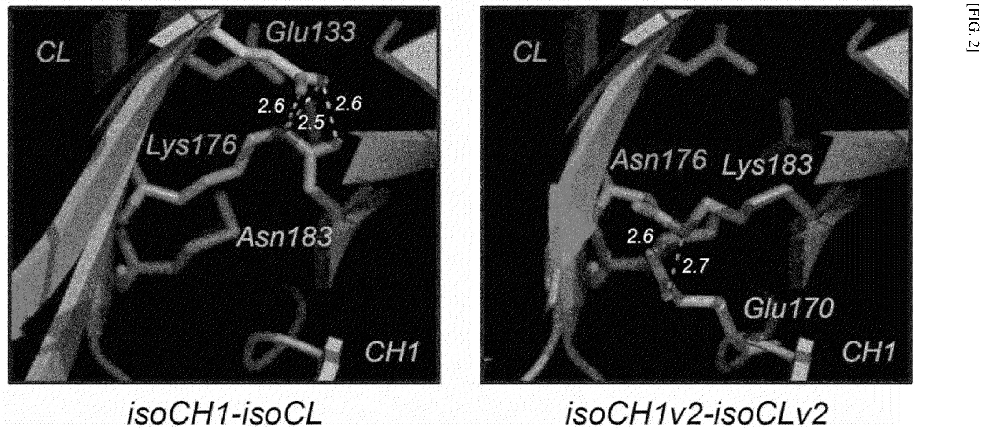

When the structure of the antibody after introduction of the above mutations was constructed by recalculation, the side chains of asparagine, lysine, and glutamic acid involved in isopeptide bond formation were arranged as shown in FIG. 1. In addition, the result of recalculating the three-dimensional structure after isopeptide bond formation is shown in FIG. 2.

### (Example 2: Production and Evaluation of Binding Activity of Isopeptide Bond-Forming Trastuzumab IgG)

The polypeptide designed as described above was produced and evaluated.

### (Production of iSoMAb of isoCH1 and isoCL)

### - Preparation of h4D5 iSoMAb IgG expression vector

Mutant CH1 (isoCH1) and CL (isoCL) genes were synthesized by commissioning Eurofins Genomics. The isoCH1 gene and the hinge-Fc gene were linked by Overlap Extension PCR, and digested with restriction enzymes Nhe I and Not I. The product was inserted into pCAGEN containing the h4D5VH gene that had been previously digested with the same enzymes to produce expression vector pCA h4D5 isoHC. In addition, the h4D5VL gene and the isoCL gene were linked by Overlap Extension PCR, and digested with restriction enzymes Eco RI and Not I. The product was inserted into pCAGEN that had been previously digested with the same enzymes to produce expression vector pCA h4D5 isoLC.

### - Expression of h4D5 iSoMAb IgG

Expi293F cells (Gibco, Thermo Fisher Scientific) were used as host cells. Expi293F cells were cultured using HE200 medium (Gmep) containing 2 mM L-alanyl-L-glutamine under conditions of 37 °C, 5% CO2, and 125 rpm. 7.5 x 10⁷ cells were collected by centrifugation at 1,500 rpm for 5 minutes and suspended in 27 mL of HE400 medium (Gmep) containing 5 mM L-alanyl-L-glutamine. 30 µg of plasmid (15 µg of pCA h4D5 isoHC and 15 µg of pCA h4D5 isoLC) was added to 1.5 mL of Opti-MEM medium (Gibco) and mixed. In addition, 120 µl of PEI MAX (1 mg/mL; Polysciences) was added to 1.5 mL of Opti-MEM medium and mixed, and the mixture was left to stand at room temperature for 5 minutes. After mixing the plasmid dilution and the PEI dilution, the mixture was left to stand at room temperature for 20 minutes, and this was used as the transfection solution. The transfection solution was added to Expi293F cells suspended in HE400 medium and cultured. After 20 hours, 75 µl of 0.5 M sodium valproate, 120 µl of 1 M sodium propionate, and 750 µl of 20% (w/v) tryptone were added, and the cells were cultured for an additional 6 days.

### - Purification of h4D5 iSoMAb IgG

Cells were removed from the culture supernatant by centrifugation at 4 °C, 6,000 rpm for 10 minutes. The culture supernatant was filtered using a filter with a pore size of 0.45 µm (Membrane Solutions) and applied to a protein A column (column volume 250 µl; Bipo Resin Protein A; Protein Express) that had been previously equilibrated with phosphate-buffered saline (PBS). After washing with 10 mL of PBS, the target antibody was eluted with 100 mM glycine-HCl (pH 2.5). Fractions of 200 µl each were collected and neutralized with 10 µl of 100 mM Tris-HCl (pH 9.0) that had been previously added to sample tubes. Each fraction was analyzed by SDS-PAGE, and fractions containing the target antibody were dialyzed against PBS. Absorbance at 280 nm was measured with a spectrophotometer (NanoPhotometer NP80; Implen), and the absorbance at 320 nm was subtracted as background to quantify the target antibody. The calculation was performed using a molar extinction coefficient of 195400 M⁻¹ cm⁻¹ per tetramer. The purified antibody was further analyzed by Western blotting. HRP-labeled anti-human Fc antibody (Sigma) and HRP-labeled anti-human kappa chain antibody (Sigma) were used as detection antibodies.

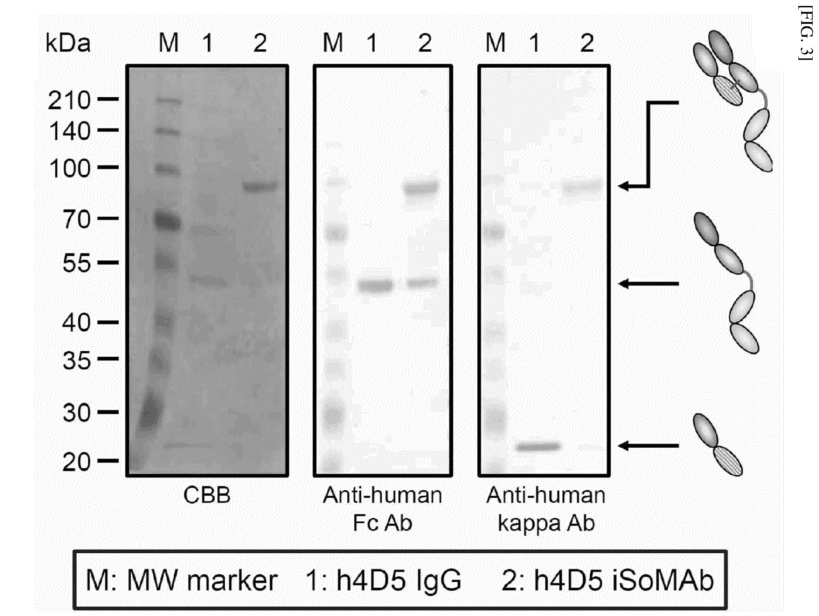

The results are shown in FIG. 3. Since the disulfide bond has been reductively cleaved, the complex of heavy chain and light chain is not formed in wild-type h4D5 IgG. On the other hand, in h4D5 iSoMAb, a band was confirmed at the target molecular weight portion of the complex of heavy chain and light chain, and it is considered that an isopeptide bond has been formed between the heavy chain and the light chain.

### - Evaluation of binding activity against HER2-positive cells

Binding activity against the HER2-positive human breast cancer cell line SKBR-3 was evaluated by flow cytometry. SKBR-3 cultured in 10% FBS/D-MEM medium (Wako) under conditions of 37 °C and 5% CO2 was detached from the culture dish using trypsin/EDTA (0.25% (w/v) trypsin-1 mM EDTA-4Na; Gibco). Cells were collected by centrifugation at room temperature, 1,000 rpm for 5 minutes, and 5 x 10⁵ cells were dispensed into sample tubes. After washing with 0.1% NaN3/PBS, h4D5 iSoMAb IgG at a final concentration of 500 nM was reacted as a primary antibody on ice for 30 minutes. After washing in the same manner, 1 µl of anti-human IgG (Fc-specific)-FITC antibody (2 mg/mL; Sigma) and 499 µl of 0.1% NaN3/PBS were added as a secondary antibody, and the mixture was reacted on ice for 30 minutes. After washing again, the cells were suspended in 500 µl of 0.1% NaN3/PBS. After filtration through a nylon filter, the suspension was dispensed into plastic tubes and analyzed using a flow cytometer (Accuri C6; BD Biosciences).

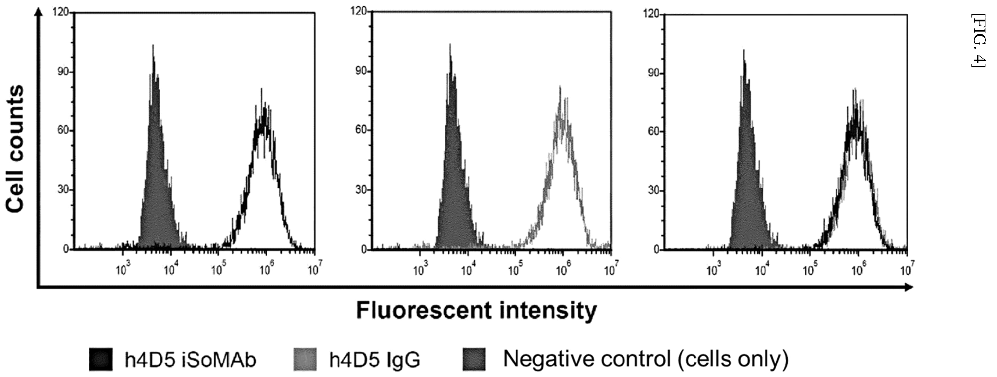

The results are shown in FIG. 4. It was found that iSoMAb has HER2-binding ability similar to that of the original antibody.

### (Example 3: Production of Isopeptide Bond-Forming Antibody in Fab Format)

An antibody fragment in Fab format was produced based on the iSoMAb produced above and evaluated.

### - Preparation of h4D5 iSoMAb Fab heavy chain expression vector

Using the synthesized isoCH1 gene as a template, a histidine tag sequence was added to the C-terminal side of the CH1 region by PCR. The PCR product was digested with restriction enzymes Nhe I and Not I. The product was inserted into pCAGEN containing the h4D5VH gene that had been previously digested with the same enzymes to produce expression vector pCA h4D5VH-isoCH1.

### - Expression of h4D5 iSoMAb Fab

h4D5 iSoMAb Fab was expressed using the same method as described above. pCA h4D5VH-isoCH1 and pCA h4D5 isoLC were used as plasmids for transfection.

### - Purification of h4D5 iSoMAb Fab

Cells were removed from the culture supernatant by centrifugation at 4 °C, 6,000 rpm for 10 minutes. The culture supernatant was filtered using a filter with a pore size of 0.45 µm (Membrane Solutions) and applied to Ni Sepharose excel (column volume 500 µl; Cytiva) that had been previously equilibrated with 20 mM sodium phosphate/500 mM NaCl (pH 7.4). After washing with 10 mL of 20 mM sodium phosphate/500 mM NaCl (pH 7.4) containing 30 mM imidazole, the target antibody was eluted with 20 mM sodium phosphate/500 mM NaCl (pH 7.4) containing 500 mM imidazole. Fractions of 200 µl each were collected, each fraction was analyzed by SDS-PAGE, and fractions containing the target antibody were dialyzed against PBS. Absorbance at 280 nm was measured with a spectrophotometer (NanoPhotometer NP80), and the absorbance at 320 nm was subtracted as background to quantify the target antibody. The calculation was performed using a molar extinction coefficient of 64900 M⁻¹ cm⁻¹ per dimer.

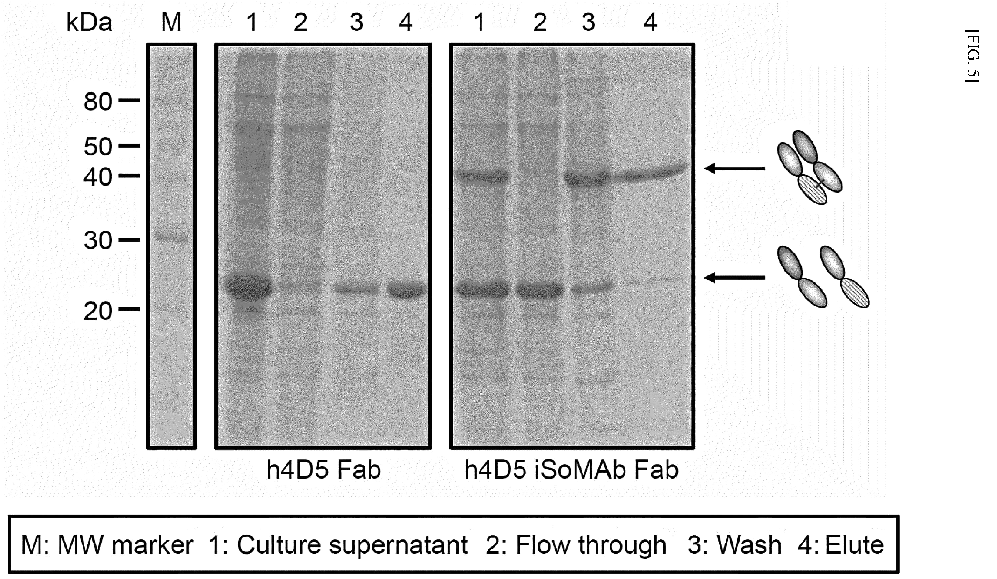

The results are shown in FIG. 5. Since the disulfide bond has been reductively cleaved, the complex of heavy chain and light chain is not formed in wild-type h4D5 Fab. On the other hand, in h4D5 iSoMAb Fab, a band was confirmed at the target molecular weight portion of the complex of heavy chain and light chain, and it is considered that an isopeptide bond has been formed between the heavy chain and the light chain.

### - Evaluation of thermal stability by differential scanning fluorimetry

To 70 µl of an antibody solution at 0.1 mg/mL, 17.5 µl of a 50-fold diluted fluorescent dye (SYPRO (registered trademark) dye, protein gel stain; Merck) was added and mixed. 25 µl each of the antibody-dye mixed solution was dispensed into 3 wells of a 48-well plate for PCR. Using a Real-Time PCR instrument, the temperature was raised from 25 °C to 99 °C over approximately 40 minutes, and fluorescence intensity was measured. In addition, to examine thermal stability under reducing conditions, TCEP (tris(2-carboxyethyl)phosphine) was added to the antibody solution at a final concentration of 1 mM, and the mixture was left to stand on ice for 3 hours, after which measurement was performed in the same manner.

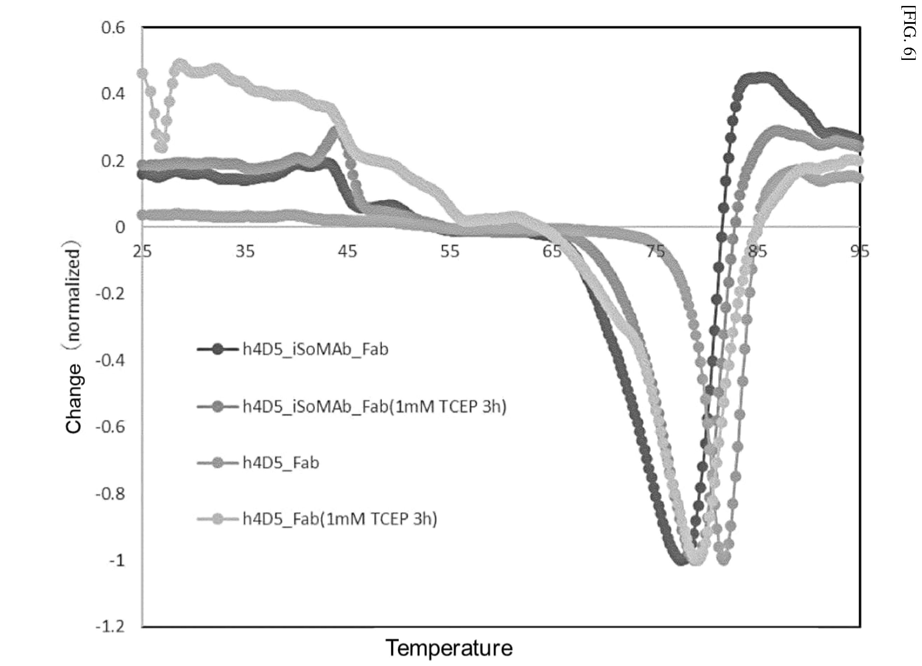

The results are shown in FIG. 6 and the table below. Although a slight decrease in thermal stability was observed for h4D5 iSoMAb Fab compared with h4D5 Fab, the decrease in thermal stability was not to the extent that would hinder purification or use in vivo. In addition, unlike h4D5 Fab, h4D5 iSoMAb Fab showed no decrease in stability under reducing conditions (in the presence of TCEP), confirming that the isopeptide bond is resistant to cleavage under reducing conditions.

**[Table 2]**

| Antibody | h4D5 iSoMAb Fab | | h4D5 Fab | |
|---|---|---|---|---|
| Additive | Free | 1 mM TCEP | Free | 1 mM TCEP |
| Tm (° C) | 77.6 | 79.0 | 81.8 | 79.2 |

### (Example 4: Design of Isopeptide Bond-Forming Anti-CD63 Antibody)

Similarly to the above example based on anti-HER2 antibody h4D5, production of a modified antibody in which the heavy chain and the light chain were linked by an isopeptide bond was attempted based on an anti-CD63 antibody (clone No. 2 described in Monoclon. Antib. Immunodiagn. Immunother. 39, 74-76 (2020)).

A three-dimensional model was constructed in the same manner as in Example 1, and based thereon, the following sequences were designed. Asparagine was introduced into isoCH1v1, and lysine was introduced into isoCLv1. Conversely, lysine was introduced into isoCH1v2, and asparagine was introduced into isoCLv2. Next, glutamic acid was introduced such that its side chain would be in proximity to the side chains of the introduced asparagine and lysine.

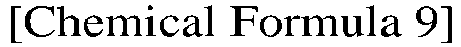

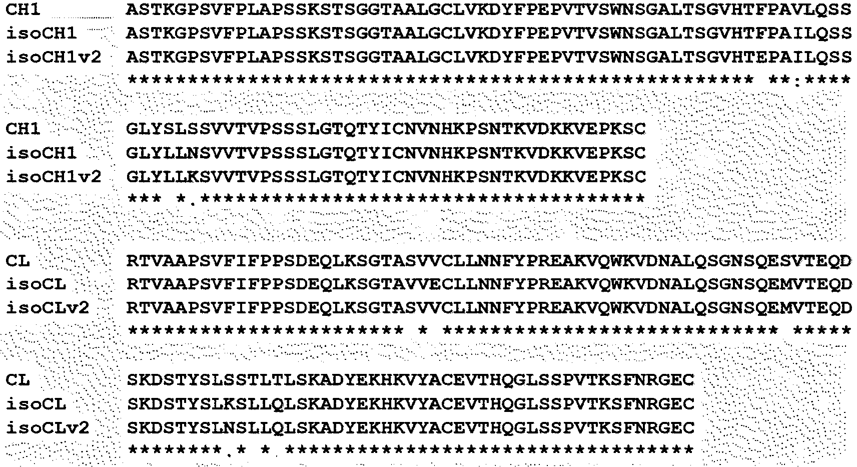

The positional relationships between the atoms of a), b), c), and hydrophobic mutation residues in the three-dimensional structure were as follows.

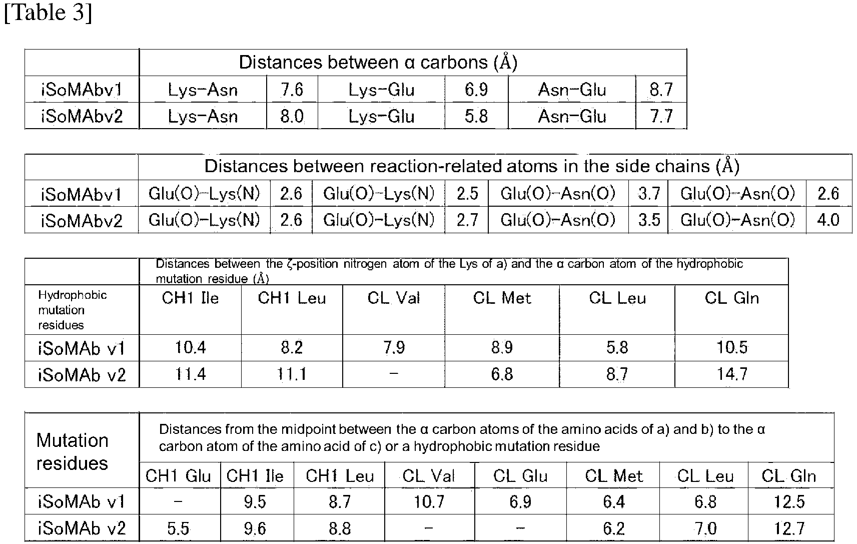

### - Preparation of hCD63 iSoMAb IgG expression vector

Mutant CH1 (isoCH1)-containing IgG and CL (isoCL) genes were synthesized by commissioning Eurofins Genomics. The synthesized genes were each inserted into pCEC4.3W by seamless cloning to produce pCEC4.3W-isoCH1vC (Af1 II) or pCEC4.3W-isoCLvC (BsrG I). This vector was digested with restriction enzyme Afl II or BsrG I, and the humanized hCD63 antibody genes were each inserted for the heavy chain and light chain by seamless cloning to produce expression vectors pCEC4.3W-isoCH1vC hCD63 H1 and pCEC4.3W-isoCLvC hCD63 K1.

### - Expression of hCD63 iSoMAb IgG

ExpiCHO-S cells (Gibco, Thermo Fisher Scientific) were used as host cells. ExpiCHO-S cells were cultured using ExpiCHO Expression Medium (Gibco, Thermo Fisher Scientific) under conditions of 37 °C, 5% CO2, and 125 rpm. The culture method and transfection method followed the protocol of the ExpiCHO Expression System (Gibco, Thermo Fisher Scientific). 18.0 x 10⁷ cells were collected by centrifugation at 300 x g for 5 minutes and suspended in 30 mL of ExpiCHO Expression Medium. 24 µg of plasmid (12 µg of pCEC4.3W-isoCH1vC hCD63 H1 and 12 µg of pCEC4.3W-isoCLvC hCD63 K1) was added to 1.2 mL of OptiPRO SFM Complexation Medium (Gibco, Thermo Fisher Scientific) and mixed. In addition, 96 µl of ExpiFectamine CHO Reagent (Gibco, Thermo Fisher Scientific) was added to 1.104 mL of OptiPRO SFM Complexation Medium and mixed, and the plasmid dilution and the ExpiFectamine (registered trademark) CHO Reagent dilution were mixed, after which the mixture was left to stand at room temperature for 5 minutes, and this was used as the transfection solution. The transfection solution was added to CHO-S cells suspended in ExpiCHO Expression Medium and cultured. After 18 hours, 180 µl of ExpiFectamine (registered trademark) CHO Enhancer and 7.2 mL of ExpiCHO (registered trademark) Feed were added, and the cells were cultured for an additional 12 days.

### - Purification of hCD63 iSoMAb IgG

Cells were removed from the culture supernatant by centrifugation at 4 °C, 12,000 x g for 10 minutes. The culture supernatant was filtered using a filter with a pore size of 0.45 µm (GVS), and 1.0 mL of protein A Sepharose that had been previously equilibrated with PBS buffer (PBS) was added to the filtered supernatant. After reaction with stirring at 4 °C overnight, the gel was added together to a column. After washing with 30 mL of PBS, the target antibody was eluted with 0.1 M citric acid eluent (pH 3.0). Fractions of 1.0 mL each were collected, each fraction was analyzed by SDS-PAGE, and fractions containing the target antibody were solvent-exchanged to PBS using Pierce (registered trademark) Protein Concentrators PES, 10K MWCO, 2-6 ml. Absorbance at 280 nm was measured with a spectrophotometer (Eppendorf BioPhotometer plus), and the absorbance at 340 nm was subtracted as background to quantify the target antibody. Thereafter, a more accurate concentration was determined using Pierce (registered trademark) BCA Protein Assay Kits.

### - Evaluation of binding activity against hCD63-positive cells

Binding activity against a human fetal kidney-derived cell line 293 strain, which is hCD63-positive, was evaluated by flow cytometry. Expi293F cells cultured in Expi293 Expression Medium (Gibco, Thermo Fisher Scientific) under conditions of 37 °C, 5% CO2, and 125 rpm were collected by centrifugation at room temperature, 300 x g for 5 minutes, and 5 x 10⁵ cells were dispensed into sample tubes. After washing with 0.2% BSA/PBS, hCD63 iSoMAb IgG at a final concentration of 2 µg/mL was reacted as a primary antibody at 4 °C for 60 minutes. After washing in the same manner, goat F(ab')2 anti-human IgG-Fc (PE), pre-adsorbed (ab98596, Abcam) at a final concentration of 0.5 µg/mL was reacted as a secondary antibody at 4 °C for 30 minutes. After washing again, the cells were suspended in 200 µl of 0.2% BSA/PBS. The suspension was dispensed into plastic tubes and analyzed using a flow cytometer (FACS Verse; BD Biosciences).

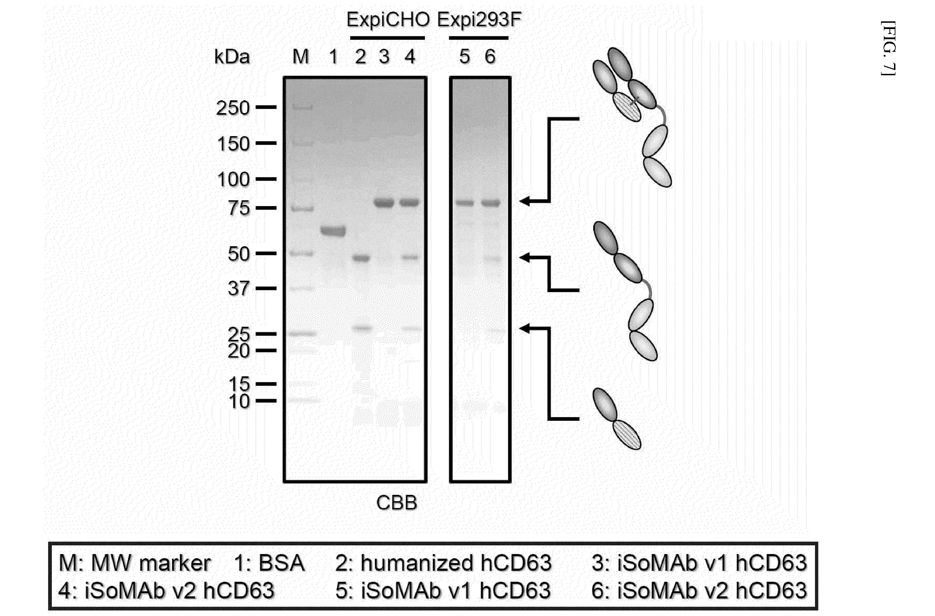

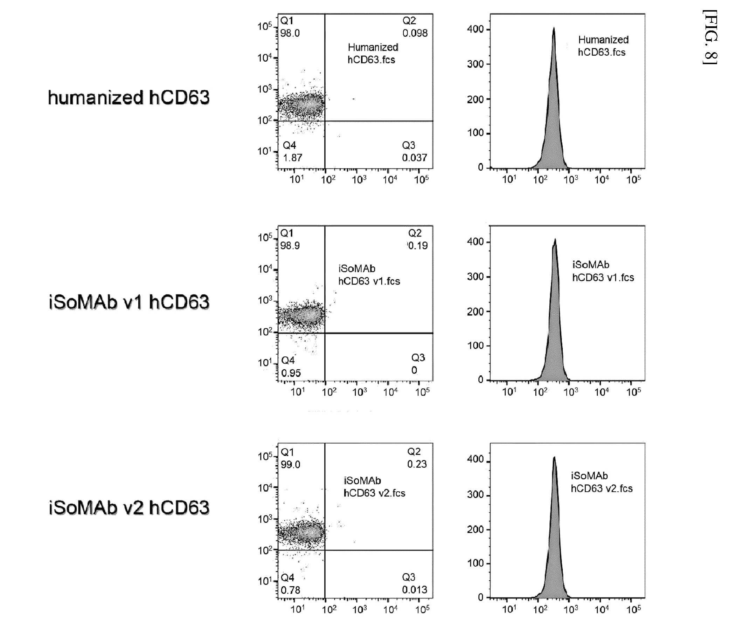

From the gel electrophoresis results (FIG. 7), bands were confirmed at the target molecular weight portion of the complex of heavy chain and light chain of iSoMAb under reducing conditions, and it is considered that an isopeptide bond has been formed between the heavy chain and the light chain (lanes 3 and 4). In addition, expression of iSoMAb in Expi293F cells was also confirmed (lanes 5 and 6). Furthermore, from the flow cytometry results (FIG. 8), it was confirmed that both types of iSoMAb maintained binding to CD63-expressing cells (293 strain). Namely, when, of the amino acid at position 186 of the heavy chain and the amino acid at position 176 of the light chain, one was lysine and the other was asparagine, an isopeptide bond was formed between these amino acids.

### (Example 5: Production of Bispecific Antibody Containing Isopeptide Bond)

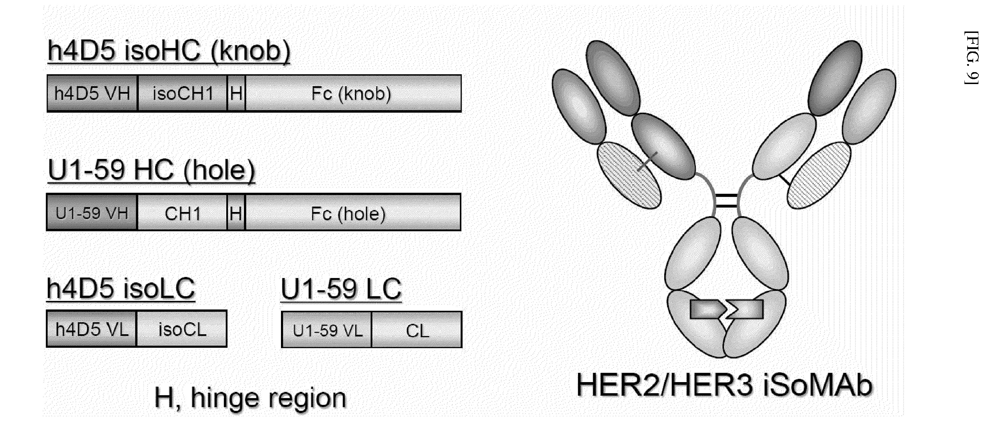

Production of a bispecific antibody containing one isopeptide bond was attempted. An overview of the structure of the constructed bispecific antibody is shown in FIG. 9. This is a bispecific antibody comprising the variable regions of h4D5 (anti-HER2) and U1-59 (anti-HER3), wherein h4D5 was modified to form an isopeptide bond according to Examples 1 to 2.

### - Preparation of h4D5xU1-59 iSoMAb bispecific IgG expression vector

The isoCH1 gene and the hinge-Fc (knob) gene were linked by Overlap Extension PCR, and digested with restriction enzymes Nhe I and Not I. The product was inserted into pCAGEN containing the h4D5VH gene that had been previously digested with the same enzymes to produce expression vector pCA h4D5 isoHC (knob). Next, U1-59VH and VL genes were synthesized by commissioning Eurofins Genomics. The plasmid containing the U1-59VH gene was digested with restriction enzymes Afl II and Nhe I, and then inserted into pCAGEN containing the CH1-Fc (hole) gene that had been previously digested with the same enzymes to produce expression vector pCA U1-59 HC (hole). In addition, the U1-59VL gene and the CL gene were linked by Overlap Extension PCR, and digested with restriction enzymes Eco RI and Not I. The product was inserted into pCAGEN that had been previously digested with the same enzymes to produce expression vector pCA U1-59 LC.

### - Expression of h4D5xU1-59 iSoMAb bispecific IgG expression vector

Expi293F cells (Gibco, Thermo Fisher Scientific) were used as host cells. Expi293F cells were cultured using HE200 medium (Gmep) containing 2 mM L-alanyl-L-glutamine under conditions of 37 °C, 5% CO2, and 125 rpm. 7.5 x 10⁷ cells were collected by centrifugation at 1,500 rpm for 5 minutes and suspended in 27 mL of HE400 medium (Gmep) containing 5 mM L-alanyl-L-glutamine. 30 µg of plasmid (7.5 µg of pCA h4D5 isoHC (knob), 7.5 µg of pCA U1-59 HC (hole), 7.5 µg of pCA h4D5 isoLC, and 7.5 µg of pCA U1-59 LC) was added to 1.5 mL of Opti-MEM medium (Gibco) and mixed. In addition, 120 µl of PEI MAX (1 mg/mL; Polysciences) was added to 1.5 mL of Opti-MEM medium and mixed, and the mixture was left to stand at room temperature for 5 minutes. After mixing the plasmid dilution and the PEI dilution, the mixture was left to stand at room temperature for 20 minutes, and this was used as the transfection solution. The transfection solution was added to Expi293F cells suspended in HE400 medium and cultured. After 20 hours, 75 µl of 0.5 M sodium valproate, 120 µl of 1 M sodium propionate, and 750 µl of 20% (w/v) tryptone were added, and the cells were cultured for an additional 6 days.

### - Purification of h4D5xU1-59 iSoMAb bispecific IgG

Cells were removed from the culture supernatant by centrifugation at 4 °C, 6,000 rpm for 10 minutes. The culture supernatant was filtered using a filter with a pore size of 0.45 µm (Membrane Solutions) and applied to a protein A column (column volume 250 µl; Bipo Resin Protein A; Protein Express) that had been previously equilibrated with phosphate-buffered saline (PBS). After washing with 10 mL of PBS, the target antibody was eluted with 100 mM glycine-HCl (pH 2.5). Fractions of 200 µl each were collected and neutralized with 10 µl of 100 mM Tris-HCl (pH 9.0) that had been previously added to sample tubes. Each fraction was analyzed by SDS-PAGE, and fractions containing the target antibody were dialyzed against PBS. Absorbance at 280 nm was measured with a spectrophotometer (NanoPhotometer NP80; Implen), and the absorbance at 320 nm was subtracted as background to quantify the target antibody. The calculation was performed using a molar extinction coefficient of 220500 M⁻¹ cm⁻¹ per tetramer.

### - Evaluation of bispecificity by sandwich ELISA

10 µg/mL of HER2-ECD-human Fc was added at 50 µl each to a 96-well EIA/RIA plate (Corning), and the plate was left to stand at 37 °C for 30 minutes. After removing the solution, 200 µl each of PBS containing 0.05% (v/v) Tween (registered trademark) 20 (PBST) was added and washed 3 times. 100 µl each of PBST containing 1% (w/v) BSA was added as blocking buffer. After standing at 37 °C for 1 hour, the blocking buffer was removed, and 200 µl each of PBST was added and washed 3 times. 50 µl each of bispecific antibody solutions at each concentration diluted with PBST containing 1% (w/v) BSA was added, and the plate was left to stand at 37 °C for 30 minutes. After removing the antibody solution, 200 µl each of PBST was added and washed 3 times. 50 µl each of 10 µg/mL HER3-ECD-mouse Fc was added. After standing at 37 °C for 30 minutes, the solution was removed, and 200 µl each of PBST was added and washed 3 times. 50 µl each of 0.25 µg/mL anti-IgG (H+L), Mouse, Goat-Poly, HRP (SeraCare Life Sciences, Inc.) was added as a detection antibody, and the plate was left to stand at 37 °C for 30 minutes. After removing the antibody solution, 200 µl each of PBST was added and washed 3 times. 50 µl each of chromogenic substrate solution [10 mg/mL TMB 50 µl, 3% H2O2 4 µl, substrate solution A (50 mM Na₂HPO₄-12H₂O, 25 mM citric acid, pH 5.5) 5 mL] was added, and after standing for 15 minutes, 50 µl each of stop solution (1 N H₂SO₄) was added. Absorbance at 450 nm was measured using a plate reader Infinite 200 PRO (TECAN).

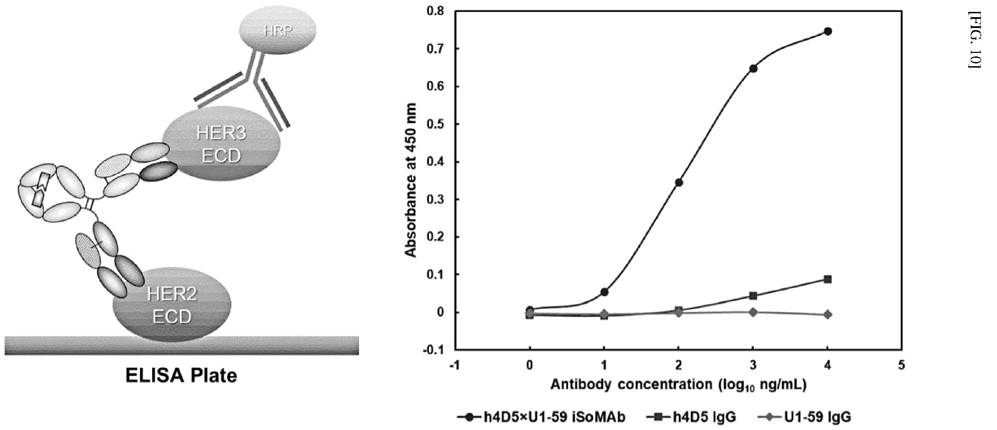

The results are shown in FIG. 10. Whereas no color development was observed with monospecific h4D5 IgG and U1-59 IgG, pronounced color development was observed with h4D5xU1-59 iSoMAb bispecific IgG. From this, it was found that an antibody containing an isopeptide bond is applicable to a bispecific antibody format. It is considered that a bispecific antibody containing isopeptide bonds at both antigen-binding sites of the bispecific antibody can also be constructed in a similar manner. Here, a knob-into-hole structure was adopted to promote pairing between the heavy chains of specific antibodies so that the target bispecific antibody can be obtained efficiently, but this knob-into-hole structure may be replaced with the isopeptide bond of the present disclosure.

### (Example 6: Production of Variant Without Hydrophobic Mutations)

A polypeptide in which only the K, N, and E mutations in the above isoCH1v1, isoCLv2, isoCLv1, and isoCH1v2 are introduced is produced. As a result, it is expected that formation of isopeptide bonds will be confirmed.

### (Example 7: Production of Antibodies of Various Formats Containing Isopeptide Bonds)

Based on the sequences into which the above isopeptide bond-forming mutations have been introduced, antibodies in Fab, Fab', or F(ab')₂ format are produced. It is expected that isopeptide bonds are still formed even in these different formats.

### (Example 8: Measurement of Cytotoxic Activity of Isopeptide Bond-Forming Anti-CD63 Antibody)

### - Production of antibody-drug conjugate (conjugate)

According to the method described in Example 4, anti-hCD63 iSoMAbv1 (heavy chain having SEQ ID NO: 45, light chain having SEQ ID NO: 46) and anti-hCD63 WT (wild-type) MAb having no mutations (heavy chain having SEQ ID NO: 43, light chain having SEQ ID NO: 44) were prepared. TCEP (tris(2-carboxyethyl)phosphine) at a final concentration of 100 µM was added to each IgG (1 mg/mL), mixed, and left to stand at 37 °C for 2 hours. After leaving this reaction solution to stand at room temperature for 30 minutes, VcMMAE (MedChemExpress) at a final concentration of 100 µM was added and left to stand at room temperature for 30 minutes. Next, NAC (N-acetylcysteine) at a final concentration of 1 mM was added and left to stand on ice for 30 minutes to stop the reaction, after which the mixture was dialyzed against PBS(-) to produce antibody-drug conjugates. When the drug-to-antibody ratio of the obtained antibody-drug conjugates was measured by UV absorption method, the drug-to-antibody ratio of both anti-hCD63 WT MAb-VcMMAE and anti-hCD63 iSoMAbv1-MMAE was approximately 4.

### - Measurement of cytotoxic activity

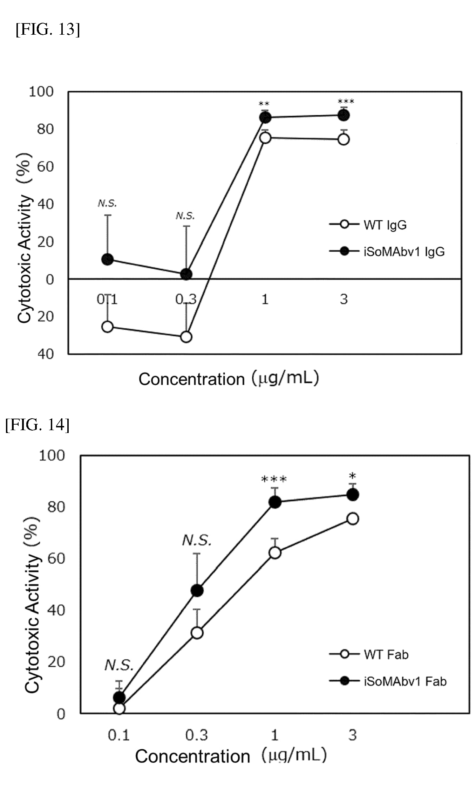

Human pancreatic cancer cell line SUIT-2 was seeded at 4 x 10³ cells per well in a 96-well plate, and the following day, antibody-drug conjugate at a final concentration of 0.1 to 3 µg/mL was added and cultured at 37 °C in a CO2 incubator for 96 hours. For the negative control, the same volume of medium was added and cultured for 96 hours in the same manner. After culturing, as a positive control, Tween 20 at a final concentration of 0.1% was added and left to stand in a CO2 incubator for 30 minutes. Next, Cell Counting Kit-8 (Dojindo) was added to each well and left to stand in a CO2 incubator for 1 hour, after which absorbance at 450 nm was measured using a plate reader. Cytotoxic activity (%) was calculated according to the following formula: Cytotoxic activity (%) = ((absorbance of negative control - absorbance of antibody-drug conjugate) / (absorbance of negative control - absorbance of positive control)) x 100 The results are shown in FIG. 13. At antibody-drug conjugate concentrations of 1 and 3 µg/mL, anti-hCD63 iSoMAbv1-MMAE showed significantly higher cytotoxic activity compared with anti-hCD63 WT MAb-VcMMAE.

### (Example 9: Measurement of Cytotoxic Activity of Isopeptide Bond-Forming Anti-CD63 Antibody in Fab Format)

### - Production of antibody-drug conjugate

An antibody-drug conjugate of a Fab format antibody fragment (heavy chain fragment having SEQ ID NO: 49, light chain fragment having SEQ ID NO: 50) was produced based on the anti-hCD63 iSoMAbv1 produced in Example 8. The control was an antibody-drug conjugate of anti-hCD63 WT Fab (heavy chain having SEQ ID NO: 47, light chain having SEQ ID NO: 48). When the drug-to-antibody ratio of the obtained antibody-drug conjugates was measured by UV absorption method, the drug-to-antibody ratio of both anti-hCD63 WT Fab-VcMMAE and anti-hCD63 iSoMAbv1 Fab-MMAE was approximately 1.

### - Measurement of cytotoxic activity

The cytotoxic activity of antibody-drug conjugates of Fab format antibody fragments was measured according to the method described in Example 8.

The results are shown in FIG. 14. At antibody-drug conjugate concentrations of 1 and 3 µg/mL, anti-hCD63 iSoMAbv1 Fab-MMAE showed significantly higher cytotoxic activity compared with anti-hCD63 WT Fab-VcMMAE.

### (Example 10: Measurement of Blood Concentration in Mice of Isopeptide Bond-Forming Trastuzumab IgG)

Isopeptide bond-forming trastuzumab IgG (h4D5 iSoMAb) (heavy chain having SEQ ID NO: 53, light chain having SEQ ID NO: 54) produced in Example 2 and WT (wild-type) trastuzumab IgG (h4D5 WT MAb) (heavy chain having SEQ ID NO: 51, light chain having SEQ ID NO: 52) that does not form an isopeptide bond were administered to mice, and blood was collected over time to measure the maximum blood concentration and blood half-life.

### - Administration and preparation of plasma

A PBS solution of IgG (1 mg/mL) was administered intraperitoneally at 10 mg/kg to five 5-week-old male ICR mice. Blood was collected from the tail vein at 1 hour, 1 day, 2 days, 3 days, 7 days, 10 days, and 15 days after administration. The collected blood was centrifuged at 3,500 rpm, 4 °C for 15 minutes to prepare plasma, and immediately stored at -30 °C.

### - Measurement of human IgG concentration in mouse plasma

Human IgG concentration in mouse plasma was measured using a Human IgG ELISA Kit (Assaypro).

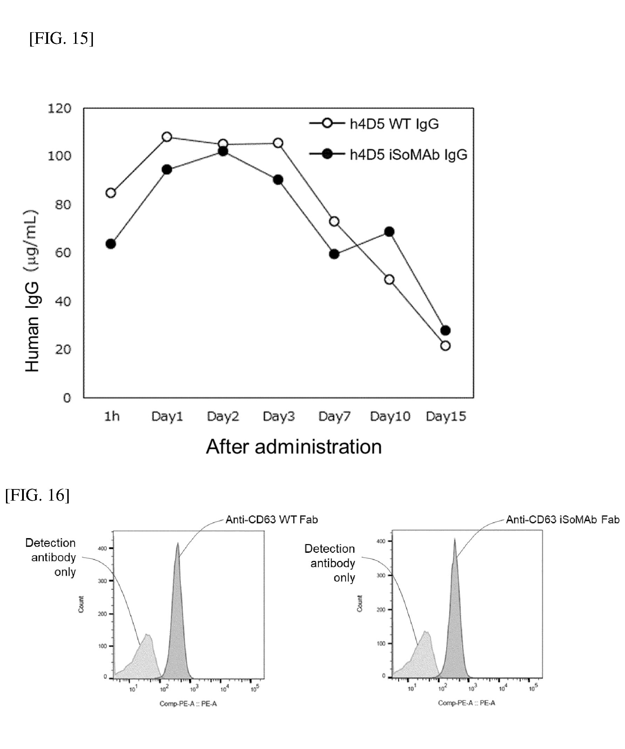

The results are shown in FIG. 15. The maximum blood concentration was approximately equivalent at 107.8 +/- 10.6 µg/mL (day 1) for h4D5 WT MAb and 102.0 +/- 10.8 µg/mL (day 2) for h4D5 iSoMAb. On the other hand, the blood half-life was 6.3 +/- 4.1 days for h4D5 WT MAb and 8.2 +/- 3.7 days for h4D5 iSoMAb, and h4D5 iSoMAb had a longer blood half-life compared with h4D5 WT MAb.

### (Example 11: Measurement of Binding of Isopeptide Bond-Forming Anti-CD63 Antibody in Fab Format to Antigen-Positive Cells)

### - Expression of hCD63 iSoMAb Fab

hCD63 iSoMAb Fab (heavy chain fragment having SEQ ID NO: 57, light chain having SEQ ID NO: 58) was expressed using the same method as in Example 4. pCEC4.3W-iSoMAb Fab hCD63 H1 and pCEC4.3W-isoCLvC hCD63 K1 were used as plasmids for transfection.

### - Purification of hCD63 iSoMAb Fab

Cells were removed from the culture supernatant by centrifugation at 4 °C, 12,000 x g for 10 minutes. The culture supernatant was filtered using a filter with a pore size of 0.45 µm (GVS) and applied to KanCapG (column volume 1.0 mL; KANEKA) that had been previously equilibrated with PBS. After washing with PBS, the target antibody was eluted with 0.1 M Glycine-HCl (pH 2.5) and neutralized with 1 M Tris-HCl (pH 8). Wash fractions (5 mL) and elution fractions (1 mL each) were analyzed by CBB staining after SDS-PAGE, and fractions containing the target antibody were solvent-exchanged to PBS using Pierce (TM) Protein Concentrators PES, 10K MWCO, 2-6 ml. Absorbance at 280 nm was measured with a spectrophotometer (Eppendorf BioPhotometer plus), and the absorbance at 340 nm was subtracted as background to quantify the target antibody. Thereafter, a more accurate concentration was determined using Pierce (TM) BCA Protein Assay Kit. Purified antibody of hCD63 WT (wild-type) Fab (heavy chain fragment having SEQ ID NO: 55, light chain having SEQ ID NO: 56) was also prepared in the same manner.

### - Evaluation of binding activity against hCD63-positive cells

Binding activity against a human fetal kidney-derived cell line 293 strain, which is hCD63-positive, was evaluated by flow cytometry. Expi293F cells cultured in Expi293 Expression Medium (Gibco, Thermo Fisher Scientific) under conditions of 37 °C, 5% CO2, and 125 rpm were collected by centrifugation at room temperature, 300 x g for 5 minutes, washed with 0.2% BSA/PBS, and 5 x 10⁵ cells were dispensed into sample tubes. hCD63 iSoMAb Fab at a final concentration of 2 µg/mL was reacted as a primary antibody at 4 °C for 60 minutes. After washing in the same manner, mouse anti-human IgKappa (PE), (TB28-2, BD Biosciences) at a final concentration of 12.5 µg/mL was reacted as a secondary antibody at 4 °C for 30 minutes. After washing again, the cells were suspended in 200 µl of 0.2% BSA/PBS. The suspension was dispensed into plastic tubes and analyzed using a flow cytometer (FACS Verse; BD Biosciences).

The results are shown in FIG. 16. The binding of hCD63 iSoMAb Fab and hCD63 WT (wild-type) Fab to antigen-positive cells was equivalent.

### (Example 12: Analysis of isopeptide bond formation of h4D5 iSoMAbvl variants)

### - Preparation of h4D5 iSoMAb variant expression plasmids

In order to investigate the effects of amino acid mutations at positions 173, 181, and 183 of the heavy chain, and positions 131, 133, 162, 176, 178, and 180 of the light chain (in particular the amino acid mutations shown in Tables 4 to 6) on isopeptide bond formation, Eurofins Genomics was commissioned to prepare variant expression plasmids in which any one of the amino acids at positions 173, 181, and 183 of the heavy chain, and positions 131, 133, 162, 176, 178, and 180 of the light chain were substituted according to Tables 4 to 6, based on the expression plasmids for h4D5 iSoMAb heavy chain (SEQ ID NO: 53) and light chain (SEQ ID NO: 54). h4D5 iSoMAb heavy chain mut1 to mut4 had SEQ ID NOs: 59 to 62, respectively, and h4D5 iSoMAb light chain mut1 to mut15 had SEQ ID NOs: 63 to 77, respectively.

**[Table 4]**

| isoCH1 | | | |
|---|---|---|---|
| | amino acid residue number | | |
| | 173 | 181 | 183 |
| iSoMAb | I | L | N |
| mut1 | V | L | N |
| mut2 | I | S | N |
| mut3 | V | S | N |
| mut4 | I | L | D |
| original | V | S | S |

**[Table 5]**

| isoCL | | | | | | |
|---|---|---|---|---|---|---|
| | amino acid residue number | | | | | |
| | 131 | 133 | 162 | 176 | 178 | 180 |
| iSoMAb | V | E | M | K | L | Q |
| mut1 | S | E | M | K | L | Q |
| mut2 | V | E | S | K | L | Q |
| mut3 | V | E | M | K | T | Q |
| mut4 | V | E | M | K | L | T |
| mut5 | S | E | S | K | L | Q |
| mut6 | S | E | M | K | T | Q |
| mut7 | S | E | M | K | L | T |

**[Table 6]**

| isoCL | | | | | | |
|---|---|---|---|---|---|---|
| | amino acid residue number | | | | | |
| | 131 | 133 | 162 | 176 | 178 | 180 |
| mut8 | V | E | S | K | T | Q |
| mut9 | V | E | S | K | L | T |
| mut10 | V | E | M | K | T | T |
| mut11 | S | E | S | K | T | Q |
| mut12 | S | E | S | K | L | T |
| mut13 | S | E | M | K | T | T |
| mut14 | V | E | S | K | T | T |
| mut15 | S | E | S | K | T | T |
| original | S | V | S | S | T | T |

### - Expression of h4D5 iSoMAb variants

HEK293T cells were used as host cells. All cultures were performed under conditions of 37 °C and 5% CO2. First, HEK293T cells were cultured in DMEM containing 10% FBS using 96-well plates. When 80-90% confluence was reached, transfection was performed. For transfection, 2 µL of 10 mg/mL polyethyleneimine solution was mixed with 1100 µL of FBS-free DMEM in advance, 13 µL was dispensed into each well of a 96-well plate, and 0.12 µg of plasmid (heavy chain: 0.065 µg, light chain: 0.065 µg, combinations according to Table 7) was mixed.

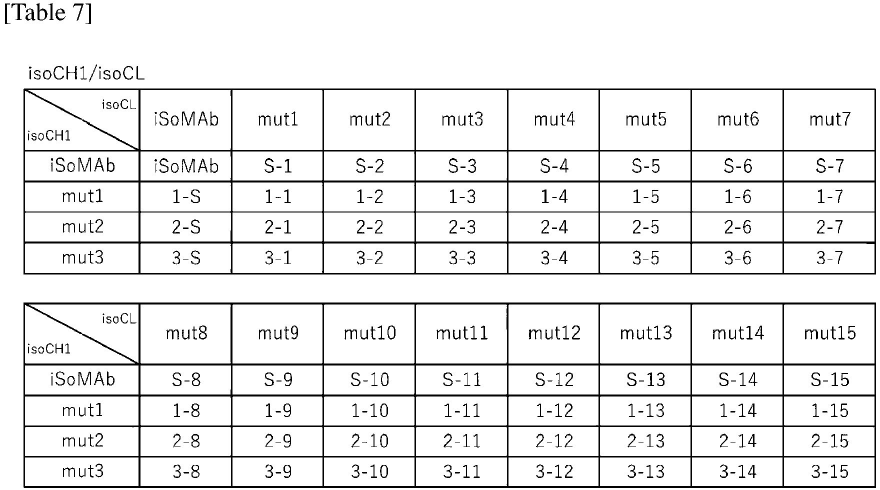

After standing for 20 minutes, the mixture was added to 200 µL of DMEM containing 2% FBS, and this was used as the transfection solution. After removing the medium from the 96-well plate in which cells had been seeded, 200 µL of the transfection solution was added to each well, and the cells were cultured for 8 days. The culture supernatant was collected, and expression was examined by sodium dodecyl sulfate-polyacrylamide gel electrophoresis (SDS-PAGE) and Western blotting using an anti-human Fc antibody (A0170, SIGMA-ALDRICH). In addition, band intensities were calculated using ImageJ, and the formation ratio of isopeptide bonds was estimated. The formation ratio (%) was calculated according to the following formula. 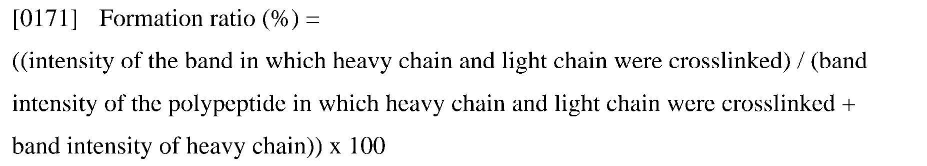
Formation ratio (%) = ((intensity of the band in which heavy chain and light chain were crosslinked) / (band intensity of the polypeptide in which heavy chain and light chain were crosslinked + band intensity of heavy chain)) × 100

The results are shown in Table 8. In the variant (variant 3-15) in which position 183 of the heavy chain, position 133 of the light chain, and position 176 of the light chain were substituted with asparagine, glutamic acid, and lysine, respectively, a polypeptide in which heavy chain and light chain were crosslinked by an isopeptide bond was detected.

**[Table 8]**

| Formation efficiency of the isopeptide bond | | | | | | | |
|---|---|---|---|---|---|---|---|
| Antibody | Formation rate(%) | Antibody | Formation rate(%) | Antibody | Formation rate(%) | Antibody | Formation rate(%) |
| iSoMAb | 85 | 1-S | 89 | 2-S | 80 | 3-S | 69 |
| S-1 | 73 | 1-1 | 73 | 2-1 | 85 | 3-1 | 83 |
| S-2 | 59 | 1-2 | 66 | 2-2 | 81 | 3-2 | 57 |
| S-3 | 78 | 1-3 | 92 | 2-3 | 55 | 3-3 | 41 |
| S-4 | 66 | 1-4 | 79 | 2-4 | 83 | 3-4 | 74 |
| S-5 | 27 | 1-5 | 54 | 2-5 | 76 | 3-5 | n.d. |
| S-6 | 84 | 1-6 | 93 | 2-6 | 49 | 3-6 | 50 |
| S-7 | 67 | 1-7 | 94 | 2-7 | 91 | 3-7 | 88 |
| S-8 | 93 | 1-8 | 94 | 2-8 | 80 | 3-8 | 73 |
| S-9 | 63 | 1-9 | 66 | 2-9 | 82 | 3-9 | 80 |
| S-10 | 79 | 1-10 | 80 | 2-10 | 61 | 3-10 | 46 |
| S-11 | 76 | 1-11 | 88 | 2-11 | 75 | 3-11 | 66 |
| S-12 | n.d. | 1-12 | n.d. | 2-12 | 89 | 3-12 | 62 |
| S-13 | 80 | 1-13 | 86 | 2-13 | 66 | 3-13 | 63 |
| S-14 | 86 | 1-14 | 95 | 2-14 | 80 | 3-14 | 73 |
| S-15 | 95 | 1-15 | 96 | 2-15 | 86 | 3-15 | 86 |

| | | | | | | | |
|---|---|---|---|---|---|---|---|
| n.d.: not detected. | | | | | | | |

### (Example 13: Preparation of h4D5 iSoMAbv1 variants)

### - Expression and purification of h4D5 iSoMAb variants

Using the variant expression plasmids prepared in Example 12, expression was performed by the same method as in Example 2, and purification was performed from the culture supernatant using a Protein A column. The purified antibodies were analyzed by SDS-PAGE under reducing conditions. In addition, band intensities were calculated using ImageJ, and the formation ratio of isopeptide bonds was estimated. The formation ratio (%) was calculated according to the following formula. 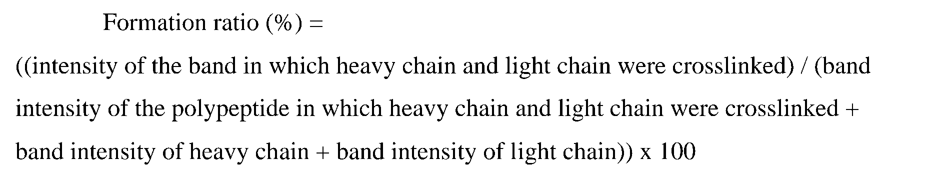
Formation ratio (%) = ((intensity of the band in which heavy chain and light chain were crosslinked) / (band intensity of the polypeptide in which heavy chain and light chain were crosslinked + band intensity of heavy chain + band intensity of light chain)) × 100

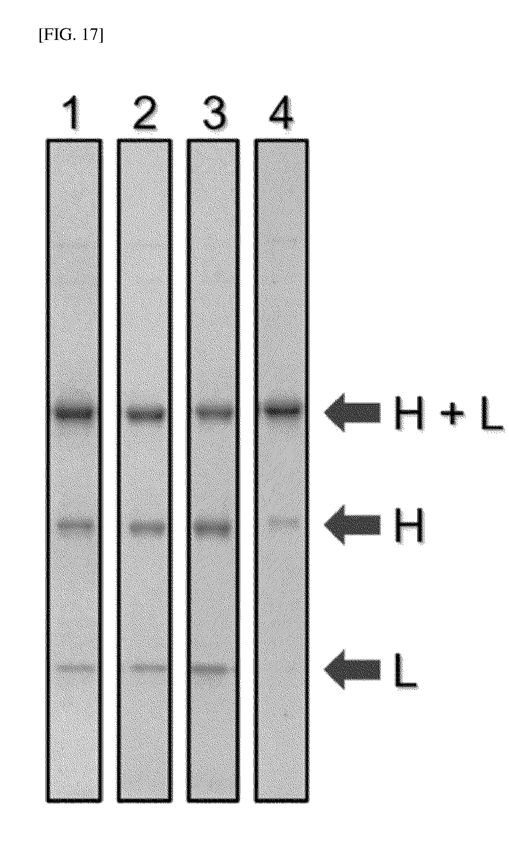

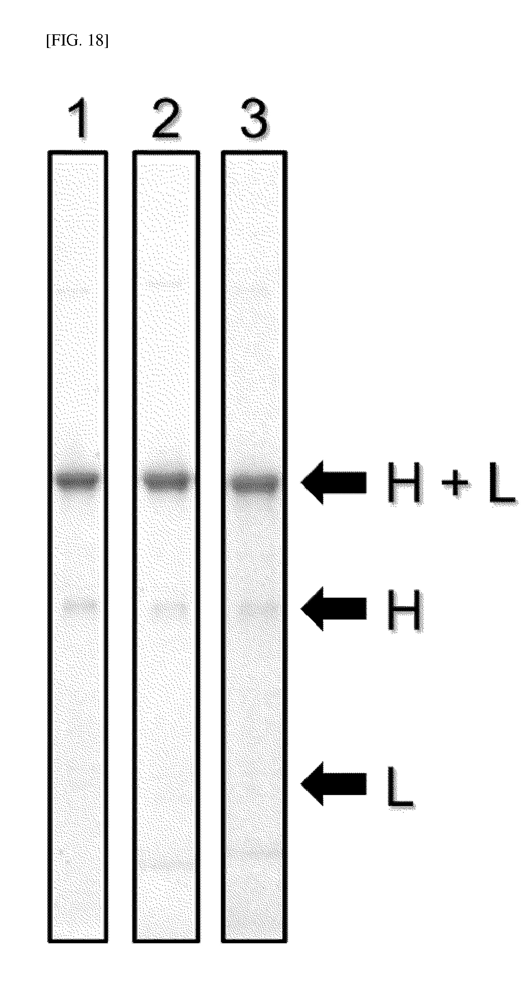

The results are shown in FIG. 17 and 18. The variant (variant 3-15, FIG. 17 lane 1) in which position 183 of the heavy chain, position 133 of the light chain, and position 176 of the light chain were substituted with asparagine, glutamic acid, and lysine, respectively, was sufficient for formation of isopeptide bonds between CH1 and CL under physiological conditions. In addition, although the yield was higher compared to h4D5 iSoMAbvl (FIG. 17 lane 4), the formation ratio of isopeptide bonds was slightly decreased. In these antibodies, it is considered that an isopeptide bond was formed between asparagine at position 183 of the heavy chain and lysine at position 176 of the light chain.

**[Table 9]**

| Table 9 : Lane configuration of FIG. 17 | | | |
|---|---|---|---|
| Lane No. | Formation rate (%) | Yield (mg/L culture) | Introduced mutations |
| 1 | 63 | 370 | H-S183N |
| | | | L-V133E, L-S176K |
| 2 | 53 | 360 | H-S183N |
| | | | L-S131V, L-V133E, L-S176K |
| 3 | 45 | 330 | H-S183N, L-V133E |
| | | | L-S162M, L-S176K |
| 4 | 85 | 330 | H-V173I, H-S181L, H-S183N, L-S131V, L-V133E, L-S162M |
| | | | L-S176K, L-T178L, L-T180Q |

| | | | |
|---|---|---|---|
| 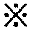 All variants comprise the three mutations of H-S183N, L-V133E, and L-S176K | | | |

On the other hand, the variant (variant 1-15, FIG. 18 lane 2) in which, in addition to the respective substitutions of position 183 of the heavy chain, position 133 of the light chain, and position 176 of the light chain with asparagine, glutamic acid, and lysine, position 181 of the heavy chain was substituted with leucine exhibited a formation ratio of isopeptide bonds equivalent to that of h4D5 iSoMAbv1. In these antibodies, it is considered that an isopeptide bond was formed between asparagine at position 183 of the heavy chain and lysine at position 176 of the light chain.

**[Table 10]**

| Table 10 : Lane configuration of FIG. 18 | | |
|---|---|---|
| Lane No. | Formation rate (%) | Introduced mutations |
| 1 | 89 | H-S181L, H-S183N |
| | | L-S131V, L-V133E, L-S176K |
| 2 | 93 | H-S181L, H-S183N |
| | | L-V133E, L-S176K |
| 3 | 89 | H-V173I, H-S181L, H-S183N, L-V133E, L-S176K |

| | | |
|---|---|---|
| 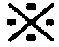 All variants comprise the three mutations of H-S183N, L-V133E, and L-S176K | | |

### (Example 14: Analysis of isopeptide bond formation of h4D5 iSoMAbv1 H-S183D variants)

### - Preparation of h4D5 iSoMAbvl H-S183D variant expression plasmids

Eurofins Genomics was commissioned to prepare a heavy chain variant expression plasmid in which positions 173, 181, and 183 of the heavy chain were substituted with isoleucine, leucine, and aspartic acid, respectively.

### - Expression of h4D5 iSoMAbv1 H-S183D variants

The heavy chain variant plasmid prepared above was combined with the light chain variant expression plasmid prepared in Example 12 (a variant in which positions 133 and 176 of the light chain were substituted with glutamic acid and lysine, respectively) (the heavy chain has SEQ ID NO: 45 and the light chain has SEQ ID NO: 78), and expression was performed by the same method as in Example 12, followed by analysis by SDS-PAGE and Western blotting. The formation ratio of isopeptide bonds was estimated in the same manner as in Example 12.

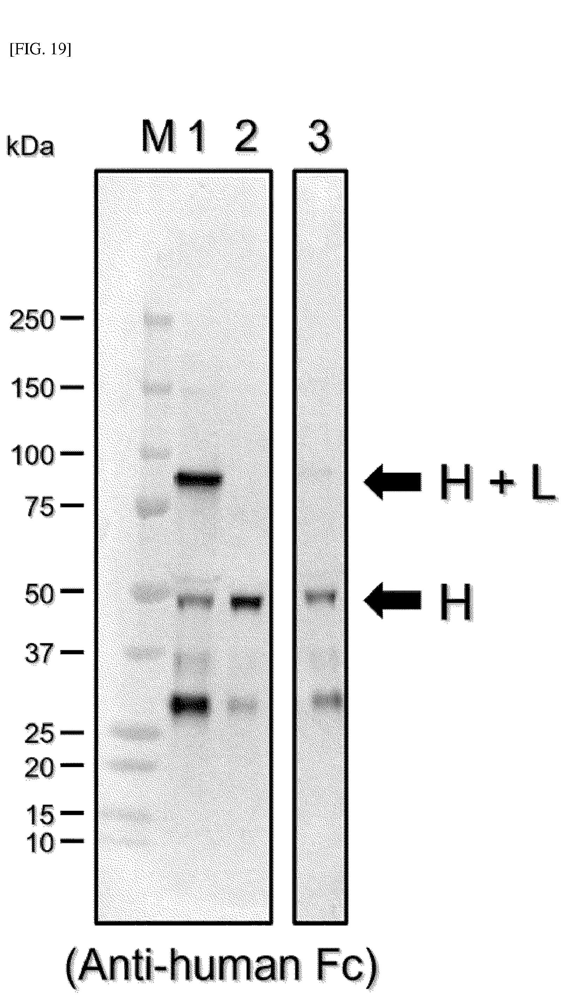

The results are shown in FIG. 19. When position 183 of the heavy chain was substituted with aspartic acid, a polypeptide in which heavy chain and light chain were crosslinked by an isopeptide bond was detected. The formation ratio of isopeptide bonds was about 10%. In these antibodies, it is considered that an isopeptide bond was formed between asparagine or aspartic acid at position 183 of the heavy chain and lysine at position 176 of the light chain.

**[Table 11]**

| Table 11 : Lane configuration of FIG. 19 | | |
|---|---|---|
| Lane No. | Formation rate (%) | Introduced mutations |
| 1 | 75 | H-V173I, H-S181L, H-S183N, L-S131V, L-V133E, L-S162M |
| | | L-S176K, L-T178L, L-T180Q |
| 2 | n.d. | H-S183N |
| | | L-S176K |
| 3 | 10 | H-V173I, H-S181L, H-S183D, L-V133E, L-S176K |

| | | |
|---|---|---|
| n.d.: not detected. | | |

### (Example 15: Analysis of binding of h4D5 iSoMAb Fab to HER2 extracellular domain)

### - Analysis of h4D5 Fab-HER2 extracellular domain interaction by surface plasmon resonance

The binding of h4D5 iSoMAb Fab (the heavy chain has SEQ ID NO: 80 and the light chain has SEQ ID NO: 46) prepared in Example 3 to the HER2 extracellular domain was analyzed by surface plasmon resonance. As a comparison, h4D5 WT (wild-type) Fab (the heavy chain has SEQ ID NO: 79 and the light chain has SEQ ID NO: 46) was used. Measurements were performed using a molecular interaction analyzer BIACORE3000 (Cytiva). Using a Mouse Antibody Capture Kit (Cytiva), HER2 extracellular domain fused with a mouse-derived Fc region was captured on a sensor chip (CM5, Cytiva), and then Fab solutions prepared at various concentrations were added to examine binding behavior. The obtained sensorgrams were fitted with a 1:1 Langmuir binding model using BIAevaluation Software (Cytiva), and kinetic parameters related to the interaction were calculated.

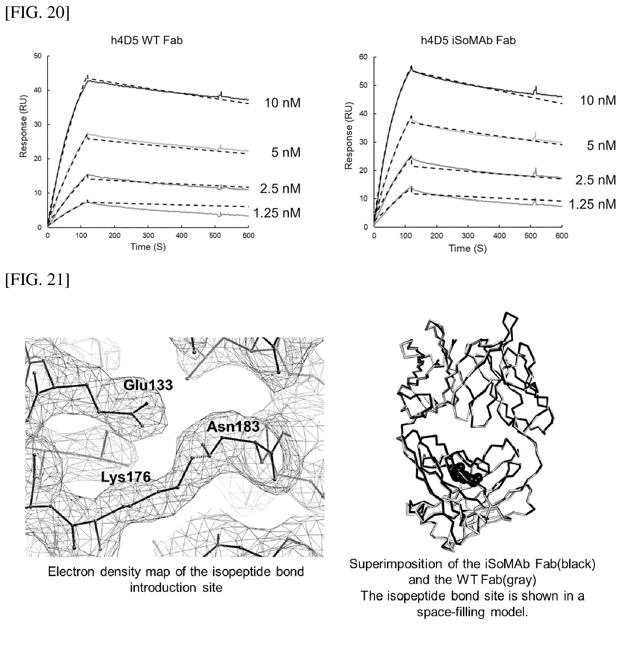

The results are shown in FIG. 20. The binding activity of h4D5 iSoMAb Fab to the HER2 extracellular domain was equivalent to or higher than that of h4D5 WT (wild-type) Fab.

**[Table 12]**

| Table 12 : Parameters of FIG. 20 | | | |
|---|---|---|---|
| Kinetic parameters regarding the interaction between h4D5 Fab and the HER2 extracellular domain | | | |
| | *K*_{D} (nM) | *k*ₒₙ (10⁵ M⁻¹ s⁻¹) | *k*_{off} (10⁻⁴ s⁻¹) |
| h4D5 WT Fab | 0.611 | 6.31 | 3.86 |
| h4D5 iSoMAb Fab | 0.420 | 11.7 | 4.90 |

### (Example 16: Crystal structure analysis of hCD63 iSoMAb Fab)

### - Crystallization of hCD63 iSoMAb Fab

Crystallization was performed using the 16.0 mg/mL hCD63 iSoMAb Fab sample prepared in Example 11. Crystallization was performed at 20 °C by the hanging drop vapor diffusion method using a crystallization buffer of 0.1 M sodium cacodylate pH 6.5, 0.2 M calcium acetate, and 43% PEG300.

### - Crystallization of hCD63 WT Fab

Crystallization was performed using the 15.8 mg/mL hCD63 WT Fab sample prepared in Example 11. Crystallization was performed at 20 °C by the hanging drop vapor diffusion method using a crystallization buffer of 0.1 M sodium cacodylate pH 6.5, 0.2 M calcium acetate, and 42% PEG300.

### - Structure analysis of hCD63 iSoMAb Fab and hCD63 WT Fab

Diffraction data were collected from the obtained crystals at the Tsukuba KEK Photon Factory BL5A beamline. Molecular replacement was performed using a similar molecular structure, and refinement was performed using Phenix Refine. Molecular model building was performed using Coot.

The results are shown in FIG. 21. Electron density indicating the formation of an isopeptide bond was observed between the side chains of the asparagine residue at position 183 of the heavy chain and the lysine residue at position 176 of the light chain. In addition, a glutamic acid residue at position 133 of the light chain was present in the vicinity thereof. When the structures of the iSoMAb Fab and the wild-type Fab were superimposed, the RMSD (root mean square deviation) was 0.34 A, and no significant change was observed in the overall structure.

### (Example 17: Formation of isopeptide bonds in human IgG4 κ)

### - Preparation of expression plasmids

Based on the amino acid sequence information of Nivolumab (Human IgG4 κ), mutations were introduced at sites involved in isopeptide bond formation (positions 183 of the heavy chain, 133 of the light chain, and 176 of the light chain were substituted with asparagine, glutamic acid, and lysine, respectively), and the amino acid sequence of Nivolumab iSoMAb was prepared. Eurofins Genomics was commissioned to prepare synthetic genes for the heavy chain (SEQ ID NO: 81) and the light chain (SEQ ID NO: 82) based on the amino acid sequence, and the genes were inserted into expression plasmids.

### - Expression of Nivolumab iSoMAb

Using the expression plasmids prepared above, recombinant antibodies were expressed in HEK293T cells in the same manner as in Example 12. The culture supernatant was collected, and expression was examined by SDS-PAGE and Western blotting. Anti-human Fc antibody (SIGMA: A0170) and anti-human kappa chain antibody (SIGMA: A7164) were used as detection antibodies.

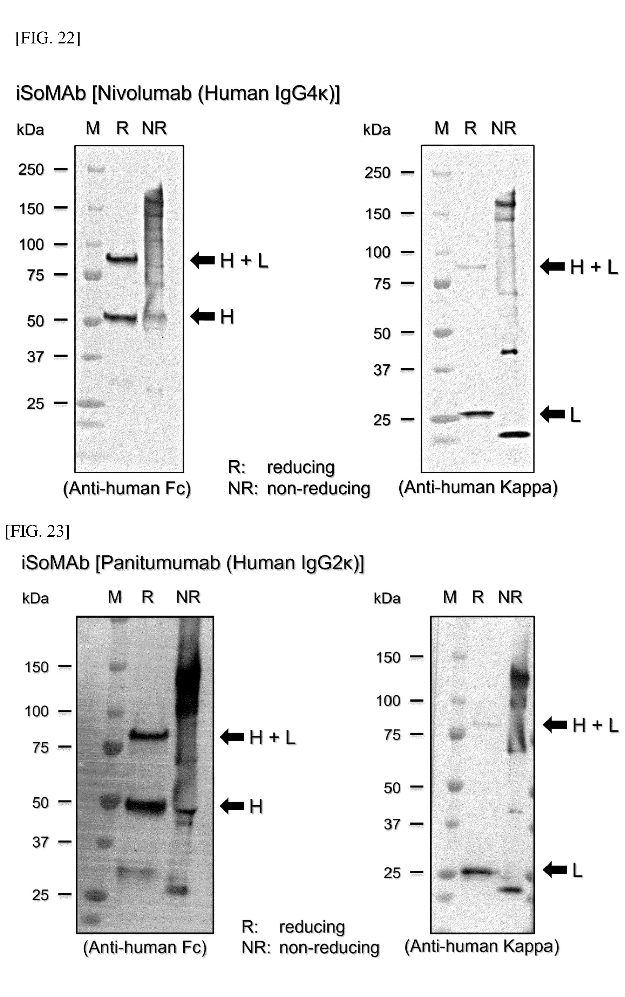

The results are shown in FIG. 22. When either the anti-human Fc antibody or the anti-human kappa chain antibody was used as the detection antibody, a polypeptide in which heavy chain and light chain were crosslinked by an isopeptide bond was detected.

### (Example 18: Formation of isopeptide bonds in human IgG2 κ)

### - Preparation of expression plasmids

Based on the amino acid sequence information of Panitumumab (Human IgG2 κ), mutations were introduced at sites involved in isopeptide bond formation (positions 183 of the heavy chain, 133 of the light chain, and 176 of the light chain were substituted with asparagine, glutamic acid, and lysine, respectively), and the amino acid sequence of Panitumumab iSoMAb was prepared. Eurofins Genomics was commissioned to prepare synthetic genes for the heavy chain (SEQ ID NO: 83) and the light chain (SEQ ID NO: 84) based on the amino acid sequence, and the genes were inserted into expression plasmids.

### - Expression of Panitumumab iSoMAb

Using the expression plasmids prepared above, recombinant antibodies were expressed in HEK293T cells in the same manner as in Example 12. The culture supernatant was collected, and expression was examined by SDS-PAGE and Western blotting. Anti-human Fc antibody (SIGMA: A9544) and anti-human kappa chain antibody (SIGMA: A7164) were used as detection antibodies. The results are shown in FIG. 23. When either the anti-human Fc antibody or the anti-human kappa chain antibody was used as the detection antibody, a polypeptide in which heavy chain and light chain were crosslinked by an isopeptide bond was detected.

### (Example 19: Formation of isopeptide bonds in human IgG1 λ)

### - Preparation of expression plasmids

Eurofins Genomics was commissioned to synthesize as synthetic genes the heavy chain (SEQ ID NO: 85) and the light chain variable region (SEQ ID NO: 86) of an anti-BCMA antibody (clone name 372, Human IgG1 λ). The heavy chain expression plasmid of h4D5 iSoMAbv1 prepared in Example 12 was digested with restriction enzymes Eco RI and Nhe I, and the 372 heavy chain variable region digested with the same enzymes was inserted to prepare the 372 heavy chain expression plasmid. In addition, the light chain constant region synthesized as a synthetic gene in the same manner (positions 131, 133, 162, 176, 178, and 180 substituted with valine, glutamic acid, methionine, lysine, leucine, and glutamine, respectively) and the 372 light chain variable region were linked by Overlap Extension PCR, then digested with restriction enzymes Eco RI and Not I, and inserted into an expression plasmid that had been digested with the same enzymes in advance, thereby preparing the 372 light chain expression plasmid.

### - Expression and purification of 372 iSoMAb

Using the above expression plasmids, recombinant antibodies were expressed by the same method as in Example 2, and purified from the culture supernatant by Protein A affinity chromatography. The recombinant antibodies after Protein A purification were analyzed by SDS-PAGE under reducing conditions.

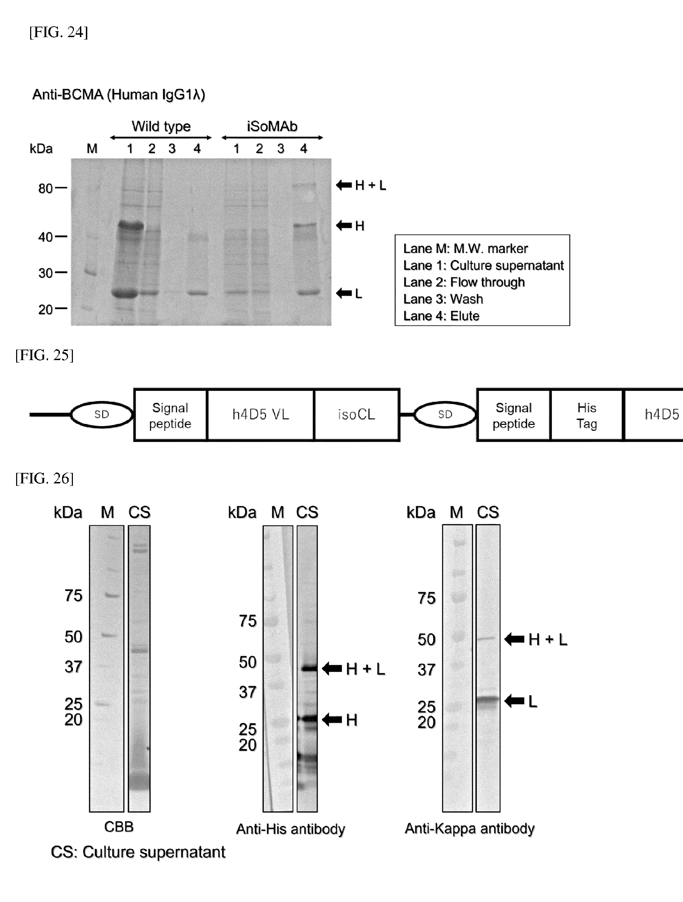

The results are shown in FIG. 24. In the purified 372 iSoMAb, a polypeptide in which heavy chain and light chain were crosslinked by an isopeptide bond was detected.

### (Example 20: Production of h4D5 iSoMAb Fab using Brevibacillus)

- Preparation of expression plasmids Eurofins Genomics was commissioned to prepare a plasmid comprising the h4D5 iSoMAb light chain sequence (amino acid substitution sequence at positions 133 and 176 of Table 6) (SEQ ID NO: 87) and the heavy chain sequence (amino acid substitution sequence at position 183 of Table 4) (SEQ ID NO: 88). Next, using the plasmid as a template, PCR was performed using primers (5'-AGTTCCGCATTCGCTGACATC-3' and 5'-CATCCTGTTAAGCTTAGTGGGTCTTGTCGCTGC-3') to amplify a target sequence having a His tag sequence, which was then inserted into the pBIC3 DNA vector (the vector sequence is shown in FIG. 25).
- Purification of h4D5 iSoMAb Fab

The expression vector obtained above was introduced into Brevibacillus to prepare a transformant, and the transformant was cultured in MT medium at 30 °C for 3 days. To the culture supernatant obtained by centrifugation, Ni-NTA resin (Ni Sepharose excel, Cytiva) was added to purify h4D5 iSoMAb Fab. The culture supernatant was analyzed by SDS-PAGE, CBB staining, and Western blotting. Anti-His tag antibody (MBL: Code No. D291-3) and anti-human kappa chain antibody (SIGMA: A7164) were used as detection antibodies. For the purified antibody, CBB staining was performed. In the same manner, purified antibody of h4D5 WT (wild-type) Fab was also prepared, and CBB staining was performed.

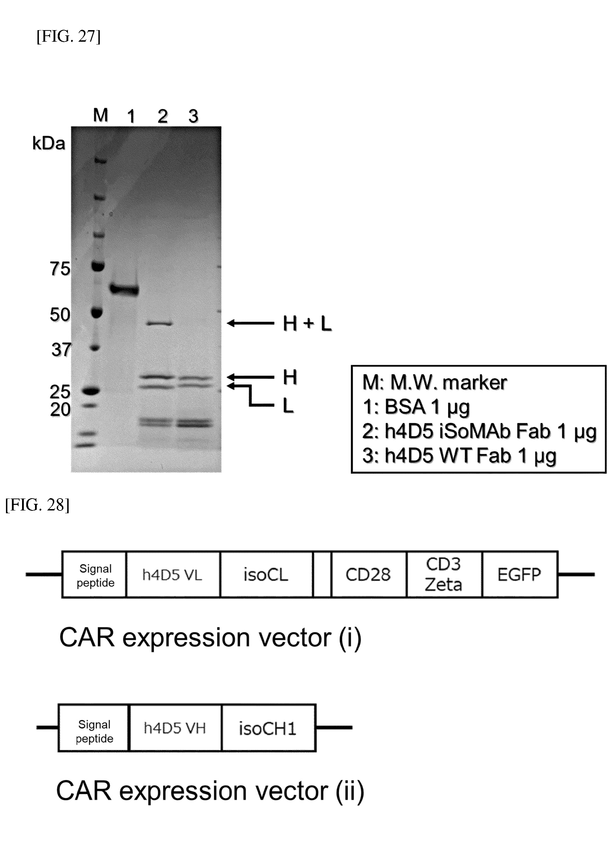

The results are shown in FIG. 26 and 27. When either the anti-His tag antibody or the anti-human kappa chain antibody was used as the detection antibody, a polypeptide in which heavy chain and light chain were crosslinked by an isopeptide bond was detected. In addition, a polypeptide in which heavy chain and light chain were crosslinked by an isopeptide bond was also detected in the purified h4D5 iSoMAb Fab.

### (Example 20: Preparation of chimeric antigen receptor of isopeptide bond-forming trastuzumab Fab and measurement of binding activity to antigen)

In general, a chimeric antigen receptor (CAR) is an artificial chimeric protein in which a single-chain antibody that recognizes a cell surface antigen of a cancer cell is fused with a signaling domain that induces activation of T cells. However, it is known that the preparation of a single-chain antibody that recognizes a cell surface antigen is not easy. Therefore, the preparation of a CAR comprising an antibody that forms an isopeptide bond and the binding activity to an antigen were measured.

### - Preparation and expression confirmation of CAR expression vector of isopeptide bond-forming trastuzumab Fab

A CAR expression vector (i) consisting of human CD28, CD3zeta, and enhanced green fluorescent protein (EGFP) downstream of the h4D5 iSoMAb Fab light chain prepared in Example 3 was prepared. In addition, a CAR expression vector (ii) comprising the h4D5 iSoMAb Fab heavy chain was prepared (shown in FIG. 28). The obtained CAR had SEQ ID NO: 89.

HEK293 cells were seeded at 2 x 10⁵ cells per well in a 24-well plate, and the next day, a total of 500 ng of CAR expression vector (i) and/or CAR expression vector (ii) per well and 1.5 µL of polyethyleneimine (1 mg/mL) were mixed at room temperature for 20 minutes and then added to the HEK293 cells. Three days after transfection, a RIPA buffer containing a surfactant was added and pipetting was performed to prepare a lysate, a sample buffer containing a reducing agent was added and the mixture was boiled, followed by SDS-PAGE, transfer to a PVDF membrane, and blotting with an anti-GFP antibody.

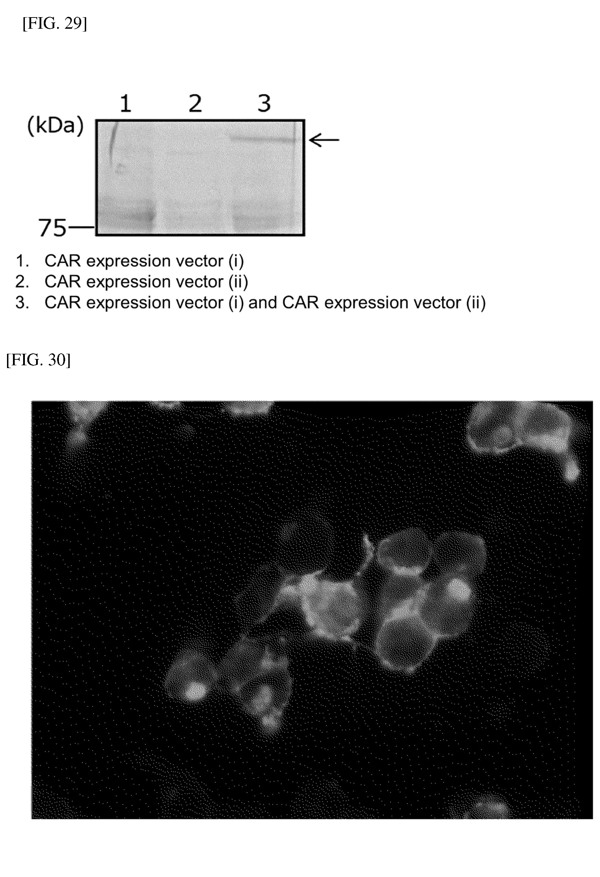

The results are shown in FIG. 29. In cells transfected with only CAR expression vector (i), a band of about 80 kDa was detected, whereas in cells transfected with CAR expression vector (i) and CAR expression vector (ii), the intensity of the band of about 80 kDa was decreased, while a band containing isopeptide bond-forming trastuzumab Fab (indicated by an arrow) was detected on the higher molecular weight side. In addition, when HEK293 cells into which both vectors had been introduced were observed under a fluorescence microscope, it was shown that the chimeric antigen receptor of isopeptide bond-forming trastuzumab Fab was present on the cell membrane surface, as shown in FIG. 30.

### - Measurement of binding activity to antigen

An expression vector of HER2 (extracellular domain with a secretion signal)-mouse Fc was transfected into HEK293 cells, and the culture supernatant 2 days later was prepared. The culture supernatant quantified by dot blot method using mouse IgG as a standard (containing 1 µg/mL of HER2-mouse Fc) was added to HEK293 cells 3 days after transfection with CAR expression vector (i) and/or CAR expression vector (ii), and the mixture was allowed to stand at 4 °C for 1 hour. After washing three times with PBS(-) containing 1% BSA, anti-mouse IgG-PE and Hoechst 33342 for nuclear staining were added, and the mixture was similarly allowed to stand at 4 °C for 1 hour. Next, after washing three times with PBS(-) containing 1% BSA, the cells were fixed with 4% formaldehyde and observed under a fluorescence microscope.

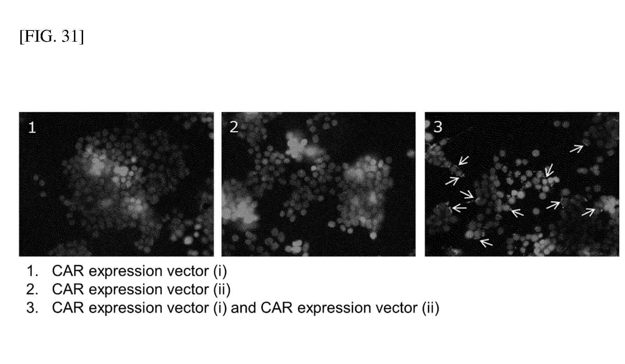

The results are shown in FIG. 31. By introducing CAR expression vector (i) and (ii), it was confirmed that HER2-mouse Fc indicated by arrows was bound to the cell membrane of HEK293 cells.

### (Example 21)

In the same manner as described above, the variants shown in the following tables were prepared, and the formation of isopeptide bonds between CH1 and CL was confirmed. The heavy chain of iSoMAbv2 had SEQ ID NO: 91, and iSoMAbv2 HC mut15 had SEQ ID NO: 92.

**[Table 13]**

| isoCH1v2 | | | | |
|---|---|---|---|---|
| | amino acid residue number | | | |
| | 170 | 173 | 181 | 183 |
| iSoMAbv2 | E | I | L | K |
| mut15 | E | V | S | K |
| original | F | V | S | S |

**[Table 14]**

| isoCLv2 | | | | | | |
|---|---|---|---|---|---|---|
| | amino acid residue number | | | | | |
| | 131 | 133 | 162 | 176 | 178 | 180 |
| iSoMAbv2 | S | V | M | N | L | Q |
| original | S | V | S | S | T | T |

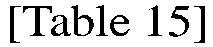

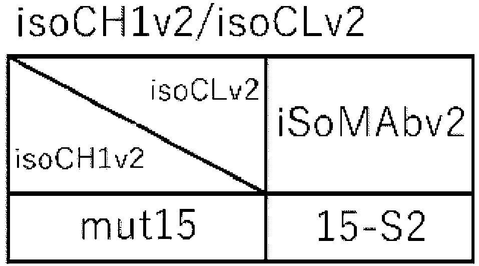

**[Table 16]**

| Table 16 : Lane configuration of FIG. 32 | |
|---|---|
| Lane No. | Introduced mutations |
| 1 | H-F170E, H-S183K L-S162M, L-S176N, L-T178L, L-T180Q |
| 2 | H-F170E, H-V1731, H-S181L, H-S183K L-S162M, L-S176N, L-T178L, L-T180Q |

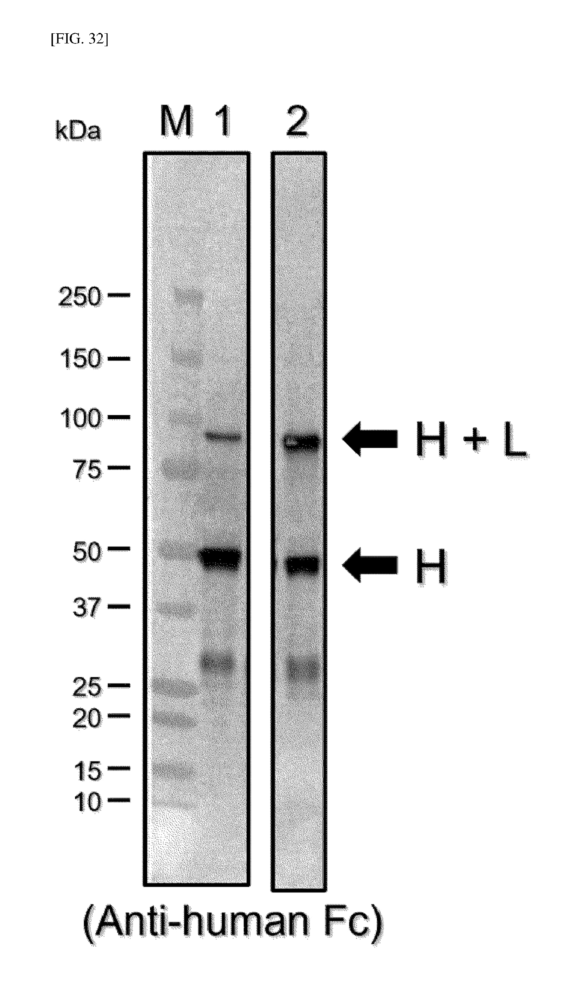

As a result, as shown in FIG. 32, in all variants, a band appeared in the molecular weight region of H+L under denaturing conditions, suggesting the presence of isopeptide bonds between CH1 and CL. In these antibodies, it is considered that an isopeptide bond was formed between lysine at position 183 of the heavy chain and asparagine at position 176 of the light chain.

### (Example 22)

In the same manner as described above, the variants shown in the following tables were prepared, and the formation of isopeptide bonds between CH1 and CL was confirmed. The heavy chain of iSoMAbv2 had SEQ ID NO: 91, and iSoMAbv2 HC mut15 had SEQ ID NO: 92. iSoMAbv2 LC mut3 had SEQ ID NO: 94.

**[Table 17]**

| isoCH1v2 | | | | |
|---|---|---|---|---|
| | amino acid residue number | | | |
| | 170 | 173 | 181 | 183 |
| iSoMAbv2 | E | I | L | K |
| mut15 | E | V | S | K |
| original | F | V | S | S |

**[Table 18]**

| isoCLv2 | | | | | | |
|---|---|---|---|---|---|---|
| | amino acid residue number | | | | | |
| | 131 | 133 | 162 | 176 | 178 | 180 |
| iSoMAbv2 | S | V | M | N | L | Q |
| mut3 | S | V | S | N | T | T |
| original | S | V | S | S | T | T |

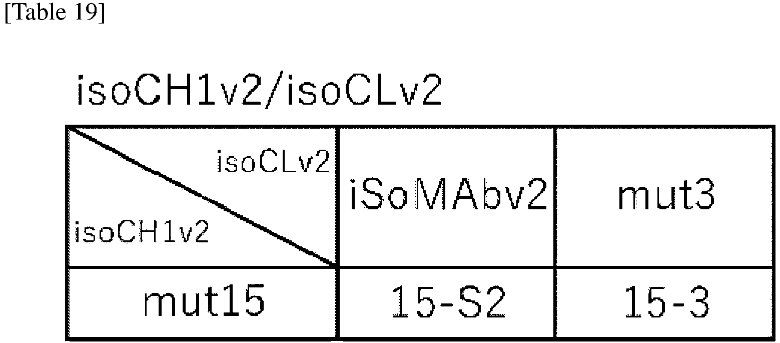

**[Table 20]**

| Table 20 : Lane configuration of FIG. 33 | |
|---|---|
| Lane No. | Introduced mutations |
| 1 | H-F170E, H-S183K L-S162M, L-S176N, L-T178L, L-T180Q |
| 2 | H-F170E, H-S183K L-S176N |
| 3 | H-F170E, H-V1731, H-S181L, H-S183K L-S162M, L-S176N, L-T178L, L-T180Q |

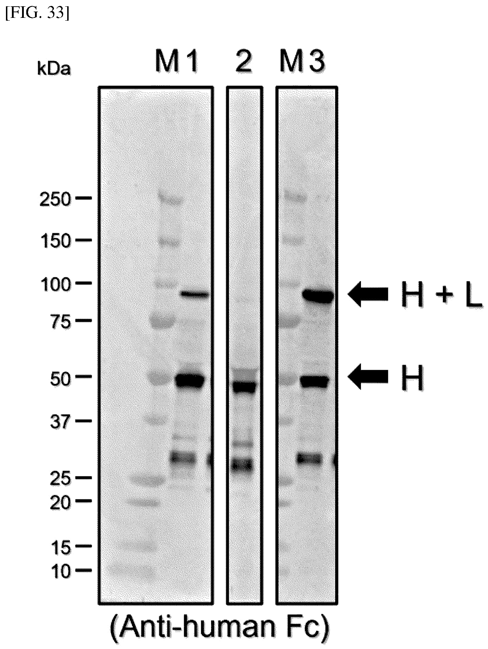

As a result, as shown in FIG. 33, in all variants (lanes 1, 2, and 3), a band appeared in the molecular weight region of H+L under denaturing conditions, suggesting the presence of isopeptide bonds between CH1 and CL. The mutations of heavy chain F170E and S183K, and light chain S176N mutation were sufficient for the formation of isopeptide bonds between CH1 and CL under physiological conditions, and the efficiency of isopeptide bond formation could be improved by introducing further mutations. In these antibodies, it is considered that an isopeptide bond was formed between lysine at position 183 of the heavy chain and asparagine at position 176 of the light chain.

As can be understood from the above examples, the formation of a covalent bond by isopeptide bonds between CH1 and CL does not negatively affect the binding affinity of the antibody to the antigen, but rather can stabilize the complex formed by the heavy chain and light chain. As a result of stabilization of the complex structure, it is evident that the introduction of isopeptide bonds between CH1 and CL into an antibody can even improve the binding affinity of the antibody to the antigen. In addition, the formation reaction of isopeptide bonds between CH1 and CL is a bioorthogonal reaction and is a reaction that can proceed in vivo, and can be beneficial in genetic engineering of antibodies and antibody manufacturing processes. Furthermore, asparagine and glutamine, and aspartic acid and glutamic acid are amino acids having very similar biochemical properties. It will be understood that isopeptide bonds can be formed not only between asparagine and lysine, and between aspartic acid and lysine, but also between glutamine and lysine, and between glutamic acid and lysine in the same manner.

### (Note)

As described above, the present disclosure has been illustrated using preferred embodiments of the present disclosure, but it is understood that the scope of the present disclosure should be interpreted only by the claims. It is understood that the patents, patent applications, and documents cited in the present specification should be incorporated herein by reference in the same manner as if the contents thereof were specifically described in the present specification.

### Industrial Applicability

The polypeptide comprising an isopeptide bond of the present disclosure provides one or more useful properties such as improved stability and production of a target substance in high purity. The present invention is applicable to a wide range of polypeptide production, such as production of recombinant antibodies having complex structures, including trispecific antibodies.

### [Sequence Listing Free Text]

- SEQ ID NO: 1: CH1 of h4D5
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSC
- SEQ ID NO: 2: modified CH1 of h4D5
ASTKGPSVFPLAPSSKSTSGGTAALGCLVKDYFPEPVTVSWNSGALTSGVHTFPA
ILQSSGLYLLNSVVTVPSSSLGTQTYICNVNHKPSNTKVDKKVEPKSS
- SEQ ID NO: 3: CL of h4D5


- SEQ ID NO: 4: modified CL of h4D5
RTVAAPSVFIFPPSDEQLKSGTAVVECLLNNFYPREAKVQWKVDNALQSGNSQE
MVTEQDSKDSTYSLKSLLQLSKADYEKHKVYACEVTHQGLSSPVTKSFNRGES
- SEQ ID NO: 5: CH1 of CD63 antibody
ASTKGPSVFPLAPSSKSTSGGTAALGCLVKDYFPEPVTVSWNSGALTSGVHTFPA

VLQSSGLYSLSSVVTVPSSSLGTQTYICNVNHKPSNTKVDKKVEPKSC
- SEQ ID NO: 6: modified CH1v1 of CD63 antibody
ASTKGPSVFPLAPSSKSTSGGTAALGCLVKDYFPEPVTVSWNSGALTSGVHTFPA
ILQSSGLYLLNSVVTVPSSSLGTQTYICNVNHKPSNTKVDKKVEPKSC
- SEQ ID NO: 7: modified CH1v2 of CD63 antibody
ASTKGPSVFPLAPSSKSTSGGTAALGCLVKDYFPEPVTVSWNSGALTSGVHTEPA
ILQSSGLYLLKSVVTVPSSSLGTQTYICNVNHKPSNTKVDKKVEPKSC
- SEQ ID NO: 8: CL of CD63 antibody


- SEQ ID NO: 9: modified CLv1 of CD63 antibody
RTVAAPSVFIFPPSDEQLKSGTAVVECLLNNFYPREAKVQWKVDNALQSGNSQE
MVTEQDSKDSTYSLKSLLQLSKADYEKHKVYACEVTHQGLSSPVTKSFNRGEC
- SEQ ID NO: 10: modified CLv2 of CD63 antibody
RTVAAPSVFIFPPSDEQLKSGTASVVCLLNNFYPREAKVQWKVDNALQSGNSQE
MVTEQDSKDSTYSLNSLLQLSKADYEKHKVYACEVTHQGLSSPVTKSFNRGEC
- SEQ ID NO: 11: Human_IgG1







- SEQ ID NO: 12: Human_IgG2
ASTKGPSVFPLAPCSRSTSESTAALGCLVKDYFPEPVTVSWNSGALTSGVHTFPA
VLQSSGLYSLSSVVTVPSSNFGTQTYTCNVDHKPSNTKVDKTVERKCCVECPPC
PAPPVAGPSVFLFPPKPKDTLMISRTPEVTCVVVDVSHEDPEVQFNWYVDGVEV
HNAKTKPREEQFNSTFRVVSVLTVVHQDWLNGKEYKCKVSNKGLPAPIEKTISK
TKGQPREPQVYTLPPSREEMTKNQVSLTCLVKGFYPSDIAVEWESNGQPENNYK
TTPPMLDSDGSFFLYSKLTVDKSRWQQGNVFSCSVMHEALHNHYTQKSLSLSP

GK
- SEQ ID NO: 13: Human_IgG3
ASTKGPSVFPLAPCSRSTSGGTAALGCLVKDYFPEPVTVSWNSGALTSGVHTFPA
VLQSSGLYSLSSVVTVPSSSLGTQTYTCNVNHKPSNTKVDKRVELKTPLGDTTH
TCPRCPEPKSCDTPPPCPRCPEPKSCDTPPPCPRCPEPKSCDTPPPCPRCPAPELLG
GPSVFLFPPKPKDTLMISRTPEVTCVVVDVSHEDPEVQFKWYVDGVEVHNAKT
KPREEQYNSTFRVVSVLTVLHQDWLNGKEYKCKVSNKALPAPIEKTISKTKGQP
REPQVYTLPPSREEMTKNQVSLTCLVKGFYPSDIAVEWESSGQPENNYNTTPPM
LDSDGSFFLYSKLTVDKSRWQQGNIFSCSVMHEALHNRFTQKSLSLSPGK
- SEQ ID NO: 14: Human_IgG4
ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPSCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K
- SEQ ID NO: 15: Mouse_IgG1
AKTTPPSVYPLAPGSAAQTNSMVTLGCLVKGYFPEPVTVTWNSGSLSSGVHTFP
AVLESDLYTLSSSVTVPSSPRPSETVTCNVAHPASSTKVDKKIVPRDCGCKPCICT
VPEVSSVFIFPPKPKDVLTITLTPKVTCVVVDISKDDPEVQFSWFVDDVEVHTAQ
TQPREEQFNSTFRSVSELPIMHQDWLNGKEFKCRVNSAAFPAPIEKTISKTKGRP
KAPQVYTIPPPKEQMAKDKVSLTCMITDFFPEDITVEWQWNGQPAENYKNTQPI
MNTNGSYFVYSKLNVQKSNWEAGNTFTCSVLHEGLHNHHTEKSLSHSPGK
- SEQ ID NO: 16: Mouse_IgG2A
AKTTAPSVYPLAPVCGDTTGSSVTLGCLVKGYFPEPVTLTWNSGSLSSGVHTFPA
VLQSDLYTLSSSVTVTSSTWPSQSITCNVAHPASSTKVDKKIEPRGPTIKPCPPCK
CPAPNLLGGPSVFIFPPKIKDVLMISLSPIVTCVVVDVSEDDPDVQISWFVNNVEV
HTAQTQTHREDYNSTLRVVSALPIQHQDWMSGKEFKCKVNNKDLPAPIERTISK
PKGSVRAPQVYVLPPPEEEMTKKQVTLTCMVTDFMPEDIYVEWTNNGKTELNY
KNTEPVLDSDGSYFMYSKLRVEKKNWVERNSYSCSVVHEGLHNHHTTKSFSRT

PGK
- SEQ ID NO: 17: Mouse_IgG2B
AKTTPPSVYPLAPGCGDTTGSSVTSGCLVKGYFPEPVTVTWNSGSLSSSVHTFPA
LLQSGLYTMSSSVTVPSSTWPSQTVTCSVAHPASSTTVDKKLEPSGPISTINPCPP
CKECHKCPAPNLEGGPSVFIFPPNIKDVLMISLTPKVTCVVVDVSEDDPDVQISW
FVNNVEVHTAQTQTHREDYNSTIRVVSTLPIQHQDWMSGKEFKCKVNNKDLPS
PIERTISKIKGLVRAPQVYTLPPPAEQLSRKDVSLTCLVVGFNPGDISVEWTSNGH
TEENYKDTAPVLDSDGSYFIYSKLNMKTSKWEKTDSFSCNVRHEGLKNYYLKK
TISRSPGK
- SEQ ID NO: 18: Mouse_IgG2C
AKTTAPSVYPLAPVCGGTTGSSVTLGCLVKGYFPEPVTLTWNSGSLSSGVHTFPA
LLQSGLYTLSSSVTVTSNTWPSQTITCNVAHPASSTKVDKKIEPRVPITQNPCPPL
KECPPCAAPDLLGGPSVFIFPPKIKDVLMISLSPMVTCVVVDVSEDDPDVQISWF
VNNVEVHTAQTQTHREDYNSTLRVVSALPIQHQDWMSGKEFKCKVNNRALPS
PIEKTISKPRGPVRAPQVYVLPPPAEEMTKKEFSLTCMITGFLPAEIAVDWTSNGR
TEQNYKNTATVLDSDGSYFMYSKLRVQKSTWERGSLFACSVVHEVLHNHLTTK
TISRSLGK
- SEQ ID NO: 19: Mouse_IgG3
ATTTAPSVYPLVPGCSDTSGSSVTLGCLVKGYFPEPVTVKWNYGALSSGVRTVS
SVLQSGFYSLSSLVTVPSSTWPSQTVICNVAHPASKTELIKRIEPRIPKPSTPPGSSC
PPGNILGGPSVFIFPPKPKDALMISLTPKVTCVVVDVSEDDPDVHVSWFVDNKE
VHTAWTQPREAQYNSTFRVVSALPIQHQDWMRGKEFKCKVNNKALPAPIERTIS
KPKGRAQTPQVYTIPPPREQMSKKKVSLTCLVTNFFSEAISVEWERNGELEQDY
KNTPPILDSDGTYFLYSKLTVDTDSWLQGEIFTCSVVHEALHNHHTQKNLSRSP
GK
- SEQ ID NO: 20: Rat_IgG1
AETTAPSVYPLAPGTALKSNSMVTLGCLVKGYFPEPVTVTWNSGALSSGVHTFP
AVLQSGLYTLTSSVTVPSSTWPSQTVTCNVAHPASSTKVDKKIVPRNCGGDCKP
CICTGSEVSSVFIFPPKPKDVLTITLTPKVTCVVVDISQDDPEVHFSWFVDDVEVH
TAQTRPPEEQFNSTFRSVSELPILHQDWLNGRTFRCKVTSAAFPSPIEKTISKPEG
RTQVPHVYTMSPTKEEMTQNEVSITCMVKGFYPPDIYVEWQMNGQPQENYKN

TPPTMDTDGSYFLYSKLNVKKEKWQQGNTFTCSVLHEGLHNHHTEKSLSHSPG
K
- SEQ ID NO: 21: Rat_IgG2A
AETTAPSVYPLAPGTALKSNSMVTLGCLVKGYFPEPVTVTWNSGALSSGVHTFP
AVLQSGLYTLTSSVTVPSSTWSSQAVTCNVAHPASSTKVDKKIVPRECNPCGCTG
SEVSSVFIFPPKTKDVLTITLTPKVTCVVVDISQNDPEVRFSWFIDDVEVHTAQTH
APEKQSNSTLRSVSELPIVHRDWLNGKTFKCKVNSGAFPAPIEKSISKPEGTPRGP
QVYTMAPPKEEMTQSQVSITCMVKGFYPPDIYTEWKMNGQPQENYKNTPPTM
DTDGSYFLYSKLNVKKETWQQGNTFTCSVLHEGLHNHHTEKSLSHSPGK
- SEQ ID NO: 22: Rat_IgG2B
AQTTAPSVYPLAPGCGDTTSSTVTLGCLVKGYFPEPVTVTWNSGALSSDVHTFP
AVLQSGLYTLTSSVTSSTWPSQTVTCNVAHPASSTKVDKKVERRNGGIGHKCPT
CPTCHKCPVPELLGGPSVFIFPPKPKDILLISQNAKVTCVVVDVSEEEPDVQFSW
FVNNVEVHTAQTQPREEQYNSTFRVVSALPIQHQDWMSGKEFKCKVNNKALPS
PIEKTISKPKGLVRKPQVYVMGPPTEQLTEQTVSLTCLTSGFLPNDIGVEWTSNG
HIEKNYKNTEPVMDSDGSFFMYSKLNVERSRWDSRAPFVCSVVHEGLHNHHV
EKSISRPPGK
- SEQ ID NO: 23: Rat_IgG2C
ARTTAPSVYPLVPGCSGTSGSLVTLGCLVKGYFPEPVTVKWNSGALSSGVHTFP
AVLQSGLYTLSSSVTVPSSTWSSQTVTCSVAHPATKSNLIKRIEPRRPKPRPPTDIC
SCDDNLGRPSVFIFPPKPKDILMITLTPKVTCVVVDVSEEEPDVQFSWFVDNVRV
FTAQTQPHEEQLNGTFRVVSTLHIQHQDWMSGKEFKCKVNNKDLPSPIEKTISK
PRGKARTPQVYTIPPPREQMSKNKVSLTCMVTSFYPASISVEWERNGELEQDYK
NTLPVLDSDESYFLYSKLSVDTDSWMRGDIYTCSVVHEALHNHHTQKNLSRSP
GK
- SEQ ID NO: 24: Rabbit_IgG
GQPKAPSVFPLAPCCGDTPSSTVTLGCLVKGYLPEPVTVTWNSGTLTNGVRTFPS
VRQSSGLYSLSSVVSVTSSSQPVTCNVAHPATNTKVDKTVAPSTCSKPTCPPPELL
GGPSVFIFPPKPKDTLMISRTPEVTCVVVDVSEDDPEVQFTWYINNEQVRTARPP
LREQQFNSTIRVVSTLPIAHEDWLRGKEFKCKVHNKALPAPIEKTISKARGQPLE
PKVYTMGPPREELSSRSVSLTCMINGFYPSDISVEWEKNGKAEDNYKTTPAVLD

SDGSYFLYSKLSVPTSEWQRGDVFTCSVMHEALHNHYTQKSISRSPGK
- SEQ ID NO: 25: Canine_IgG-A
ASTTAPSVFPLAPSCGSTSGSTVALACLVSGYFPEPVTVSWNSGSLTSGVHTFPSV
LQSSGLHSLSSMVTVPSSRWPSETFTCNVVHPASNTKVDKPVFNECRCTDTPPCP
VPEPLGGPSVLIFPPKPKDILRITRTPEVTCVVLDLGREDPEVQISWFVDGKEVHT
AKTQSREQQFNGTYRVVSVLPIEHQDWLTGKEFKCRVNHIDLPSPIERTISKARG
RAHKPSVYVLPPSPKELSSSDTVSITCLIKDFYPPDIDVEWQSNGQQEPERKHRM
TPPQLDEDGSYFLYSKLSVDKSRWQQGDPFTCAVMHETLQNHYTDLSLSHSPG
K
- SEQ ID NO: 26: Canine_IgG-B
ASTTAPSVFPLAPSCGSTSGSTVALACLVSGYFPEPVTVSWNSGSLTSGVHTFPSV
LQSSGLYSLSSMVTVPSSRWPSETFTCNVAHPASKTKVDKPVPKRENGRVPRPPD
CPKCPAPEMLGGPSVFIFPPKPKDTLLIARTPEVTCVVVDLDPEDPEVQISWFVD
GKQMQTAKTQPREEQFNGTYRVVSVLPIGHQDWLKGKQFTCKVNNKALPSPIE
RTISKARGQAHQPSVYVLPPSREELSKNTVSLTCLIKDFFPPDIDVEWQSNGQQE
PESKYRTTPPQLDEDGSYFLYSKLSVDKSRWQRGDTFICAVMHEALHNHYTQES
LSHSPGK
- SEQ ID NO: 27: Canine_IgG-C
ASTTAPSVFPLAPSCGSQSGSTVALACLVSGYIPEPVTVSWNSVSLTSGVHTFPSV
LQSSGLYSLSSMVTVPSSRWPSETFTCNVAHPATNTKVDKPVAKECECKCNCNN
CPCPGCGLLGGPSVFIFPPKPKDILVTARTPTVTCVVVDLDPENPEVQISWFVDSK
QVQTANTQPREEQSNGTYRVVSVLPIGHQDWLSGKQFKCKVNNKALPSPIEEIIS
KTPGQAHQPNVYVLPPSRDEMSKNTVTLTCLVKDFFPPEIDVEWQSNGQQEPES
KYRMTPPQLDEDGSYFLYSKLSVDKSRWQRGDTFICAVMHEALHNHYTQISLS
HSPGK
- SEQ ID NO: 28: Canine_IgG-D
ASTTAPSVFPLAPSCGSTSGSTVALACLVSGYFPEPVTVSWNSGSLTSGVHTFPSV
LQSSGLYSLSSTVTVPSSRWPSETFTCNVVHPASNTKVDKPVPKESTCKCISPCPV
PESLGGPSVFIFPPKPKDILRITRTPEITCVVLDLGREDPEVQISWFVDGKEVHTA
KTQPREQQFNSTYRVVSVLPIEHQDWLTGKEFKCRVNHIGLPSPIERTISKARGQ
AHQPSVYVLPPSPKELSSSDTVTLTCLIKDFFPPEIDVEWQSNGQPEPESKYHTTA

PQLDEDGSYFLYSKLSVDKSRWQQGDTFTCAVMHEALQNHYTDLSLSHSPGK
- SEQ ID NO: 29: Feline_IgG1a
ASTTAPSVFPLAPSCGTTSGATVALACLVLGYFPEPVTVSWNSGALTSGVHTFPA
VLQASGLYSLSSMVTVPSSRWLSDTFTCNVAHPPSNTKVDKTVRKTDHPPGPKP
CDCPKCPPPEMLGGPSIFIFPPKPKDTLSISRTPEVTCLVVDLGPDDSDVQITWFV
DNTQVYTAKTSPREEQFNSTYRVVSVLPILHQDWLKGKEFKCKVNSKSLPSPIE
RTISKAKGQPHEPQVYVLPPAQEELSRNKVSVTCLIKSFHPPDIAVEWEITGQPEP
ENNYRTTPPQLDSDGTYFVYSKLSVDRSHWQRGNTYTCSVSHEALHSHHTQKS
LTQSPGK
- SEQ ID NO: 30: Feline_IgG2
ASTTASSVFPLAPSCGTTSGATVALACLVLGYFPEPVTVSWNSGALTSGVHTFPS
VLQASGLYSLSSMVTVPSSRWLSDTFTCNVAHRPSSTKVDKTVPKTASTIESKTG
EGPKCPVPEIPGAPSVFIFPPKPKDTLSISRTPEVTCLVVDLGPDDSNVQITWFVD
NTEMHTAKTRPREEQFNSTYRVVSVLPILHQDWLKGKEFKCKVNSKSLPSAME
RTISKAKGQPHEPQVYVLPPTQEELSENKVSVTCLIKGFHPPDIAVEWEITGQPEP
ENNYQTTPPQLDSDGTYFLYSRLSVDRSHWQRGNTYTCSVSHEALHSHHTQKS
LTQSPGK
- SEQ ID NO: 31: Human_CL-kappa


- SEQ ID NO: 32: Mouse_CL-kappa
RADAAPTVSIFPPSSEQLTSGGASVVCFLNNFYPKDINVKWKIDGSERQNGVLNS
WTDQDSKDSTYSMSSTLTLTKDEYERHNSYTCEATHKTSTSPIVKSFNRNEC
- SEQ ID NO: 33: Rat_CL-kappa
RADAAPTVSIFPPSTEQLATGGASVVCLMNNFYPRDISVKWKIDGTERRDGVLD
SVTDQDSKDSTYSMSSTLSLTKADYESHNLYTCEVVHKTSSSPVVKSFNRNEC
- SEQ ID NO: 34: Rabbit_CL-kappa
GDPVAPTVLIFPPAADQVATGTVTIVCVANKYFPDVTVTWEVDGTTQTTGIENSK
TPQNSADCTYNLSSTLTLTSTQYNSHKEYTCKVTQGTTSVVQSFNRGDC
- SEQ ID NO: 35: Canine_CL-kappa
RNDAQPAVYLFQPSPDQLHTGSASVVCLLNSFYPKDINVKWKVDGVIQDTGIQE

SVTEQDKDSTYSLSSTLTMSSTEYLSHELYSCEITHKSLPSTLIKSFQRSECQRVD
- SEQ ID NO: 36: Feline_CL-kappa
RSDAQPSVFLFQPSLDELHTGSASIVCILNDFYPKEVNVKWKVDGVVQNKGIQE
STTEQNSKDSTYSLSSTLTMSSTEYQSHEKFSCEVTHKSLASTLVKSFNRSECQR
E
- SEQ ID NO: 37: Human_CL-lambda
GQPKAAPSVTLFPPSSEELQANKATLVCLISDFYPGAVTVAWKADGSPVKAGVE
TTKPSKQSNNKYAASSYLSLTPEQWKSHRSYSCQVTHEGSTVEKTVAPTECS
- SEQ ID NO: 38: Mouse_CL-lambda
GQPKSSPSVTLFPPSSEELETNKATLVCTITDFYPGVVTVDWKVDGTPVTQGME
TTQPSKQSNNKYMASSYLTLTARAWERHSSYSCQVTHEGHTVEKSLSRADCS
- SEQ ID NO: 39: Rat_CL-lambda
GQPKSTPTLTVFPPSTEELQGNKATLVCLISDFYPSDVEVAWKANGAPISQGVDT
ANPTKQGNKYIASSFLRLTAEQWRSRNSFTCQVTHEGNTVEKSLSPAECV
- SEQ ID NO: 40: Rabbit_CL-lambda
GQPVVAPLVTLFPPSSEELKANKATLVCLISDFYPGTLTVAWKADGVTITQGVDT
TQPSKQSNNKYMASSFLSLSRGQWTSHSRFTCQVTHEGKTTEKSVAPARCP
- SEQ ID NO: 41: Canine_CL-lambda
GQPKASPSVTLFPPSSEELGANKATLVCLISDFYPSGVTVAWKASGSPVTQGVET
TKPSKQSNNKYAASSYLSLTPDKWKSHSSFSCLVTHEGSTVEKKVAPAECS
- SEQ ID NO: 42: Feline_CL-lambda
GQPKSAPSVTLFPPSNEELSANKATLVCLISDFYPSGLTVAWKADGTPITQGVETT
KPSKQSNNKYAASSYLSLSPNEWKSRSRFTCQVTHEGSTVEKNVVPAECS
- SEQ ID NO: 43: hCD63 WT HC
MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD

ELTKNQVSLTCLVKGFYPSDIAVEWESNGQPENNYKTTPPVLDSDGSFFLYSKLT
VDKSRWQQGNVFSCSVMHEALHNHYTQKSLSLSP
- SEQ ID NO: 44: hCD63 WT LC
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTASVVCLLNNFYPREA
KVQWKVDNALQSGNSQESVTEQDSKDSTYSLSSTLTLSKADYEKHKVYACEVT
HQGLSSPVTKSFNRGEC
- SEQ ID NO: 45: hCD63 iSoMAbv1 HC
MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTFPAILQSSGLYLLNSVVTVPSSSLGT
QTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPG
- SEQ ID NO: 46: hCD63 iSoMAbv1 LC
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTAVVECLLNNFYPREA
KVQWKVDNALQSGNSQEMVTEQDSKDSTYSLKSLLQLSKADYEKHKVYACEV
THQGLSSPVTKSFNRGEC
- SEQ ID NO: 47: hCD63 WT VH-CH1 of Fab
MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTFPAVLQSSGLYSLSSVVTVPSSSLGT
QTYICNVNHKPSNTKVDKKVEPKSC
- SEQ ID NO: 48: hCD63 WT LC of Fab

MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTASVVCLLNNFYPREA
KVQWKVDNALQSGNSQESVTEQDSKDSTYSLSSTLTLSKADYEKHKVYACEVT
HQGLSSPVTKSFNRGEC
- SEQ ID NO: 49: hCD63 iSoMAbv1 VH-CH1 of Fab
MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTFPAILQSSGLYLLNSVVTVPSSSLGT
QTYICNVNHKPSNTKVDKKVEPKSC
- SEQ ID NO: 50: hCD63 iSoMAbv1 LC of Fab
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTAVVECLLNNFYPREA
KVQWKVDNALQSGNSQEMVTEQDSKDSTYSLKSLLQLSKADYEKHKVYACEV
THQGLSSPVTKSFNRGEC
- SEQ ID NO: 51: h4D5 WT HC
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
- SEQ ID NO: 52: h4D5 WT LC
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVVCLLNNFYPREAKV

QWKVDNALQSGNSQESVTEQDSKDSTYSLSSTLTLSKADYEKHKVYACEVTHQ
GLSSPVTKSFNRGEC
- SEQ ID NO: 53: h4D5 iSoMAb HC
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAILQSSGLYLLNSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
- SEQ ID NO: 54: h4D5 iSoMAb LC
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQEMVTEQDSKDSTYSLKSLLQLSKADYEKHKVYACEVTH
QGLSSPVTKSFNRGEC
- SEQ ID NO: 55: hCD63 WT VH-CH1 of Fab
MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTFPAVLQSSGLYSLSSVVTVPSSSLGT
QTYICNVNHKPSNTKVDKKVEPKSC
- SEQ ID NO: 56: hCD63 WT LC of Fab
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTASVVCLLNNFYPREA
KVQWKVDNALQSGNSQESVTEQDSKDSTYSLSSTLTLSKADYEKHKVYACEVT
HQGLSSPVTKSFNRGEC
- SEQ ID NO: 57: hCD63 iSoMAb VH-CH1 of Fab

MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTFPAILQSSGLYLLNSVVTVPSSSLGT
QTYICNVNHKPSNTKVDKKVEPKSC
- SEQ ID NO: 58: hCD63 iSoMAb LC of Fab
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTAVVECLLNNFYPREA
KVQWKVDNALQSGNSQEMVTEQDSKDSTYSLKSLLQLSKADYEKHKVYACEV
THQGLSSPVTKSFNRGEC
- SEQ ID NO: 59: h4D5 iSoMAb HC mut1
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAVLQSSGLYLLNSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
- SEQ ID NO: 60: h4D5 iSoMAb HC mut2
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAILQSSGLYSLNSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSK
LTVDKSRWQQGNVFSCSVMHEALHNHYTQKSLSLSPGK

- SEQ ID NO: 61: h4D5 iSoMAb HC mut3
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAVLQSSGLYSLNSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
- SEQ ID NO: 62: h4D5 iSoMAb HC mut4
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAILQSSGLYLLDSVVTVPSSSL
GTQTYICNVNHKPSNTKVDKKVEPKSCDKTHTCPPCPAPELLGGPSVFLFPPKPK
DTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPS
RDELTKNQVSLTCLVKGFYPSDIAVEWESNGQPENNYKTTPPVLDSDGSFFLYSK
LTVDKSRWQQGNVFSCSVMHEALHNHYTQKSLSLSPGK
- SEQ ID NO: 63: h4D5 iSoMAb LC mut1
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQEMVTEQDSKDSTYSLKSLLQLSKADYEKHKVYACEVTH
QGLSSPVTKSFNRGEC
- SEQ ID NO: 64: h4D5 iSoMAb LC mut2
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQESVTEQDSKDSTYSLKSLLQLSKADYEKHKVYACEVTH

QGLSSPVTKSFNRGEC
- SEQ ID NO: 65: h4D5 iSoMAb LC mut3
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQEMVTEQDSKDSTYSLKSTLQLSKADYEKHKVYACEVTH
QGLSSPVTKSFNRGEC
- SEQ ID NO: 66: h4D5 iSoMAb LC mut4
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQEMVTEQDSKDSTYSLKSLLTLSKADYEKHKVYACEVTH
QGLSSPVTKSFNRGEC
- SEQ ID NO: 67: h4D5 iSoMAb LC mut5
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQESVTEQDSKDSTYSLKSLLQLSKADYEKHKVYACEVTH
QGLSSPVTKSFNRGEC
- SEQ ID NO: 68: h4D5 iSoMAb LC mut6
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQEMVTEQDSKDSTYSLKSTLQLSKADYEKHKVYACEVTH
QGLSSPVTKSFNRGEC
- SEQ ID NO: 69: h4D5 iSoMAb LC mut7
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQEMVTEQDSKDSTYSLKSLLTLSKADYEKHKVYACEVTH
QGLSSPVTKSFNRGEC

- SEQ ID NO: 70: h4D5 iSoMAb LC mut8
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQESVTEQDSKDSTYSLKSTLQLSKADYEKHKVYACEVTH
QGLSSPVTKSFNRGEC
- SEQ ID NO: 71: h4D5 iSoMAb LC mut9
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQESVTEQDSKDSTYSLKSLLTLSKADYEKHKVYACEVTH
QGLSSPVTKSFNRGEC
- SEQ ID NO: 72: h4D5 iSoMAb LC mut10
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQEMVTEQDSKDSTYSLKSTLTLSKADYEKHKVYACEVTH
QGLSSPVTKSFNRGEC
- SEQ ID NO: 73: h4D5 iSoMAb LC mut11
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQESVTEQDSKDSTYSLKSTLQLSKADYEKHKVYACEVTH
QGLSSPVTKSFNRGEC
- SEQ ID NO: 74: h4D5 iSoMAb LC mut12
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQESVTEQDSKDSTYSLKSLLTLSKADYEKHKVYACEVTH
QGLSSPVTKSFNRGEC
- SEQ ID NO: 75: h4D5 iSoMAb LC mut13

METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQEMVTEQDSKDSTYSLKSTLTLSKADYEKHKVYACEVTH
QGLSSPVTKSFNRGEC
- SEQ ID NO: 76: h4D5 iSoMAb LC mut14
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTAVVECLLNNFYPREAKV
QWKVDNALQSGNSQESVTEQDSKDSTYSLKSTLTLSKADYEKHKVYACEVTH
QGLSSPVTKSFNRGEC
- SEQ ID NO: 77: h4D5 iSoMAb LC mut15
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQESVTEQDSKDSTYSLKSTLTLSKADYEKHKVYACEVTH
QGLSSPVTKSFNRGEC
- SEQ ID NO: 78: h4D5 iSoMAb LC mut17
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVVCLLNNFYPREAKV
QWKVDNALQSGNSQESVTEQDSKDSTYSLKSTLTLSKADYEKHKVYACEVTH
QGLSSPVTKSFNRGEC
- SEQ ID NO: 79: h4D5 WT VH-CH1-His
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAVLQSSGLYSLSSVVTVPSSSL
GTQTYICNVNHKPSNTKVDKKVEPKSCGSSHHHHHH
- SEQ ID NO: 80: h4D5 iSoMAb VH-CH1-His
MEFGLSWLFLVAILKGVQCEVQLVESGGGLVQPGGSLRLSCAASGFNIKDTYIH

WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAILQSSGLYLLNSVVTVPSSSL
GTQTYICNVNHKPSNTKVDKKVEPKSSGSSHHHHHH
- SEQ ID NO: 81: Nivolumab iSoMAb HC
MEFGLSWVFLVALLRGVQCQVQLVESGGGVVQPGRSLRLDCKASGITFSNSGM
HWVRQAPGKGLEWVAVIWYDGSKRYYADSVKGRFTISRDNSKNTLFLQMNSL
RAEDTAVYYCATNDDYWGQGTLVTVSSASTKGPSVFPLAPCSRSTSESTAALGC
LVKDYFPEPVTVSWNSGALTSGVHTFPAVLQSSGLYSLNSVVTVPSSSLGTKTYT
CNVDHKPSNTKVDKRVESKYGPPCPPCPAPEFLGGPSVFLFPPKPKDTLMISRTP
EVTCVVVDVSQEDPEVQFNWYVDGVEVHNAKTKPREEQFNSTYRVVSVLTVL
HQDWLNGKEYKCKVSNKGLPSSIEKTISKAKGQPREPQVYTLPPSQEEMTKNQ
VSLTCLVKGFYPSDIAVEWESNGQPENNYKTTPPVLDSDGSFFLYSRLTVDKSRW
QEGNVFSCSVMHEALHNHYTQKSLSLSLGK
- SEQ ID NO: 82: Nivolumab iSoMAb LC
MEAPAQLLFLLLLWLPDTTGEIVLTQSPATLSLSPGERATLSCRASQSVSSYLAW
YQQKPGQAPRLLIYDASNRATGIPARFSGSGSGTDFTLTISSLEPEDFAVYYCQQS
SNWPRTFGQGTKVEIKRTVAAPSVFIFPPSDEQLKSGTASVECLLNNFYPREAKV
QWKVDNALQSGNSQESVTEQDSKDSTYSLKSTLTLSKADYEKHKVYACEVTH
QGLSSPVTKSFNRGEC
- SEQ ID NO: 83: Panitumumab iSoMAb HC
MEFGLSWVFLVALLRGVQCQVQLQESGPGLVKPSETLSLTCTVSGGSVSSGDYY
WTWIRQSPGKGLEWIGHIYYSGNTNYNPSLKSRLTISIDTSKTQFSLKLSSVTAA
DTAIYYCVRDRVTGAFDIWGQGTMVTVSSASTKGPSVFPLAPCSRSTSESTAAL
GCLVKDYFPEPVTVSWNSGALTSGVHTFPAVLQSSGLYSLNSVVTVPSSNFGTQT
YTCNVDHKPSNTKVDKTVERKCCVECPPCPAPPVAGPSVFLFPPKPKDTLMISRT
PEVTCVVVDVSHEDPEVQFNWYVDGVEVHNAKTKPREEQFNSTFRVVSVLTV
VHQDWLNGKEYKCKVSNKGLPAPIEKTISKTKGQPREPQVYTLPPSREEMTKN
QVSLTCLVKGFYPSDIAVEWESNGQPENNYKTTPPMLDSDGSFFLYSKLTVDKS
RWQQGNVFSCSVMHEALHNHYTQKSLSLSPGK
- SEQ ID NO: 84: Panitumumab iSoMAb LC

MEAPAQLLFLLLLWLPDTTGDIQMTQSPSSLSASVGDRVTITCQASQDISNYLN
WYQQKPGKAPKLLIYDASNLETGVPSRFSGSGSGTDFTFTISSLQPEDIATYFCQ
HFDHLPLAFGGGTKVEIKRTVAAPSVFIFPPSDEQLKSGTASVECLLNNFYPREA
KVQWKVDNALQSGNSQESVTEQDSKDSTYSLKSTLTLSKADYEKHKVYACEV
THQGLSSPVTKSFNRGEC
- SEQ ID NO: 85: Anti-BCMA iSoMAb HC
MEFGLSWLFLVAILKGVQCEVQLVESGGGLVKPGGSLRLSCAASGFTFSNSGMI
WVRQAPGKGLEWVGHIRSKTDGGTTDYAAPVKGRFTISRDDSKNTLYLQMNSL
KTEDTAVYYCTTGGSGSFDYWGQGTLVTVSSASTKGPSVFPLAPSSKSTSGGTA
ALGCLVKDYFPEPVTVSWNSGALTSGVHTFPAILQSSGLYLLNSVVTVPSSSLGT
QTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPGK
- SEQ ID NO: 86: Anti-BCMA iSoMAb LC
MAWSPLLLTLLAHCTGSWAQAVVTQEPSLTVSPGGTVTLTCGSSTGAVTTSNYA
NWVQEKPGQAFRGLIGGTNKRAPGTPARFSGSLLGGKAALTLSGAQPEDEAEY
YCALWYSNLWVFGGGTKLTVLGQPKAAPSVTLFPPSSEELQANKAVLECLISDF
YPGAVTVAWKADGSPVKAGVETMKPSKQSNNKYAAKSLLQLTPEQWKSHRSY
SCQVTHEGSTVEKTVAPTECS
- SEQ ID NO: 87: h4D5VL-isoCL
MSISVRFKSLIALLMTVVFLLVPSSAFADIQMTQSPSSLSASVGDRVTITCRASQD
VNTAVAWYQQKPGKAPKLLIYSASFLYSGVPSRFSGSRSGTDFTLTISSLQPEDFA
TYYCQQHYTTPPTFGQGTKVEIKRTVAAPSVFIFPPSDEQLKSGTASVECLLNNF
YPREAKVQWKVDNALQSGNSQESVTEQDSKDSTYSLKSTLTLSKADYEKHKV
YACEVTHQGLSSPVTKSFNRGES
- SEQ ID NO: 88: His-h4D5VH-isoCH1
MKKRRVVNSVLLLLLLASALALTVAPMAFAADHHHHHHDDDDKEVQLVESGG
GLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTL

VTVSSASTKGPSVFPLAPSSKSTSGGTAALGCLVKDYFPEPVTVSWNSGALTSGV
HTFPAVLQSSGLYSLNSVVTVPSSSLGTQTYICNVNHKPSNTKVDKKVEPKSSDK
TH
- SEQ ID NO: 89: h4D5VL-isoCL-CD28-CD3Zeta-EGFP
METPAQLLFLLLLWLPESTGDIQMTQSPSSLSASVGDRVTITCRASQDVNTAVAW
YQQKPGKAPKLLIYSASFLYSGVPSRFSGSRSGTDFTLTISSLQPEDFATYYCQQH
YTTPPTFGQGTKVEIKRTVAAPSVFIFPPSDEQLKSGTASVECLLNNFYPREAKV
QWKVDNALQSGNSQESVTEQDSKDSTYSLKSTLTLSKADYEKHKVYACEVTH
QGLSSPVTKSFNRGECCFNGGSGGAAAIEVMYPPPYLDNEKSNGTIIHVKGKHL
CPSPLFPGPSKPFWVLVVVGGVLACYSLLVTVAFIIFWVRSKRSRLLHSDYMNM
TPRRPGPTRKHYQPYAPPRDFAAYRSRVKFSRSADAPAYQQGQNQLYNELNLGR
REEYDVLDKRRGRDPEMGGKPRRKNPQEGLYNELQKDKMAEAYSEIGMKGER
RRGKGHDGLYQGLSTATKDTYDALHMQALPPRGGGGSMVSKGEELFTGVVPIL
VELDGDVNGHKFSVSGEGEGDATYGKLTLKFICTTGKLPVPWPTLVTTLTYGVQ
CFSRYPDHMKQHDFFKSAMPEGYVQERTIFFKDDGNYKTRAEVKFEGDTLVNR
IELKGIDFKEDGNILGHKLEYNYNSHNVYIMADKQKNGIKVNFKIRHNIEDGSV
QLADHYQQNTPIGDGPVLLPDNHYLSTQSALSKDPNEKRDHMVLLEFVTAAGI
TLGMDELYK
- SEQ ID NO: 90: h4D5VH-isoCH1
MEFGLSWLFLVAILKGVQCEVQLVESGGGLVQPGGSLRLSCAASGFNIKDTYIH
WVRQAPGKGLEWVARIYPTNGYTRYADSVKGRFTISADTSKNTAYLQMNSLRA
EDTAVYYCSRWGGDGFYAMDYWGQGTLVTVSSASTKGPSVFPLAPSSKSTSGG
TAALGCLVKDYFPEPVTVSWNSGALTSGVHTFPAVLQSSGLYLLNSVVTVPSSSL
GTQTYICNVNHKPSNTKVDKKVEPKSC
- SEQ ID NO: 91: hCD63 iSoMAbv2 HC
KHLWFFLLLVAAPRWVLSQVQLQESGPGLVKPSETLSLTCTVSGFSLTSYYVQWI
RQPPGKGLEWMGFIRSGGNTDYNSEFKSRLTISRDTSKNQVSLKLSSVTAADTA
VYYCVRGGEYNWDYFEYWGQGTLVTVSSASTKGPSVFPLAPSSKSTSGGTAAL
GCLVKDYFPEPVTVSWNSGALTSGVHTEPAILQSSGLYLLKSVVTVPSSSLGTQT
YICNVNHKPSNTKVDKKVEPKSCDKTHTCPPCPAPELLGGPSVFLFPPKPKDTL
MISRTPEVTCVVVDVSHEDPEVKFNWYVDGVEVHNAKTKPREEQYNSTYRVV

SVLTVLHQDWLNGKEYKCKVSNKALPAPIEKTISKAKGQPREPQVYTLPPSRDE
LTKNQVSLTCLVKGFYPSDIAVEWESNGQPENNYKTTPPVLDSDGSFFLYSKLTV
DKSRWQQGNVFSCSVMHEALHNHYTQKSLSLSPG
- SEQ ID NO: 92: hCD63 iSoMAbv2 HC mut15
MKHLWFFLLLVAAPRWVLSQVQLQESGPGLVKPSETLSLTCTVSGFSLTSYYVQ
WIRQPPGKGLEWMGFIRSGGNTDYNSEFKSRLTISRDTSKNQVSLKLSSVTAAD
TAVYYCVRGGEYNWDYFEYWGQGTLVTVSSASTKGPSVFPLAPSSKSTSGGTA
ALGCLVKDYFPEPVTVSWNSGALTSGVHTEPAVLQSSGLYSLKSVVTVPSSSLGT
QTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRD
ELTKNQVSLTCLVKGFYPSDIAVEWESNGQPENNYKTTPPVLDSDGSFFLYSKLT
VDKSRWQQGNVFSCSVMHEALHNHYTQKSLSLSPG
- SEQ ID NO: 93: hCD63 iSoMAbv2 LC
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTASVVCLLNNFYPREA
KVQWKVDNALQSGNSQEMVTEQDSKDSTYSLNSLLQLSKADYEKHKVYACEV
THQGLSSPVTKSFNRGEC
- SEQ ID NO: 94: hCD63 iSoMAbv2 LC mut3
MVLQTQVFISLLLWISGAYGDIVLTQSPASLAVSPGERATISCRASQSVTIFSINLM
QWFQQKPGQPPKLLIYRASNLASGVPDRFSGSGSGTDFTLTISRVEAEDVAVYYC
QQTRESPPTFGQGTKLEIKRTVAAPSVFIFPPSDEQLKSGTASVVCLLNNFYPREA
KVQWKVDNALQSGNSQESVTEQDSKDSTYSLNSTLTLSKADYEKHKVYACEV
THQGLSSPVTKSFNRGEC

## Claims

1. A method for producing a modified polypeptide comprising an interpeptide linkage represented by the following formula: 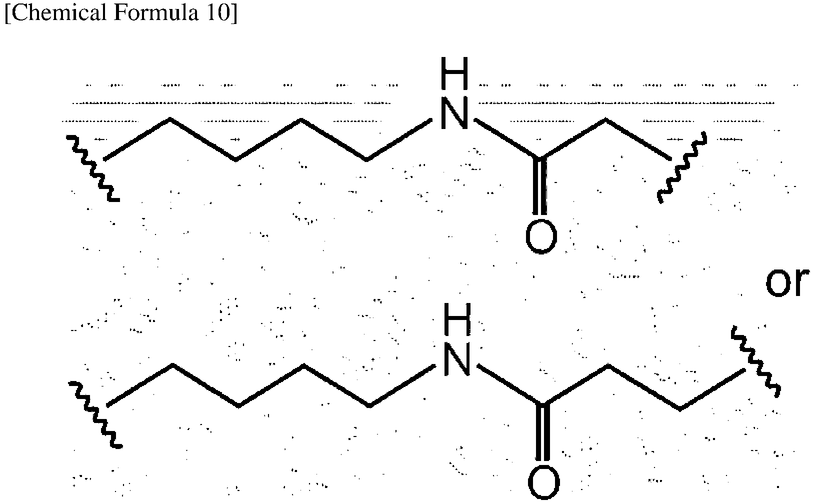 (wherein the dashed lines represent a first or second polypeptide chain, respectively) between a first and a second polypeptide chain, the method comprising:
a step of constructing a three-dimensional structure of an intermediate A comprising the first and second polypeptide chains;
a step of identifying an amino acid sequence present in an interface region where the first and second polypeptide chains are in close proximity to each other in the three-dimensional structure;
a step of mutating the intermediate A, wherein an intermediate B is prepared by introducing into the intermediate A at least one residue of
a) lysine,
b) glutamine, glutamic acid, asparagine, or aspartic acid, and
c) glutamic acid or aspartic acid,
wherein the intermediate B comprises a), b), and c), and the first and second polypeptide chains in the intermediate B comprise a) and b) in the interface region, respectively; and
a step of obtaining the modified polypeptide by forming the interpeptide linkage in the intermediate B.

2. The method according to claim 1, wherein a) and/or b) are introduced based on a distance between a first amino acid residue of the first polypeptide chain and a second amino acid residue of the second polypeptide chain present within the interface region, and types of the first and second amino acid residues.

3. The method according to claim 1, wherein b) is asparagine or aspartic acid, and two amino acid positions at which a distance between α carbons is about 7 to 9 Å in the three-dimensional structure of the intermediate A are identified as amino acid positions for a) and b).

4. The method according to claim 1, wherein b) is glutamine or glutamic acid, and two amino acid positions at which a distance between α carbons is about 8.5 to 10 Å in the three-dimensional structure of the intermediate A are identified as amino acid positions for a) and b).

5. The method according to claim 1, wherein positions of two amino acids whose side chains are disposed in the interior of the three-dimensional structure in the three-dimensional structure of the intermediate A are identified as amino acid positions for a) and b).

6. The method according to claim 1, further comprising a step of constructing a three-dimensional structure of the intermediate B.

7. The method according to claim 6, wherein in the three-dimensional structure of the intermediate B, a distance between the ζ-position nitrogen atom of the residue of a) and an oxygen atom of a carboxyl group in the side chain of the residue of c) is about 2 to 5 Å.

8. The method according to claim 6, wherein in the three-dimensional structure of the intermediate B, a distance between an oxygen atom of a carboxyl group or an amide group in the side chain of the residue of b) and an oxygen atom of a carboxyl group in the side chain of the residue of c) is about 2 to 5 Å.

9. The method according to claim 6, wherein in the three-dimensional structure of the intermediate B, a distance between the ζ-position nitrogen atom of the residue of a) and an oxygen atom of a carboxyl group or an amide group in the side chain of the residue of b) is about 2 to 5 Å.

10. The method according to claim 1, wherein the first polypeptide chain comprises c).

11. The method according to claim 1, further comprising a step of determining hydrophobicity of the interface region.

12. The method according to claim 1, further comprising a step of, in an amino acid sequence present in the interface region, a mutation that deletes a hydrophilic amino acid residue; a mutation that replaces an amino acid residue with a hydrophobic amino acid residue; or insertion of a hydrophobic amino acid residue.

13. The method according to claim 6, wherein a hydrophobic amino acid having an α carbon at a location within 12 Å from the ζ-position nitrogen atom of the residue of a) in the three-dimensional structure of the intermediate B is introduced.

14. The method according to claim 1, wherein hydrophobic amino acids having side chains of different volumes are introduced so as to fill space of the interface region.

15. The method according to claim 1, wherein the first and second polypeptide chains are each contained in different molecules.

16. The method according to claim 15, further comprising a step of introducing a mutation that deletes, or substitutes with another amino acid, at least one of two cysteine residues that form a disulfide bond between the first polypeptide chain and the second polypeptide chain in the intermediate A.

17. The method according to claim 15, further producing a secondary modified polypeptide comprising an interpeptide linkage represented by the following formula: 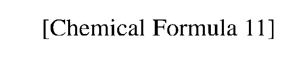 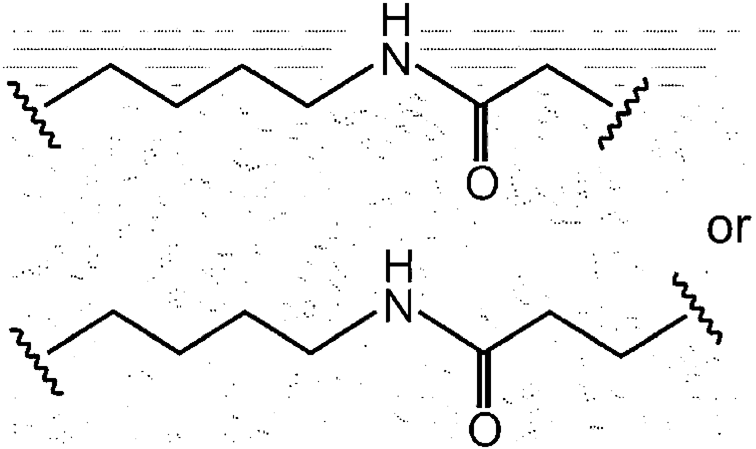 (wherein the dashed lines represent bonds to a polypeptide backbone)
between the modified polypeptide and a third polypeptide chain contained in a molecule different from the modified polypeptide, the method comprising:
a step of constructing a three-dimensional structure of a secondary intermediate A comprising the modified polypeptide and the third polypeptide chain;
a step of identifying an amino acid sequence present in an interface region where the modified polypeptide and the third polypeptide chain are in close proximity to each other in the three-dimensional structure;
a step of mutating the secondary intermediate A, wherein a secondary intermediate B is prepared by introducing into the secondary intermediate A at least one residue of
a) lysine,
b) glutamine, glutamic acid, asparagine, or aspartic acid, and
c) glutamic acid or aspartic acid,
wherein the secondary intermediate B comprises a), b), and c), and the modified polypeptide and the third polypeptide chain in the secondary intermediate B comprise a) and b) in the interface region, respectively; and
a step of obtaining the secondary modified polypeptide by forming the interpeptide linkage in the secondary intermediate B.

18. A polypeptide comprising an interpeptide linkage represented by the following formula: 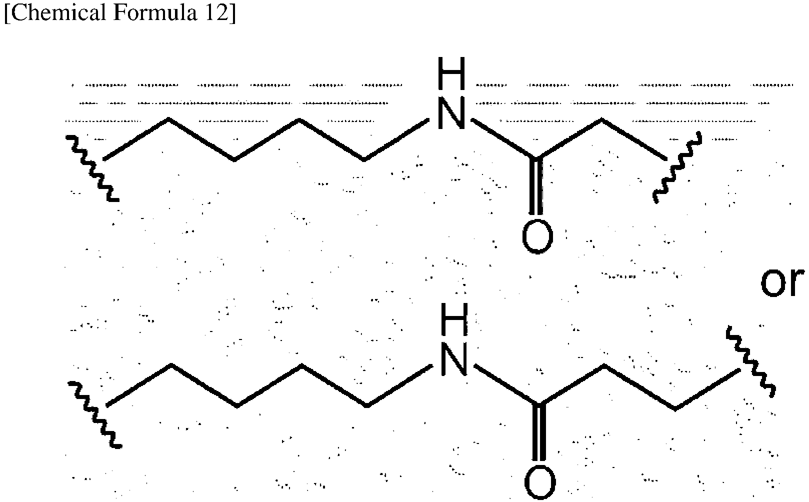 (wherein the dashed lines represent bonds to a polypeptide backbone)
between a first and a second polypeptide chain, the polypeptide comprising
a) lysine,
b) glutamine, glutamic acid, asparagine, or aspartic acid, and
c) glutamic acid or aspartic acid,
wherein the first and second polypeptide chains each comprise a) and b) in an interface region where the first and second polypeptide chains are in close proximity to each other in a three-dimensional structure of the polypeptide.

19. The polypeptide according to claim 18, which is an antibody or a T cell receptor (TCR).

20. The polypeptide according to claim 19, wherein the interpeptide linkage is present in a constant region.

21. The polypeptide according to claim 18, comprising an interpeptide linkage between a heavy chain constant region and a light chain constant region of the antibody.

22. The polypeptide according to claim 18, wherein the first and second polypeptide chains are present on molecules different from each other.

23. A CH1 region in which the amino acid at position 183 of a heavy chain is substituted with asparagine, or a nucleic acid encoding the region;
a heavy chain constant region comprising the CH1 region, or a nucleic acid encoding the region; or
a heavy chain comprising the CH1 region, or a nucleic acid encoding the heavy chain.

24. A CL region in which the amino acid at position 133 of a light chain is substituted with glutamic acid and the amino acid at position 176 of the light chain is substituted with lysine, or a nucleic acid encoding the region;
a light chain constant region comprising the CL region, or a nucleic acid encoding the region; or
a light chain comprising the CL region, or a nucleic acid encoding the light chain.

25. An antibody comprising the heavy chain according to claim 23 and the light chain according to claim 24.

26. A Fab fragment, a Fab' fragment, or an F(ab')₂ fragment, comprising the CH1 region according to claim 23 and the CL region according to claim 24.

27. A chimeric antigen receptor having: a Fab fragment comprising the CH1 region according to claim 23 and the CL region according to claim 24; a hinge region; a transmembrane domain; and an intracellular domain.

28. A cell expressing the chimeric antigen receptor according to claim 27.
